# EUROPEAN PATENT SPECIFICATION

(11) **EP 1 951 232 B1**
(45) Date of publication and mention of the grant of the patent: **03.04.2013**
(21) Application number: 06836771.3
(22) Date of filing: 31.10.2006
(51) Int. Cl.: A61K 9/22

(54) **METHODS OF REDUCING ALCOHOL-INDUCED DOSE DUMPING FOR OPIOID SUSTAINED RELEASE ORAL DOSAGE FORMS**
VERFAHREN ZUR REDUZIERUNG VON ALKOHOL-INDUZIERTEM DOSIS-DUMPING FÜR ORALE OPIOID-DOSIERFORMEN MIT VERZÖGERTER FREISETZUNG
METHODES DE REDUCTION DE LA LIBERATION MASSIVE, INDUITE PAR L'ALCOOL, DES FORMES POSOLOGIQUES ORALES A LIBERATION SOUTENUE D'OPIOIDES

(30) Priority: 31.10.2005 US 731995 P; 18.05.2006 US 802017 P; 11.08.2006 US 837049 P
(43) Date of publication of application: 06.08.2008
(73) Proprietor: ALZA Corporation, Mountain View, CA 94043-7210 (US)
(72) Inventor: SATHYAN, Gaytri, San Jose, CA 95128 (US); DAVAR, Nipun, Pleasanton, CA 94566-6489 (US); HASTEDT, Jayne, San Carlos, CA 94070 (US); PORS, Linda, Saratoga, CA 95070 (US); CASADEVALL, Gemma, E-08019 Barcelona (ES); CRUZ, Evangeline, Hayward, CA 94542 (US)
(74) Representative: Williams, Paul Edwin
(86) International application number: PCT/US2006/042687
(87) International publication number: WO 2007/053698

(56) References cited:
- WO-A-96/14058
- GB-A- 2 196 848
- US-A- 5 968 551
- US-A1- 2003 099 704
- US-B1- 6 306 438
- US-B2- 6 627 635
- MEYER R.J. ET AL.: 'Awareness Topic: Mitigating the Risks of Ethanol Induced Dose Dumping from Oral Sustained/Controlled Release Dosage Forms' FDA'S ACPS MEETING 26 October 2005, XP003014849

## Description

### FIELD OF THE INVENTION

The invention pertains to methods of sustained release administration of opioids, including but not limited to hydromorphone and oxycodone, that exhibit improved properties with respect to coadministration with aqueous alcohol.

### BACKGROUND OF THE INVENTION

Ethanol-induced dose dumping of opioid sustained release oral dosage forms can be a serious problem for patients taking such oral dosage forms.

Opioid sustained release oral dosage forms are designed to deliver opioids over an prolonged period to a patient. One opioid sustained release oral dosage form is often prescribed to take the place of multiple opioid immediate release oral dosage forms. For instance, there is great demand for once-per-day (qd) and twice-per-day (bid) opioid sustained release oral dosage forms that provide pain relief to a patient for an entire day.

Consequently, the amount of opioid contained in such dosage forms, particularly once-per-day opioid sustained release oral dosage forms, is significantly greater than is traditionally included in immediate release opioid dosage forms. Anything that causes dose dumping from such opioid sustained release oral dosage forms can cause an opioid drug overdose, leading to respiratory depression and possibly even death.

The inventors have recognized that one modality for causing dose dumping (i.e. immediate release) is enhanced delivery rates caused by coadministration of the opioid sustained release oral dosage forms with aqueous alcohol, particularly aqueous ethanol. Various alcohols can increase release of the opioid from the opioid sustained release oral dosage forms at undesirably high rates, even approaching dose dumping/immediate release.

Accordingly, it would be desirable to develop opioid sustained release oral dosage forms, and related methods, that do not have the problems of the prior art relating to alcohol-induced dose dumping, especially ethanol-induced dose dumping. It would be even more desirable if those opioid sustained release oral dosage forms and related methods were once-per-day or twice-per-day opioid sustained release oral dosage forms and related methods.

### SUMMARY OF THE INVENTION

In an aspect, the invention relates to a method comprising: providing a once-per-day hydromorphone sustained release dosage form comprising hydromorphone and a sustained release dosing structure that provides once-per-day dosing; coadministering the once-per-day hydromorphone sustained release dosage form with aqueous alcohol to a patient; releasing hydromorphone from the once-per-day hydromorphone sustained release dosage form; wherein the aqueous alcohol comprises alcohol at concentrations equal to or greater than about 20% volume/volume; and wherein a ratio of a mean single dose maximum plasma hydromorphone concentration achieved when the once-per-day hydromorphone sustained release dosage form is coadministered to a patient with the aqueous alcohol to a mean single dose maximum plasma hydromorphone concentration achieved when the once-per-day hydromorphone sustained release dosage form is administered to a patient without coadministration of the aqueous alcohol is equal to or less than about 1.8:1.

In another aspect, the invention relates to a method comprising: providing a once-per-day hydromorphone sustained release dosage form comprising hydromorphone and a sustained release dosing structure that provides once-per-day dosing; coadministering the once-per-day hydromorphone sustained release dosage form with aqueous alcohol to a patient; releasing hydromorphone from the once-per-day hydromorphone sustained release dosage form; wherein the aqueous alcohol comprises alcohol at concentrations equal to or greater than about 20% volume/volume; and wherein a ratio of an individual patient single dose maximum plasma hydromorphone concentration achieved when the once-per-day hydromorphone sustained release dosage form is coadministered to the patient with the aqueous alcohol to an individual patient single dose maximum plasma hydromorphone concentration achieved when the once-per-day hydromorphone sustained release dosage form is administered to a patient without coadministration of the aqueous alcohol is equal to or less than about 5:1.

In still another aspect, the invention relates to a method comprising: providing a once-per-day hydromorphone sustained release dosage form comprising hydromorphone and a sustained release dosing structure that provides once-per-day dosing; coadministering the once-per-day hydromorphone sustained release dosage form with aqueous alcohol to a patient; releasing hydromorphone from the once-per-day hydromorphone sustained release dosage form; wherein the once-per-day hydromorphone sustained release dosage form releases less than or equal to about 80 weight percent of the dose of hydromorphone from the once-per-day hydromorphone sustained release dosage form as measured (a) using an in vitro test method that comprises test media and (b) in a period of about 2 hours following initiation of the in vitro test method; and wherein the test media comprises aqueous alcohol that comprises alcohol at concentrations equal to or greater than about 20% volume/volume.

In another aspect, the invention relates to a method comprising: providing a hydromorphone sustained release dosage form comprising hydromorphone and a sustained release dosing structure; coadministering the hydromorphone sustained release dosage form with aqueous alcohol to a patient; releasing hydromorphone from the hydromorphone sustained release dosage form; wherein the aqueous alcohol comprises alcohol at concentrations equal to or greater than about 20% volume/volume; and wherein a ratio of a mean single dose maximum plasma hydromorphone concentration achieved when the dosage form is coadministered to the patient with the aqueous alcohol to a mean single dose maximum plasma hydromorphone concentration achieved when the dosage form is administered to a patient without coadministration of the aqueous alcohol is equal to or less than about 1.8:1.

In yet another aspect, the invention relates to a method comprising: providing a hydromorphone sustained release dosage form comprising hydromorphone and a sustained release dosing structure; coadministering the hydromorphone sustained release dosage form with aqueous alcohol to a patient; releasing hydromorphone from the hydromorphone sustained release dosage form; wherein the aqueous alcohol comprises alcohol at concentrations equal to or greater than about 20% volume/volume; and wherein a ratio of an individual patient single dose maximum plasma hydromorphone concentration achieved when the dosage form is coadministered to the patient with the aqueous alcohol to an individual patient single dose maximum plasma hydromorphone concentration achieved when the dosage form is administered to a patient without coadministration of the aqueous alcohol is equal to or less than about 5:1.

In still another aspect, the invention relates to a method comprising: providing a hydromorphone sustained release dosage form comprising a dose of hydromorphone and a sustained release dosing structure; coadministering the hydromorphone sustained release dosage form with aqueous alcohol to a patient; releasing the dose of hydromorphone from the hydromorphone sustained release dosage form; wherein the hydromorphone sustained release dosage form releases less than about 80 weight percent of the dose of hydromorphone from the hydromorphone sustained release dosage form as measured (a) using an in vitro test method that comprises test media and (b) in a period of about 2 hours following initiation of the in vitro test method; and wherein the test media comprises aqueous alcohol that comprises alcohol at concentrations equal to or greater than about 20% volume/volume.

In an aspect, the invention relates to a method comprising: providing a once-per-day opioid sustained release dosage form comprising opioid and a sustained release dosing structure that provides once-per-day dosing; coadministering the once-per-day opioid sustained release dosage form with aqueous alcohol to a patient; releasing opioid from the once-per-day opioid sustained release dosage form; wherein the aqueous alcohol comprises alcohol at concentrations equal to or greater than about 20% volume/volume; and wherein a ratio of a mean single dose maximum plasma opioid concentration achieved when the once-per-day opioid sustained release dosage form is coadministered to the patient with the aqueous alcohol to a mean single dose maximum plasma opioid concentration achieved when the once-per-day opioid sustained release dosage form is administered to a patient without coadministration of the aqueous alcohol is equal to or less than about 1.8:1.

In another aspect, the invention relates to a method comprising: providing a once-per-day opioid sustained release dosage form comprising opioid and a sustained release dosing structure that provides once-per-day dosing; coadministering the once-per-day opioid sustained release dosage form with aqueous alcohol to a patient; releasing opioid from the once-per-day opioid sustained release dosage form; wherein the aqueous alcohol comprises alcohol at concentrations equal to or greater than about 20% volume/volume; and wherein a ratio of an individual patient single dose maximum plasma opioid concentration achieved when the once-per-day opioid sustained release dosage form is coadministered to the patient with the aqueous alcohol to an individual patient single dose maximum plasma opioid concentration achieved when the once-per-day opioid sustained release dosage form is administered to a patient without coadministration of the aqueous alcohol is equal to or less than about 5:1.

In still another aspect, the invention relates to a method comprising: providing a once-per-day opioid sustained release dosage form comprising opioid and a sustained release dosing structure that provides once-per-day dosing; coadministering the once-per-day opioid sustained release dosage form with aqueous alcohol to a patient; releasing opioid from the once-per-day opioid sustained release dosage form; wherein the once-per-day opioid sustained release dosage form releases less than or equal to about 80 weight percent of the dose of opioid from the once-per-day opioid sustained release dosage form as measured (a) using an in vitro test method that comprises test media and (b) in a period of about 2 hours following initiation of the in vitro test method; and wherein the test media comprises aqueous alcohol that comprises alcohol at concentrations equal to or greater than about 20% volume/volume.

In an aspect, the invention relates to a method comprising: providing a opioid sustained release dosage form comprising opioid and a sustained release dosing structure; coadministering the opioid sustained release dosage form with aqueous alcohol to a patient; releasing opioid from the opioid sustained release dosage form; wherein the aqueous alcohol comprises alcohol at concentrations equal to or greater than about 20% volume/volume; and wherein a ratio of a mean single dose maximum plasma opioid concentration achieved when the dosage form is coadministered to the patient with the aqueous alcohol to a mean single dose maximum plasma opioid concentration achieved when the dosage form is administered to a patient without coadministration of the aqueous alcohol is equal to or less than about 1.8:1.

In an aspect, the invention relates to a method comprising: providing a opioid sustained release dosage form comprising opioid and a sustained release dosing structure; coadministering the opioid sustained release dosage form with aqueous alcohol to a patient; releasing opioid from the opioid sustained release dosage form; wherein the aqueous alcohol comprises alcohol at concentrations equal to or greater than about 20% volume/volume; and wherein a ratio of an individual patient single dose maximum plasma opioid concentration achieved when the dosage form is coadministered to the patient with the aqueous alcohol to an individual patient single dose maximum plasma opioid concentration achieved when the dosage form is administered to a patient without coadministration of the aqueous alcohol is equal to or less than about 5:1.

In another aspect, the invention relates to a method comprising providing a opioid sustained release dosage form comprising a dose of opioid and a sustained release dosing structure; coadministering the opioid sustained release dosage form with aqueous alcohol to a patient; releasing the dose of opioid from the opioid sustained release dosage form; wherein the opioid sustained release dosage form releases less than about 80 weight percent of the dose of opioid from the opioid sustained release dosage form as measured (a) using an in vitro test method that comprises test media and (b) in a period of about 2 hours following initiation of the in vitro test method; and wherein the test media comprises aqueous alcohol that comprises alcohol at concentrations equal to or greater than about 20% volume/volume.

In still another aspect, the invention relates to a method comprising: providing a once-per-day hydromorphone sustained release dosage form comprising hydromorphone and a sustained release dosing structure that provides once-per-day dosing; coadministering the once-per-day hydromorphone sustained release dosage form with aqueous alcohol to a patient; releasing hydromorphone from the once-per-day hydromorphone sustained release dosage form; wherein the aqueous alcohol comprises alcohol at concentrations equal to or greater than about 20% volume/volume; and wherein a ratio of the median single dose, time to maximum plasma concentration achieved when the dosage form is coadministered to the patient with the aqueous alcohol to the median single dose, time to maximum plasma concentration achieved when the once-per-day hydromorphone sustained release dosage form is administered to a patient without coadministration of the aqueous alcohol ranges from about 0.5 to about 1.0.

In another aspect, the invention relates to a method comprising: providing a hydromorphone sustained release dosage form comprising hydromorphone and a sustained release dosing structure; coadministering the hydromorphone sustained release dosage form with aqueous alcohol to a patient; releasing hydromorphone from the hydromorphone sustained release dosage form; wherein the aqueous alcohol comprises alcohol at concentrations equal to or greater than about 20% volume/volume; and wherein a ratio of the median single dose, time to maximum plasma concentration achieved when the dosage form is coadministered to the patient with the aqueous alcohol to the median single dose, time to maximum plasma concentration achieved when the hydromorphone sustained release dosage form is administered to a patient without coadministration of the aqueous alcohol ranges from about 0.5 to about 1.0.

In an aspect, the invention relates to a method comprising: providing a once-per-day opioid sustained release dosage form comprising opioid and a sustained release dosing structure that provides once-per-day dosing; coadministering the once-per-day opioid sustained release dosage form with aqueous alcohol to a patient; releasing opioid from the once-per-day opioid sustained release dosage form; wherein the aqueous alcohol comprises alcohol at concentrations equal to or greater than about 20% volume/volume; and wherein a ratio of the median single dose, time to maximum plasma concentration achieved when the dosage form is coadministered to the patient with the aqueous alcohol to the median single dose, time to maximum plasma concentration achieved when the once-per-day opioid sustained release dosage form is administered to a patient without coadministration of the aqueous alcohol ranges from about 0.5 to about 1.0.

In another aspect, the invention relates to a method comprising: providing a opioid sustained release dosage form comprising opioid and a sustained release dosing structure; coadministering the opioid sustained release dosage form with aqueous alcohol to a patient; releasing opioid from the opioid sustained release dosage form; wherein the aqueous alcohol comprises alcohol at concentrations equal to or greater than about 20% volume/volume; and wherein a ratio of the median single dose, time to maximum plasma concentration achieved when the dosage form is coadministered to the patient with the aqueous alcohol to the median single dose, time to maximum plasma concentration achieved when the opioid sustained release dosage form is administered to a patient without coadministration of the aqueous alcohol ranges from about 0.5 to about 1.0.

In a further aspect, the invention relates to a method of use of a hydromorphone sustained release dosage form (e.g., a dosage form suitable for once-a-day dosing) comprising providing the dosage form to a patient population which includes individuals that can be expected to at least occasionally co-ingest the dosage form with an alcoholic beverage, wherein the dosage form when tested in vivo on a population of test subjects has:
(a) a mean arithmetic ratio (preferably, under fasted conditions) of:
   (i) a single dose maximum plasma hydromorphone concentration as a result of co-ingestion within about 30 minutes of the dosage form and about 240 milliliters of an aqueous solution having an alcohol concentration of about 20% volume/volume to
   (ii) a single dose maximum plasma hydromorphone concentration as a result of co-ingestion within about 30 minutes of the dosage form and about 240 milliliters of the aqueous solution with water substituted for the alcohol
   that is equal to or less than about 1.9:1 (preferably, equal to or less than about 1.7:1, more preferably, equal to or less than about 1.5:1, and most preferably, equal to or less than about 1.4:1); and/or
(b) a ratio (preferably, under fasted conditions) of:
   (i) an individual test subject's single dose maximum plasma hydromorphone concentration as a result of co-ingestion within about 30 minutes of the dosage form and about 240 milliliters of an aqueous solution having an alcohol concentration of about 20% volume/volume
      to
   (ii) the same test subject's single dose maximum plasma hydromorphone concentration as a result of co-ingestion within about 30 minutes of the dosage form and about 240 milliliters of the aqueous solution with water substituted for the alcohol
   that is equal to or less than about 5:1 (preferably, equal to or less than about 4:1, more preferably, equal to or less than about 3:1, and most preferably, equal to or less than about 2.5:1); and/or
(c) a ratio (preferably, under fasted conditions) of:
   (i) a median single dose, time to maximum plasma hydromorphone concentration as a result of co-ingestion within about 30 minutes of the dosage form and about 240 milliliters of an aqueous solution having an alcohol concentration of about 20% volume/volume
      to
   (ii) a median single dose, time to maximum plasma hydromorphone concentration as a result of co-ingestion within about 30 minutes of the dosage form and about 240 milliliters of the aqueous solution with water substituted for the alcohol
   that is in the range of from about 0.5 to about 1.0 (preferably, from about 0.6 to about 1.0, and most preferably, from about 0.7 to about 1.0).

In certain preferred embodiments of this aspect of the invention, all of the above in vivo test criteria are satisfied by the dosage form. In other preferred embodiments, one or more and preferably all of the above in vivo test criteria are also satisfied when the alcohol concentration in the aqueous solution of (a)(i), (b)(i), and/or (c)(i) is about 40% volume/volume.

In an additional aspect, the invention relates to a method of use of a hydromorphone sustained release dosage form (e.g., a dosage form suitable for once-a-day dosing) which comprises a dose of hydromorphone and a sustained release dosing structure, said method comprising providing the dosage form to a patient population which includes individuals that can be expected to at least occasionally co-ingest the dosage form with an alcoholic beverage, wherein when tested using an in vitro test method that employs a test medium that comprises aqueous alcohol at a concentration of about 20% volume/volume, the dosage form releases less than or equal to about 50 weight percent of the dose of hydromorphone (preferably, less than or equal to about 25 weight percent, more preferably, less than or equal to about 10 weight percent, most preferably, less than or equal to about 5 weight percent) in a period of about 2 hours following initiation of the in vitro test method.

In certain preferred embodiments of this aspect of the invention, the above in vitro test criterion is also satisfied when the alcohol concentration in the test medium is about 40% volume/volume.

In another aspect, the invention relates to a method for reducing adverse effects associated with alcohol-induced dose dumping in patients who are orally receiving sustained release hydromorphone comprising:
providing a sustained release dosage form which comprises hydromorphone; and
administering the dosage form to a patient;
wherein the dosage form when tested in vivo on a population of test subjects has:
(a) a mean arithmetic ratio (preferably, under fasted conditions) of:
   (i) a single dose maximum plasma hydromorphone concentration as a result of co-ingestion within about 30 minutes of the dosage form and about 240 milliliters of an aqueous solution having an alcohol concentration of about 20% volume/volume to
   (ii) a single dose maximum plasma hydromorphone concentration as a result of co-ingestion within about 30 minutes of the dosage form and about 240 milliliters of the aqueous solution with water substituted for the alcohol
   that is equal to or less than about 1.9:1 (preferably, equal to or less than about 1.7:1, more preferably, equal to or less than about 1.5:1, and most preferably, equal to or less than about 1.4:1); and/or
(b) a ratio (preferably, under fasted conditions) of:
   (i) an individual test subject's single dose maximum plasma hydromorphone concentration as a result of co-ingestion within about 30 minutes of the dosage form and about 240 milliliters of an aqueous solution having an alcohol concentration of about 20% volume/volume
      to
   (ii) the same test subject's single dose maximum plasma hydromorphone concentration as a result of co-ingestion within about 30 minutes of the dosage form and about 240 milliliters of the aqueous solution with water substituted for the alcohol
   that is equal to or less than about 5:1 (preferably, equal to or less than about 4:1, more preferably, equal to or less than about 3:1, and most preferably, equal to or less than about 2.5:1); and/or
(c) a ratio (preferably, under fasted conditions) of:
   (i) a median single dose, time to maximum plasma hydromorphone concentration as a result of co-ingestion within about 30 minutes of the dosage form and about 240 milliliters of an aqueous solution having an alcohol concentration of about 20% volume/volume
      to
   (ii) a median single dose, time to maximum plasma hydromorphone concentration as a result of co-ingestion within about 30 minutes of the dosage form and about 240 milliliters of the aqueous solution with water substituted for the alcohol
   that is in the range of from about 0.5 to about 1.0 (preferably, from about 0.6 to about 1.0, and most preferably, from about 0.7 to about 1.0).

In certain preferred embodiments of this aspect of the invention, all of the above in vivo test criteria are satisfied by the dosage form. In other preferred embodiments, one or more and preferably all of the above in vivo test criteria are also satisfied when the alcohol concentration in the aqueous solution of (a)(i), (b)(i), and/or (c)(i) is about 40% volume/volume.

In an additional aspect, the invention relates to a method for reducing adverse effects associated with alcohol-induced dose dumping in patients who are orally receiving sustained release hydromorphone comprising:
providing a sustained release dosage form which comprises a dose of hydromorphone; and
administering the dosage form to a patient;
wherein when tested using an in vitro test method that employs a test medium that comprises aqueous alcohol at a concentration of about 20% volume/volume, the dosage form releases less than or equal to about 50 weight percent of the dose of hydromorphone (preferably, less than or equal to about 25 weight percent, more preferably, less than or equal to about 10 weight percent, most preferably, less than or equal to about 5 weight percent) in a period of about 2 hours following initiation of the in vitro test method.

In certain preferred embodiments of this aspect of the invention, the above in vitro test criterion is also satisfied when the alcohol concentration in the test medium is about 40% volume/volume.

In a further aspect, the invention relates to a method of use of an opioid sustained release dosage form (e.g., a dosage form suitable for once-a-day dosing) comprising providing the dosage form to a patient population which includes individuals that can be expected to at least occasionally co-ingest the dosage form with an alcoholic beverage, wherein the dosage form when tested in vivo on a population of test subjects has:
(a) a mean arithmetic ratio (preferably, under fasted conditions) of:
   (i) a single dose maximum plasma opioid concentration as a result of co-ingestion within about 30 minutes of the dosage form and about 240 milliliters of an aqueous solution having an alcohol concentration of about 20% volume/volume
      to
   (ii) a single dose maximum plasma opioid concentration as a result of co-ingestion within about 30 minutes of the dosage form and about 240 milliliters of the aqueous solution with water substituted for the alcohol
   that is equal to or less than about 1.9:1 (preferably, equal to or less than about 1.7:1, more preferably, equal to or less than about 1.5:1, and most preferably, equal to or less than about 1.4:1); and/or
(b) a ratio (preferably, under fasted conditions) of:
   (i) an individual test subject's single dose maximum plasma opioid concentration as a result of co-ingestion within about 30 minutes of the dosage form and about 240 milliliters of an aqueous solution having an alcohol concentration of about 20% volume/volume
      to
   (ii) the same test subject's single dose maximum plasma opioid concentration as a result of co-ingestion within about 30 minutes of the dosage form and about 240 milliliters of the aqueous solution with water substituted for the alcohol
   that is equal to or less than about 5:1 (preferably, equal to or less than about 4:1, more preferably, equal to or less than about 3:1, and most preferably, equal to or less than about 2.5:1); and/or
(c) a ratio (preferably, under fasted conditions) of:
   (i) a median single dose, time to maximum plasma opioid concentration as a result of co-ingestion within about 30 minutes of the dosage form and about 240 milliliters of an aqueous solution having an alcohol concentration of about 20% volume/volume
      to
   (ii) a median single dose, time to maximum plasma opioid concentration as a result of co-ingestion within about 30 minutes of the dosage form and about 240 milliliters of the aqueous solution with water substituted for the alcohol
   that is in the range of from about 0.5 to about 1.0 (preferably, from about 0.6 to about 1.0, and most preferably, from about 0.7 to about 1.0).

In certain preferred embodiments of this aspect of the invention, all of the above in vivo test criteria are satisfied by the dosage form. In other preferred embodiments, one or more and preferably all of the above in vivo test criteria are also satisfied when the alcohol concentration in the aqueous solution of (a)(i), (b)(i), and/or (c)(i) is about 40% volume/volume.

In an additional aspect, the invention relates to a method of use of an opioid sustained release dosage form (e.g., a dosage form suitable for once-a-day dosing) which comprises a dose of an opioid and a sustained release dosing structure, said method comprising providing the dosage form to a patient population which includes individuals that can be expected to at least occasionally co-ingest the dosage form with an alcoholic beverage, wherein when tested using an in vitro test method that employs a test medium that comprises aqueous alcohol at a concentration of about 20% volume/volume, the dosage form releases less than or equal to about 50 weight percent of the dose of the opioid (preferably, less than or equal to about 25 weight percent, more preferably, less than or equal to about 10 weight percent, most preferably, less than or equal to about 5 weight percent) in a period of about 2 hours following initiation of the in vitro test method.

In certain preferred embodiments of this aspect of the invention, the above in vitro test criterion is also satisfied when the alcohol concentration in the test medium is about 40% volume/volume.

In another aspect, the invention relates to a method for reducing adverse effects associated with alcohol-induced dose dumping in patients who are orally receiving a sustained release opioid comprising:
providing a sustained release dosage form which comprises an opioid; and
administering the dosage form to a patient;
wherein the dosage form when tested in vivo on a population of test subjects has:
(a) a mean arithmetic ratio (preferably, under fasted conditions) of:
   (i) a single dose maximum plasma opioid concentration as a result of co-ingestion within about 30 minutes of the dosage form and about 240 milliliters of an aqueous solution having an alcohol concentration of about 20% volume/volume to
   (ii) a single dose maximum plasma opioid concentration as a result of co-ingestion within about 30 minutes of the dosage form and about 240 milliliters of the aqueous solution with water substituted for the alcohol
   that is equal to or less than about 1.9:1 (preferably, equal to or less than about 1.7:1, more preferably, equal to or less than about 1.5:1, and most preferably, equal to or less than about 1.4:1); and/or
(b) a ratio (preferably, under fasted conditions) of:
   (i) an individual test subject's single dose maximum plasma opioid concentration as a result of co-ingestion within about 30 minutes of the dosage form and about 240 milliliters of an aqueous solution having an alcohol concentration of about 20% volume/volume
      to
   (ii) the same test subject's single dose maximum plasma opioid concentration as a result of co-ingestion within about 30 minutes of the dosage form and about 240 milliliters of the aqueous solution with water substituted for the alcohol
   that is equal to or less than about 5:1 (preferably, equal to or less than about 4:1, more preferably, equal to or less than about 3:1, and most preferably, equal to or less than about 2.5:1); and/or
(c) a ratio (preferably, under fasted conditions) of:
   (i) a median single dose, time to maximum plasma opioid concentration as a result of co-ingestion within about 30 minutes of the dosage form and about 240 milliliters of an aqueous solution having an alcohol concentration of about 20% volume/volume
      to
   (ii) a median single dose, time to maximum plasma opioid concentration as a result of co-ingestion within about 30 minutes of the dosage form and about 240 milliliters of the aqueous solution with water substituted for the alcohol
   that is in the range of from about 0.5 to about 1.0 (preferably, from about 0.6 to about 1.0, and most preferably, from about 0.7 to about 1.0).

In certain preferred embodiments of this aspect of the invention, all of the above in vivo test criteria are satisfied by the dosage form. In other preferred embodiments, one or more and preferably all of the above in vivo test criteria are also satisfied when the alcohol concentration in the aqueous solution of (a)(i), (b)(i), and/or (c)(i) is about 40% volume/volume.

In an additional aspect, the invention relates to a method for reducing adverse effects associated with alcohol-induced dose dumping in patients who are orally receiving a sustained release opioid comprising:
providing a sustained release dosage form which comprises a dose of an opioid; and
administering the dosage form to a patient;
wherein when tested using an in vitro test method that employs a test medium that comprises aqueous alcohol at a concentration of about 20% volume/volume, the dosage form releases less than or equal to about 50 weight percent of the dose of the opioid (preferably, less than or equal to about 25 weight percent, more preferably, less than or equal to about 10 weight percent, most preferably, less than or equal to about 5 weight percent) in a period of about 2 hours following initiation of the in vitro test method.

In certain preferred embodiments of this aspect of the invention, the above in vitro test criterion is also satisfied when the alcohol concentration in the test medium is about 40% volume/volume.

In preferred embodiments of all of the above aspects of the invention, the opioid (e.g., hydromorphone) sustained release dosage form is provided to patients without special label warnings to physicians and/or patients regarding potential lethal effects from taking the dosage form with alcohol, i.e., without warnings beyond the typical warning which advises patients taking opioids of potential side effects, e.g., drowsiness, resulting from the taking of opioids with alcohol.

Additional non-limiting aspects of the invention are set forth in Section V below entitled "Features of the Invention." It is to be understood that the above aspects of the invention as well as those set forth in Section V below can be used in any and all combinations.

### BRIEF DESCRIPTION OF THE FIGURES

Figure 1 shows an elementary osmotic pump dosage form according to the invention.
Figure 2 shows certain inventive embodiments of sustained release dosage forms.
Figures 3 shows another exemplary dosage form.
Figure 4 shows another exemplary dosage form.
Figures 5A-5C show another exemplary dosage form
Figure 6 shows in vitro cumulative release profiles of hydromorphone HCl 16mg tablets according to the invention in ethanol solutions.
Figure 7 shows dissolution profiles comparison between hydromorphone HCl 16g according to the invention and Palladone XL 32mg in the presence of aqueous alcohol.
Figure 8 shows a mean (and SD) hydromorphone plasma concentration profile.
Figure 9 shows a mean (and SD) hydromorphone plasma concentration profile.
Figure 10 shows individual Cmax ratios: Group 1 alcohol study vs replicate dosing study.
Figure 11 shows individual Cmax ratios: Group 2 alcohol study vs replicate dosing study.
Figure 12 shows oxycodone HCl release from formulations with and without stearyl alcohol.
Figure 13 shows hydromorphone HCl release from formulations with and without stearyl alcohol.
Figure 14 shows the effect of Eudragit ® RS PO on oxycodone HCl drug release.
Figure 15 shows the effect of Eudragit ® RS PO on hydromorphone HCl drug release.
Figure 16 shows relative effects of stearyl alcohol, carnauba wax, and hydrogenated polyoxyl 60 castor oil on Oxycodone HCl release functionality.
Figure 17 shows in vitro dissolution profiles of OxyContin® tablets.

### DETAILED DESCRIPTION

### I. Recited Opioid Sustained Release Oral Dosage Forms

After recognizing the problems in the prior art noted above, the inventors unexpectedly discovered the inventive embodiments that may provide for solutions to alcohol-induced dose dumping, particularly ethanol-induced dose dumping.

Of note in the discovery is the failure of the prior art to appreciate the usefulness of the inventive methods and associated sustained release dosage forms in solving the problems of alcohol-induced, especially ethanol-induced, dose dumping. Dosage forms similar to those disclosed herein have been used for their abuse deterrence properties, but no teaching or suggestion exists in the prior art that these structures can be used in solving the problems associated with alcohol-induced, especially ethanol-induced, dose dumping. For instance, United States Published Patent Application 2005163856 of Maloney et al. discloses a fine particle size cationic exchange resin whose inclusion in an oxycodone dosage form improves the performance of the dosage form with respect to in vitro extractions that might be performed by a potential abuser. However, Maloney et al. provide no teaching or suggestion that this property might be useful in solving the aqueous alcohol-induced, especially aqueous ethanol-induced, in vivo dose dumping problem addressed by the inventors. That teaching or suggestion was provided by the inventors.

Further evidence that the present invention was not appreciated in the art prior to the present invention is the fact that others in the art developed sustained release opioid dosage forms that are, in fact, susceptible to alcohol-induced, especially ethanol-induced, dose dumping. For instance, Palladone® extended release hydromorphone (Purdue Pharma LP), Kadian ® (Alpharma US Pharms), and Avinza® (Ligand Pharmaceuticals) all are reported to have problems with alcohol-induced, especially ethanol-induced, dose dumping. The fact that these products were commercialized, despite the attendant dangers of alcohol-induced, especially ethanol-induced, dose dumping, is evidence that the problem and the solutions provided herein were not appreciated in the art prior to the present invention.

After recognizing the problem and its solution, the inventors considered a number of embodiments of the present invention. In certain embodiments, it may be possible to provide dosage form coatings that operate to reduce or prevent aqueous alcohol induced dose dumping. In additional embodiments, certain hydrophobic and/or hydrophilic components may be selected that operate to reduce or prevent aqueous alcohol induced dose dumping. In protective dosage form coating embodiments, the coatings selected may serve to modify the time of release, such as enteric coatings, or may be resistant to swelling or dissolution in alcohol, such as semi-permeable membrane coatings or certain non-enteric coatings.

In embodiments where hydrophobic components are selected to reduce or prevent aqueous alcohol induced dose dumping, materials that are relatively insoluble in water and minimally swell in aqueous alcohol may be advantageously selected. For instance, hydrophobic polymers can be selected that minimally swell and are relatively insoluble in water, and exhibit equal or lesser swelling and/or solubility in aqueous alcohol. In embodiments that comprise non-polymeric hydrophobic components (including but not limited to waxes or fatty acid alcohols like stearyl alcohol), those with less solubility/swelling in aqueous alcohol than in water are preferred. In embodiments where hydrophilic components are selected to reduce or prevent aqueous alcohol induced dose-dumping, materials that are less soluble and have less tendency to swell in aqueous alcohol, as compared to water, may be advantageously selected. For instance, hydrophilic polymers can be selected that that exhibit equal or lesser swelling and/or solubility in aqueous alcohol as compared to water. In embodiments that comprise non-polymeric hydrophilic components, those with less solubility/swelling in aqueous alcohol than in water are preferred.

One technique for establishing desirable coatings, and hydrophobic and hydrophobic components, useful in the practice of this invention is to cast films of the materials in question, and test these materials for swelling in the presence of aqueous alcohol. Mass screening techniques can be used with this film assay to provide a broad array of suitable materials. Similar techniques can be used to evaluate solubility of materials desired to be used in the practice of this invention. Working examples of materials found to be useful in the practice of the invention are found elsewhere herein.

As shown in the inventive embodiments disclosed in the Examples below, particularly Example 5, it is possible to control the amount of opioid released from opioid sustained release oral dosage forms when coadministered with aqueous alcohol. In the embodiments described below, aqueous alcohol (e:g. aqueous ethanol) does not result in uncontrolled, immediate release of opioid from embodiments of the dosage forms practiced in the inventive methods. For instance, in Example 5, there are ethyl alcohol concentration dependent increases observed in hydromorphone release rates resulting in slight increase in Cmax and decrease in median Tmax when treatments were administered in the fasted state (the minimum Tmax value was 4 hours with alcohol compared to 6 h with 0% ethanol and the maximum increase in Cmax observed with any individual was 2.5 fold in 40% ethanol treatment compared to 0% ethanol). However, severe dose dumping that would have resulted in a potential fatality did not occur.

In Example 5, plasma opioid (hydromorphone in this case) concentrations were close to limit of quantification at the first timepoint post dose at 2 hours; thereafter plasma concentrations rose slowly in all 4 treatments in both fed and fasted groups. Median Tmax was between 12 and 16 hours and the range of Tmax was similar between treatments in both groups. These data suggest that the controlled release property of the recited dosage forms are maintained in presence of ethanol and there is no 'dose dumping'. The maintenance of controlled release characteristics was consistent with in vitro result of inventive embodiments that are disclosed in Examples 1 & 2 that also did not show dose dumping even with continuous exposure to ethanol over 24 hours.

These data with hydromorphone dosage forms according to the invention are in contrast with results reported for a conventional formulation of hydromorphone known as Palladone® (available from Purdue Pharma). For that product, a significant amount of 'dose dumping' was seen both in vitro and in vivo. In vitro, as seen in Example 3, about 90% of the drug is released within 1 hour in ethanol. In vivo, the maximum fold increase in Cmax for the 4%, 20% and 40% ethanol relative to 0% alcohol was reported to be about 2, 6, and 16, respectively, for an individual subject, and for mean increase across subjects the maximum fold increase in Cmax for the 4%, 20% and 40% ethanol relative to 0% alcohol was reported to be about 1, 2, and 6, respectively. See FDA Alert for Healthcare Professionals entitled "Hydromorphone Hydrochloride Extended-Release Capsules (marketed as Palladone™)" dated July 2005, available at http:llwww.fda.gov/cder/drug/Infosheets/HCP/hydromorphoneHCP.pdf.

Materials that are useful in the practice of this invention are set forth through the present disclosure and in particular in the examples. A variety of materials that are useful for practicing the present invention are disclosed. One interesting point is that OxyContin®, an extended release oxycodone product available from Purdue Pharma LP that is tested below in Example 12, shows minimal signs of dose dumping in the presence of aqueous alcohol. As part of the present invention, it has been discovered that the excipient stearyl alcohol may be responsible for OxyContin's ® resistance to alcohol induced dose dumping. This discovery is evidence of the unexpected nature of the present invention. OxyContin® has been available for many years but the nature of its resistance to alcohol induced dose dumping and the discovery of a potential mechanism for that resistance was unknown until the discovery as set forth herein. Other formulation strategies, besides inclusion of stearyl alcohol, useful in the development of sustained release dosage forms and related methods that provide resistance to aqueous alcohol induced dose dumping may be found elsewhere herein. Certain such embodiments are exemplified in Examples 7-11.

The invention will now be described in more detail below.

### II. Definitions

All percentages are weight percent unless otherwise noted.

All publications cited to herein are incorporated by reference in their entirety and for all purposes as if reproduced fully herein.

The present invention is best understood by reference to the following definitions, the drawings and exemplary disclosure provided herein.

"Administering" or "administration" means providing a drug to a patient in a manner that is pharmacologically useful. "Administering" or "administration" also refers to a patient's co-ingestion of an alcoholic beverage with a dosage form, as well as to the providing of an alcohol-containing aqueous solution to a test subject in connection with in vivo testing of a dosage form.

"Alcohol" means an organic compound, having from 1 to about 5 carbon atoms, in which a hydroxyl group (-OH) is bound to a carbon atom, which in turn is bound to hydrogen and/or carbon atoms. In a preferred embodiment, alcohol comprises ethanol.

"Apparent terminal half-life" (t½) is calculated as 0.693/k, wherein "k" means the apparent elimination rate constant, estimated by linear regression of the log-transformed plasma concentration during the terminal log-linear elimination phase.

"Aqueous alcohol" means a combination comprising water and alcohol. Varying amounts of alcohol may be present in aqueous alcohol. Preferably the aqueous alcohol comprises from about 1 volume/volume percent (v/v%, i.e. volume of alcohol/total volume of aqueous alcohol, expressed as percent) to about 100 v/v% alcohol in aqueous alcohol, more preferably the aqueous alcohol comprises alcohol at concentrations equal to or greater than about 20 v/v%, still more preferably the aqueous alcohol comprises alcohol at concentrations equal to or greater than about 25 v/v%, and yet more preferably the aqueous alcohol comprises alcohol at concentrations equal to or greater than about 40 v/v%.

"Area under the curve" or "AUC" is the area as measured under a plasma drug concentration curve. Often, the AUC is specified in terms of the time interval across which the plasma drug concentration curve is being integrated, for instance AUCstart-finish. Thus, AUC0-48 refers to the AUC obtained from integrating the plasma concentration curve over a period of zero to 48 hours, where zero is conventionally the time of administration of the drug or dosage form comprising the drug to a patient. AUCt refers to area under the plasma concentration curve from hour 0 to the last detectable concentration at time t, calculated by the trapezoidal rule. AUCinf refers to the AUC value extrapolated to infinity, calculated as the sum of AUCt and the area extrapolated to infinity, calculated by the concentration at time t (Ct) divided by k. (If the t½ value was not estimable for a subject, the mean t½ value of that treatment was used to calculate AUCinf.). "Mean, single dose, area under a plasma concentration-time curve AUCinf" means the mean AUCinf obtained over several patients or multiple administrations to the same patient on different occasions with sufficient washout in between dosings to allow drug levels to subside to pre-dose levels following a single administration of a dosage form to each patient.

"C" means the concentration of drug in blood plasma, or serum, of a subject, generally expressed as mass per unit volume, typically nanograms per milliliter. For convenience, this concentration may be referred to herein as "drug plasma concentration", "plasma drug concentration" or "plasma concentration". The plasma drug concentration at any time following drug administration is referenced as Ctime, as in C9h or C24h, etc. A maximum plasma concentration obtained following administration of a dosage form obtained directly from the experimental data without interpolation is referred to as Cmax. The average or mean plasma concentration obtained during a period of interest is referred to as Cavg or Cmean. "Mean, single dose, maximum plasma concentration" means the mean Cmax obtained over several subjects or multiple administrations to the same subject with sufficient washout in between dosings to allow drug levels to subside to pre-dose levels, following a single administration of a dosage form to each subject. "Individual patient single dose maximum plasma concentration" means the Cmax obtained for a single subject upon a single administration with sufficient washout from prior dosings (if any) to allow drug levels to subside to pre-dose levels.

In an embodiment, the inventive method comprises releasing opioid (such as, but not limited to hydromorphone or oxycodone) from the opioid sustained release dosage form, preferably a once-per-day or twice-per-day opioid sustained release dosage form, wherein a ratio of a mean single dose maximum plasma opioid concentration achieved when the opioid sustained release dosage form is coadministered to the patient with the aqueous alcohol to a mean single dose maximum plasma opioid concentration achieved when the opioid sustained release dosage form is administered to a patient without coadministration of the aqueous alcohol is equal to or less than about 1.8:1, more preferably equal to or less than about 1.6:1, and even more preferably equal to or less than about 1.4:1.

In an embodiment, the inventive method comprises releasing opioid (such as, but not limited to hydromorphone or oxycodone) from the opioid sustained release dosage form wherein a ratio of an individual patient single dose maximum plasma opioid concentration achieved when the opioid sustained release dosage form, preferably a once-per-day or twice-per-day opioid sustained release dosage form, is coadministered to the patient with the aqueous alcohol to an individual patient single dose maximum plasma opioid concentration achieved when the opioid sustained release dosage form is administered to a patient without coadministration of the aqueous alcohol is equal to or less than about 5:1, preferably equal to or less than about 4:1, more preferably equal to or less than about 3:1.

"Coadminister," "Coadministration," and "Coadministering" all refer to dosing of two or more substances to a test subject or to or by a patient within a limited period, preferably within 180 minutes, more preferably within 60 minutes, even more preferably within 45 minutes, still more preferably within 30 minutes, and most preferably within 15 minutes. The dosing can be self-dosing as where a patient takes a prescription dosage form and also consumes an alcoholic beverage. Such self-dosing includes consumption of an alcoholic beverage even where the labeling for the prescription dosage form advises against the consumption of alcoholic beverages. More generally, as used herein, "coadminister," "coadministration," and "coadministering" include co-ingestion of two or more substances by a patient or a test subject however performed within a limited period, e.g., the periods referred to above.

"Dosage form" means an opioid in a medium, carrier, vehicle, or device suitable for administration to a patient. "Oral dosage form" means a dosage form suitable for oral administration. In an embodiment, the inventive dosage forms may comprise a sustained release dosing structure for sustained release of the opioid, and optionally an immediate release component for immediate release of the opioid. In an embodiment, dosage forms according to the invention may include, or exclude, opioid antagonists such as naltrexone, naloxone, or other conventional opioid antagonists.

"Dose" means a unit of drug. Conventionally, a dose is provided as a dosage form. Doses may be administered to patients according to a variety of dosing regimens. Common dosing regimens include once per day (qd), twice per day (bid), and thrice per day (tid). Doses of opioid useful in the practice of the present invention range from about 0.001 mg to about 5000 mg, preferably from about 0.01 to about 1000 mg, more preferably from about 0.1 to about 750 mg, still preferably from about 0.5 to about 500 mg, even more preferably from about 0.5 to about 250 mg, even more preferably from about 1 to about 100 mg, and most preferably from about 1 to about 50 mg.

"Immediate-release dosage form" means a dosage form that releases greater than or equal to about 75% of the drug in less than or equal to about 45 minutes following administration of the dosage form to a patient.

"Once per day" (i.e. qd) or "twice per day" (i.e. bid) refers to frequency of dosing according to the inventive methods. For example, one-per-day dosing means dosing generally once in every 24 hours, e.g. qd.

"Opioid" means an agent that binds to opioid receptors found principally in the central nervous system and gastrointestinal tract, and is selected from opium alkaloids and semi-synthetic or wholly synthetic opioids. Examples of opium alkaloids comprise morphine, codeine, and thebaine. Examples of semi-synthetic opioids comprise diamorphine (heroin), oxycodone, hydrocodone, dihydrocodeine, hydromorphone, oxymorphone, and nicomorphine. Examples of wholly synthetic opioids comprise methadone, levomethadyl acetate hydrochloride (LAAM), pethidine (meperidine), ketobemidone, propoxyphene, dextropropoxyphene, dextromoramide, bezitramide, piritramide, pentazocine, and phenazocine. Other opioids are known to one of skill in the art. Preferred opioids in the practice of this invention comprise opioids that are orally bioavailable. More preferred opioids comprise morphine, hydromorphone, hydrocodone, oxymorphone and oxycodone. Opioids, including specific opioids such as hydromorphone, comprise pharmaceutically acceptable salts, and free base or free acid forms of the opioids according to the invention. In embodiments, opioid sustained release oral dosage forms according to the present invention comprise from about 0.01 mg to about 1000 mg of opioid, preferably from about 0.1 mg to about 500 mg of opioid, more preferably from about 0.25 mg to about 300 mg of opioid, still more preferably from about 1 mg to about 100 mg of opioid. It should be noted that the solubility in water and/or aqueous alcohol of opioids according to the invention may vary significantly. In embodiments the amount of opioid in a sustained release dosage form and/or the aqueous alcohol solubility of that opioid may positively or negatively impact the dose dumping performance in aqueous alcohol of sustained release dosage forms and/or related methods according to the invention. For instance, in certain embodiments, large amounts of a highly aqueous alcohol soluble opioid and/or opioid form might increase the likelihood of aqueous alcohol-induced dose dumping. Conversely, in certain embodiments, large amounts of a highly aqueous alcohol insoluble opioid and/or opioid form might decrease the likelihood of aqueous alcohol-induced dose dumping.

"Oral sustained release dosing structure" means a structure suitable for oral administration to a patient comprising one or more drugs, wherein the structure operates to sustainably release the drug(s). "Osmotic oral sustained release dosing structure" means an oral sustained release dosing structure wherein the structure operates via an osmotic mechanism to sustainably release the drug(s).

"Patient" means an animal, preferably a mammal, more preferably a human, in need of therapeutic intervention. The word "patient" is also used herein to refer to test subjects which participate in in vivo testing of dosage forms even though such test subjects are healthy individuals who are not in need of therapeutic intervention. These latter uses of the word "patient" will be evident from the context and the well-known fact that alcohol is not coadministered with opioids to individuals in need of therapeutic intervention.

"Pharmaceutically acceptable salt" means any salt whose anion does not contribute significantly to the toxicity or pharmacological activity of the salt, and, as such, they are the pharmacological equivalents of the base of the drug. Suitable pharmaceutically acceptable salts include acid addition salts which may, for example, be formed by reacting the drug compound with a suitable pharmaceutically acceptable acid such as hydrochloric acid, sulfuric acid, fumaric acid, maleic acid, succinic acid, acetic acid, benzoic acid, citric acid, tartaric acid, carbonic acid or phosphoric acid.

Thus, representative pharmaceutically acceptable salts include, but are not limited to, the following: acetate, benzenesulfonate, benzoate, bicarbonate, bisulfate, bitartrate, borate, bromide, calcium edetate, camsylate, carbonate, chloride, clavulanate, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, oleate, pamoate (embonate), palmitate, pantothenate, phosphate/diphosphate, polygalacturonate, salicylate, stearate, sulfate, subacetate, succinate, tannate, tartrate, teoclate, tosylate, triethiodide and valerate.

"Plasma drug concentration curve" or "drug plasma concentration curve", or "plasma concentration curve" or "plasma profile" or "plasma concentration profile" refer to the curve obtained by plotting plasma drug concentration or drug plasma concentration, or plasma concentration versus time. Usually, the convention is that the zero point on the time scale (conventionally on the x axis) is the time of administration of the drug or dosage form comprising the drug to a patient.

"Prolonged period of time" means a continuous period of time of greater than about 2 hours, preferably, greater than about 4 hours, more preferably, greater than about 8 hours, more preferably greater than about 10 hours, more preferably still, greater than about 14 hours, most preferably, greater than about 14 hours and up to about 24 hours.

"Rate of release" or "release rate" means the quantity of a drug released from a dosage form per unit time, e.g., milligrams of drug released per hour (mg/hr). Drug release rates for dosage forms may be measured as an in vitro rate of drug release, i.e., a quantity of drug released from the dosage form per unit time measured under appropriate conditions and in a suitable test media.

In a preferable embodiment, the release rates referred to herein may be determined by placing a dosage form to be tested in de-ionized water in metal coil or metal cage sample holders attached to a USP Type VII bath indexer in a constant temperature water bath at 37C. Aliquots of the release rate solutions, collected at preset intervals, are then injected into a chromatographic system fitted with an ultraviolet or refractive index detector to quantify the amounts of drug released during the testing intervals. In other embodiments, other conventionally known and used in vitro release rate tests may also be used in the practice of this invention, such as use of a USP Type II apparatus, e.g. the Distek Premiere® 5100.

In an embodiment, the inventive opioid sustained release dosage form releases less than or equal to about 80 weight percent, preferably less than or equal to about 70 weight percent, more preferably less than or equal to about 60 weight percent, even more preferably less than or equal to about 50 weight percent, still more preferably less than or equal to about 40 weight percent, and most preferably less than or equal to about 25 weight percent of the dose of opioid from the opioid sustained release dosage form as measured using an in vitro test method in a specified time in the presence of a specified amount of alcohol. In a preferable embodiment, the in vitro test method, such as the in vitro test methods disclosed herein, or other conventional in vitro test methods, comprises test media into which the opioid sustained release dosage form is placed during the test period. In an embodiment, the amount of opioid released from the inventive opioid sustained release dosage form is measured for a specific period, preferably for a period of about 24 hours following initiation of the in vitro test method, more preferably for a period of about 12 hours following initiation of the in vitro test method, and even more preferably for a period of about 2 hours following initiation of the in vitro test method.

In an embodiment, the test media comprises aqueous alcohol that comprises alcohol. In a preferable embodiment, the test media comprises aqueous alcohol that comprises alcohol at concentrations equal to or greater than about 20% volume/volume (volume of alcohol/total volume of test media), preferably equal to or greater than about 25% volume/volume, more preferably equal to or greater than about 30% volume/volume, still more preferably equal to or greater than about 35% volume/volume, and most preferably equal to or greater than about 40% volume/volume.

"Sustained release" or "sustainably releasing" means continuous release or continuously releasing of a drug or a dose of a drug over a prolonged period of time.

"Sustained release dosing structure" means one or more physical elements that provide for sustained release of a drug or a dose of a drug.

"Sustained release dosage form" means a type of dosage form that provides sustained release of a drug or a dose of a drug.

"Median, single dose, time to maximum plasma concentration Tmax" is the median, obtained over several subjects or multiple administrations to the same subject with sufficient washout in between dosings to allow drug levels to subside to pre-dose levels, of the elapsed time from administration to a subject of a dosage form comprising a drug to the time at which the Cmax for that drug is obtained, following a single administration of the dosage form to each subject and obtained directly from the experimental data without interpolation. In an embodiment, a ratio of the median single dose, time to maximum plasma concentration achieved when the dosage form is coadministered to the subj ect with the aqueous alcohol to the median single dose, time to maximum plasma concentration achieved when the opioid sustained release dosage form is administered to a subject without coadministration of the aqueous alcohol ranges from about 0.5 to about 1.0, preferably from about 0.6 to about 1.0, more preferably from about 0.7 to about 1.0, most preferably from about 0.75 to about 1.0.

"Therapeutically effective amount" means that amount of drug that elicits the biological or medicinal response in a tissue system, animal or human that is being sought by a researcher, veterinarian, medical doctor or other clinician, which includes alleviation of the symptoms of the disease or disorder being treated.

### III. Dosage Forms

In embodiments, the inventive sustained release dosage forms are formulated into dosage forms administrable to patients in need thereof. Sustained release dosage forms and methods of treatment using the sustained release dosage forms will now be described. It will be appreciated that the sustained release dosage forms described below are merely exemplary.

A variety of sustained release dosage forms are suitable for use in the present invention. In certain embodiments, the dosage form is orally administrable and is sized and shaped as a conventional tablet or capsule. Orally administrable dosage forms may be manufactured according to one of various different approaches. For example, the dosage form may be manufactured as a diffusion system, such as a reservoir device or matrix device, a dissolution system, such as encapsulated dissolution systems (including, for example, "tiny time pills", and beads), a matrix.dissolution system, a combination diffusion/dissolution system, or an ion-exchange resin system, as described in Pharmaceutical Sciences, Remington, 18th Ed., pp. 1676-1686 (1990), Mack Publishing Co.; The Pharmaceutical and Clinical Pharmacokinetics, 3rd Ed., pp. 1-28 (1984), Lea and Febreger, Philadelphia.

Osmotic dosage forms in general utilize osmotic pressure to generate a driving force for imbibing fluid into a compartment formed, at least in part, by a semipermeable membrane that permits free diffusion of fluid but not drug or osmotic agent(s), if present. A significant advantage to osmotic systems is that operation is pH-independent and thus continues at the osmotically determined rate throughout an extended time period even as the dosage form transits the gastrointestinal tract and encounters differing microenvironments having significantly different pH values. A review of such dosage forms is found in Santus and Baker, "Osmotic drug delivery: a review of the patent literature," Journal of Controlled Release 35 (1995) 1-21, incorporated by reference herein. U.S. Patents Nos. 3,845,770; 3,916,899; 3,995,631; 4,008,719; 4,111,202; 4,160,020; 4,327,725; 4,578,075; 4,681,583; 5,019,397; and 5,156,850 disclose osmotic devices for the continuous dispensing of active agent.

Osmotic sustained release dosage forms in which a drug composition is delivered as a slurry, suspension or solution from a small exit orifice by the action of an expandable layer are disclosed in U.S. Patents Nos. 5,633,011; 5,190,765; 5,252,338; 5,620,705; 4,931,285; 5,006,346; 5,024,842; and 5,160,743, which are incorporated herein by reference. Typical devices include an expandable push layer and a drug layer surrounded by a semipermeable membrane. In certain instances, the drug layer is provided with a subcoat to delay release of the drug composition to the environment of use or to form an annealed coating in conjunction with the semipermeable membrane. In an embodiment, further protection from dose dumping can be had by applying an enteric coat, preferably one that is insoluble in aqueous alcohol and does not swell in aqueous alcohol and gastric pH, to the osmotic sustained release dosage form. To protect the semi-permeable membrane, a film of hydrophilic (such as polyvinyl alcohol) or hydrophobic material may be coated over the semi-permeable membrane. If the layer allows less ethanol to contact the semi-permeable membrane, swelling of the semi-permeable membrane may be avoided or minimized.

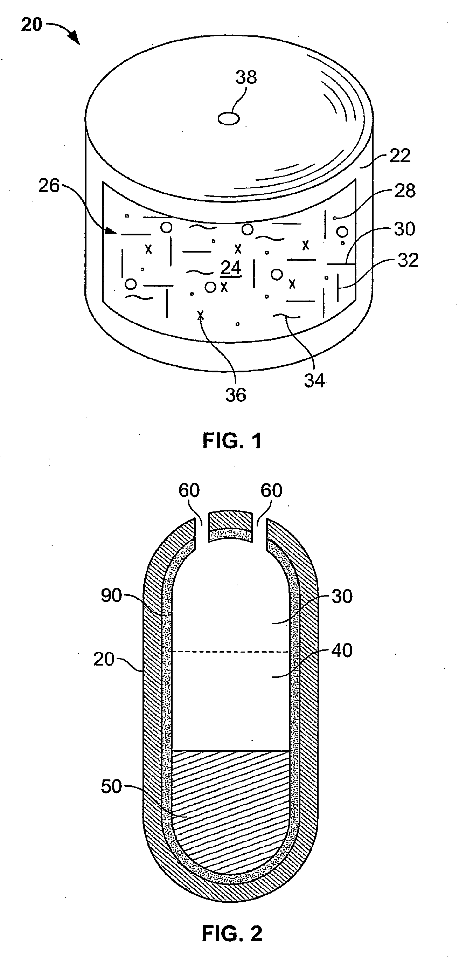

An exemplary dosage form, referred to in the art as an elementary osmotic pump dosage form, is shown in Figure 1. Dosage form 20, shown in a cutaway view, is also referred to as an elementary osmotic pump (EOP), and is comprised of a semi-permeable membrane 22 that surrounds and encloses an internal compartment 24. The internal compartment contains a single component layer referred to herein as a drug layer 26, comprising an inventive substance 28 in an admixture with selected excipients. The excipients are adapted to provide an osmotic activity gradient for attracting fluid from an external environment through membrane 22 and for forming a deliverable complex formulation upon imbibition of fluid. The excipients may include a suitable suspending agent, also referred to herein as drug carrier 30, a binder 32, a lubricant 34, and an osmotically active agent referred to as an osmagent 36. Exemplary materials useful for these components can be found disclosed throughout the present application.

Semi-permeable membrane 22 of the osmotic dosage form is permeable to the passage of an external fluid, such as water and biological fluids, but is substantially impermeable to the passage of components in the internal compartment. Materials useful for forming the membrane are essentially nonerodible and are substantially insoluble in biological fluids during the life of the dosage form. Representative polymers for forming the semi-permeable membrane include homopolymers and copolymers, such as, cellulose esters, cellulose ethers, and cellulose ester-ethers. Flux-regulating agents can be admixed with the membrane-forming material to modulate the fluid permeability of the membrane. For example, agents that produce a marked increase in permeability to fluid such as water are often essentially hydrophilic, while those that produce a marked permeability decrease to water are essentially hydrophobic. Exemplary flux regulating agents include polyhydric alcohols, polyalkylene glycols, polyalkylenediols, polyesters of alkylene glycols, and the like.

In operation, the osmotic gradient across membrane 22 due to the presence of osmotically-active agents causes gastric fluid to be imbibed through the membrane, swelling of the drug layer, and formation of a deliverable complex formulation (e.g., a solution, suspension, slurry or other flowable composition) within the internal compartment. The deliverable inventive substance formulation is released through an exit 38 as fluid continues to enter the internal compartment. Even as drug formulation is released from the dosage form, fluid continues to be drawn into the internal compartment, thereby driving continued release. In this manner, the inventive substance is released in a sustained and continuous manner over a prolonged period.

Figure 2 illustrates certain inventive embodiments of sustained release dosage forms. Dosage forms of this type are described in detail in U.S. Patent Nos.: 4,612,008; 5,082,668; and 5,091,190; and are further described below.

Figure 2 shows an embodiment of one type of sustained release dosage form, namely the osmotic sustained release dosage form. First drug layer 30 comprises osmotically active components, and a lower amount of opioid than in second drug layer 40. The osmotically active component(s) in the first component drug layer comprises an osmagent such as salt and one or more osmopolymer(s) having relatively small molecular weights which exhibit swelling as fluid is imbibed such that release of these osmopolymers through exit 60 occurs similar to that of drug layer 40. Additional excipients such as binders, lubricants, antioxidants and colorants may also be included in first drug layer 30.

Second drug layer 40 comprises opioid in an admixture with selected excipients adapted to provide an osmotic activity gradient for driving fluid from an external environment through membrane 20 and for forming a deliverable drug formulation upon imbibition of fluid. The excipients may include a suitable suspending agent, also referred to herein as a drug carrier, but no osmotically active agent, "osmagent," such as salt, sodium chloride. It has been discovered that the omission of salt from this second drug layer, which contains a higher proportion of the overall drug in the dosage form, in combination with the salt in the first drug layer, provides an improved ascending rate of release creating a longer duration of ascending rate.

Drug layer 40 has a higher concentration of opioid than does drug layer 30. The ratio of the concentration of opioid in the first drug layer 30 to the concentration of opioid in the second drug layer 40 is preferably maintained at less than 1 and preferably less than or equal to about 0.43 to provide the desired substantially ascending rate of release.

Drug layer 40 may also comprise other excipients such as lubricants, binders, etc.

Drug layer 40, as with drug layer 30, further comprises a hydrophilic polymer carrier. The hydrophilic polymer contributes to the controlled delivery of the opioid. Representative examples of these polymers are poly(alkylene oxide) of 100,000 to 750,000 number-average molecular weight, including poly(ethylene oxide), poly(methylene oxide), poly(butylene oxide) and poly(hexylene oxide); and a poly(carboxymethylcellulose) of 40,000 to 400,000 number-average molecular weight, represented by poly(alkali carboxymethylcellulose), poly(sodium carboxymethylcellulose), poly(potassium carboxymethylcellulose) and poly(lithium carboxymethylcellulose). Drug layer 40 can further comprise a hydroxypropylalkylcellulose of 9,200 to 125,000 number-average molecular weight for enhancing the delivery properties of the dosage form as represented by hydroxypropylethylcellulose, hydroxypropylmethylcellulose, hydroxypropylbutylcellulose and hydroxypropylpentylcellulose; and a poly(vinylpyrrolidone) of 7,000 to 75,000 number-average molecular weight for enhancing the flow properties of the dosage form. Preferred among these polymers are the poly(ethylene oxide) of 100,000 - 300,000 number average molecular weight. Carriers that erode in the gastric environment, i.e., bioerodible carriers, are especially preferred.

Other carriers that may be incorporated into drug layer 40, and/or drug layer 30, include carbohydrates that exhibit sufficient osmotic activity to be used alone or with other osmagents. Such carbohydrates comprise monosaccharides, disaccharides and polysaccharides. Representative examples include maltodextrins (i.e., glucose polymers produced by the hydrolysis of corn starch) and the sugars comprising lactose, glucose, raffinose, sucrose, mannitol, sorbitol, and the like. Preferred maltodextrins are those having a dextrose equivalence (DE) of 20 or less, preferably with a DE ranging from about 4 to about 20, and often 9-20. Maltodextrin having a DE of 9-12 has been found to be useful.

Drug layer 40 and drug layer 30 typically will be a substantially dry, <1% water by weight, composition formed by compression of the cairier, the opioid, and other excipients as one layer.

Drug layer 40 may be formed from particles by comminution that produces the size of the drug and the size of the accompanying polymer used in the fabrication of the drug layer, typically as a core containing the compound, according to the mode and the manner of the invention. The means for producing particles include granulation, spray drying, sieving, lyophilization, crushing, grinding, jet milling, micronizing and chopping to produce the intended micron particle size.

The process can be performed by size reduction equipment, such as a micropulverizer mill, a fluid energy grinding mill, a grinding mill, a roller mill, a hammer mill, an attrition mill, a chaser mill, a ball mill, a vibrating ball mill, an impact pulverizer mill, a centrifugal pulverizer, a coarse crusher and a fine crusher. The size of the particle can be ascertained by screening, including a grizzly screen, a flat screen, a vibrating screen, a revolving screen, a shaking screen, an oscillating screen and a reciprocating screen. The processes and equipment for preparing drug and carrier particles are disclosed in Pharmaceutical Sciences, Remington, 17th Ed., pp. 1585-1594 (1985); Chemical Engineers Handbook, Perry, 6th Ed., pp. 21-13 to 21-19 (1984); Journal of Pharmaceutical Sciences, Parrot, Vol. 61, No. 6, pp. 813-829 (1974); and Chemical Engineer, Hixon, pp. 94-103 (1990).

First drug layer 30 comprises active agent in an admixture with selected excipients adapted to provide an osmotic activity gradient for driving fluid from an external environment through membrane 20 and for forming a deliverable drug formulation upon imbibition of fluid. The excipients may include a suitable suspending agent, also referred to herein as a drug carrier, and an osmotically active agent, i.e., an "osmagent," such as salt. Other excipients such as lubricants, binders, etc. may also be included. It has been surprisingly found that when first component drug layer 30 comprises an osmotically active component, and a lower amount of active drug than in second component drug layer 40, an improved ascending rate of release can be created that provides a longer duration of ascending rate.

The osmotically active component in the first drug layer typically comprises an osmagent and one or more osmopolymer(s) having relatively small molecular weights which exhibit swelling as fluid is imbibed such that release of these osmopolymers through exit 60 occurs similar to that of drug layer 40.

The ratio of opioid concentration between the first drug layer and the second drug layer alters the release rate slope. Release rate slope is calculated as the difference between the maximum release rate and the release rate achieved at the first time point after start-up (for example, at 6 hours), divided by the average release rate calculated between these two time points.

Drug layer 30 and drug layer 40 may optionally contain surfactants and disintegrants in both drug layers. Exemplary of the surfactants are those having an HLB value of about 10 - 25, such as polyethylene glycol 400 monostearate, polyoxyethylene-4-sorbitan monolaurate, polyoxyethylene-20-sorbitan monooleate, polyoxyethylene-20-sorbitan monopalmitate, polyoxyethylene-20-monolaurate, polyoxyethylene-40 -stearate, sodium oleate and the like.

Disintegrants may be selected from starches, clays, celluloses, algins and gums and crosslinked starches, celluloses and polymers. Representative disintegrants include corn starch, potato starch, croscarmelose, crospovidone, sodium starch glycolate, Veegum HV, methylcellulose, agar, bentonite, carboxymethylcellulose, alginic acid, guar gum and the like.

Membrane 20 is formed to be permeable to the passage of an external fluid, such as water and biological fluids, and is substantially impermeable to the passage of opioid, osmagent, osmopolymer and the like. As such, it is semipermeable. The selectively semipermeable compositions used for forming membrane 20 are essentially nonerodible and substantially insoluble in biological fluids during the life of the dosage form.

Representative polymers for forming membrane 20 comprise semipermeable homopolymers, semipermeable copolymers, and the like. In one presently preferred embodiment, the compositions can comprise cellulose esters, cellulose ethers, and cellulose ester-ethers. The cellulosic polymers typically have a degree of substitution, "D.S.", on their anhydroglucose unit from greater than 0 up to 3 inclusive. By degree of substitution is meant the average number of hydroxyl groups originally present on the anhydroglucose unit that are replaced by a substituting group, or converted into another group. The anhydroglucose unit can be partially or completely substituted with groups such as acyl, alkanoyl, alkenoyl, aroyl, alkyl, alkoxy, halogen, carboalkyl, alkylcarbamate, alkylcarbonate, alkylsulfonate, alkylsulfamate, semipermeable polymer forming groups, and the like. The semipermeable compositions typically include a member selected from the group consisting of cellulose acylate, cellulose diacylate, cellulose triacylate, cellulose triacetate, cellulose acetate, cellulose diacetate, cellulose triacetate, mono-, di- and tri-cellulose alkanylates, mono-, di-, and tri-alkenylates, mono-, di-, and tri-aroylates, and the like.

Exemplary polymers can include, for example, cellulose acetate have a D.S. of 1.8 to 2.3 and an acetyl content of 32 to 39.9%; cellulose diacetate having a D.S. of 1 to 2 and an acetyl content of 21 to 35%, cellulose triacetate having a D.S. of 2 to 3 and an acetyl content of 34 to 44.8%, and the like. More specific cellulosic polymers include cellulose propionate having a D.S. of 1.8 and a propionyl content of 38.5%; cellulose acetate propionate having an acetyl content of 1.5 to 7% and an acetyl content of 39 to 42%; cellulose acetate propionate having an acetyl content of 2.5 to 3%, an average propionyl content of 39.2 to 45%, and a hydroxyl content of 2.8 to 5.4%; cellulose acetate butyrate having a D.S. of 1.8, an acetyl content of 13 to 15%, and a butyryl content of 34 to 39%; cellulose acetate butyrate having an acetyl content of 2 to 29%, a butyryl content of 17 to 53%, and a hydroxyl content of 0.5 to 4.7%; cellulose triacylates having a D.S. of 2.6 to 3 such as cellulose trivalerate, cellulose trilamate, cellulose tripalmitate, cellulose trioctanoate, and cellulose tripropionate; cellulose diesters having a D.S. of 2.2 to 2.6 such as cellulose disuccinate, cellulose dipalmitate, cellulose dioctanoate, cellulose dicarpylate, and the like; mixed cellulose esters such as cellulose acetate valerate, cellulose acetate succinate, cellulose propionate succinate, cellulose acetate octanoate, cellulose valerate palmitate, cellulose acetate heptonate, and the like. Semipermeable polymers are known in U.S. Pat. No. 4,077,407 and they can be synthesized by procedures described in Encyclopedia of Polymer Science and Technology, Vol. 3, pages 325 to 354, 1964, published by Interscience Publishers, Inc., New York.

Additional semipermeable polymers for forming the semipermeable membrane can comprise, for example, cellulose acetaldehyde dimethyl acetate; cellulose acetate ethylcarbamate; cellulose acetate methylcarbamate; cellulose dimethylaminoacetate; semipermeable polyamide; semipermeable polyurethanes; semipermeable sulfonated polystyrenes; cross-linked selectively semipermeable polymers formed by the coprecipitation of a polyanion and a polycation as disclosed in U.S. Pat. Nos. 3,173,876; 3,276,586; 3,541,005; 3,541,006; and 3,546,142; semipermeable polymers as disclosed in U.S. Pat. No. 3,133,132; semipermeable polystyrene derivatives; semipermeable poly (sodium styrenesulfonate); semipermeable poly (vinylbenzyltremethylammonium chloride); semipermeable polymers, exhibiting a fluid permeability of 10-5 to 10-2 (cc. mil/cm hr.atm) expressed as per atmosphere of hydrostatic or osmotic pressure differences across a semipermeable membrane. The polymers are known to the art in U.S. Pat. Nos. 3,845,770; 3,916,899; and 4,160,020; and in Handbook of Common Polymers, by Scott, J. R., and Roff, W. J., 1971, published by CRC Press, Cleveland. Ohio.

Membrane 20 may also comprise a flux-regulating agent. The flux regulating agent is a compound added to assist in regulating the fluid permeability or flux through the membrane 20. The flux regulating agent can be a flux enhancing agent or a decreasing agent. The agent can be preselected to increase or decrease the liquid flux. Agents that produce a marked increase in permeability to fluids such as water are often essentially hydrophilic, while those that produce a marked decrease to fluids such as water are essentially hydrophobic. The amount of regulator in membrane 20 when incorporated therein generally is from about 0.01 % to 20% by weight or more. The flux regulator agents in one embodiment that increase flux include, for example, polyhydric alcohols, polyalkylene glycols, polyalkylenediols, polyesters of alkylene glycols, and the like. Typical flux enhancers include polyethylene glycol 300, 400, 600, 1500, 4000, 6000, poly(ethylene glycol-co-propylene glycol), and the like; low molecular weight gylcols such as polypropylene glycol, polybutylene glycol and polyamylene glycol: the polyalkylenediols such as poly(1,3-propanediol), poly(1,4-butanediol), poly(1,6-hexanediol), and the like; aliphatic diols such as 1,3-butylene glycol, 1,4-pentamethylene glycol, 1,4-hexamethylene glycol, and the like; alkylene triols such as glycerine, 1,2,3-butanetriol, 1,2,4-hexanetriol, 1,3,6-hexanetriol and the like; esters such as ethylene glycol dipropionate, ethylene glycol butyrate, butylene glucol dipropionate, glycerol acetate esters, and the like. Representative flux decreasing agents include, for example, phthalates substituted with an alkyl or alkoxy or with both an alkyl and alkoxy group such as diethyl phthalate, dimethoxyethyl phthalate, dimethyl phthalate, and [di(2-ethylhexyl)phthalate], aryl phthalates such as triphenyl phthalate, and butyl benzyl phthalate; insoluble salts such as calcium sulphate; barium sulphate, calcium phosphate, and the like; insoluble oxides such as titanium oxide; polymers in powder, granule and like form such as polystyrene, polymethylmethacrylate, polycarbonate, and polysulfone; esters such as citric acid esters esterfied with long chain alkyl groups; inert and substantially water impermeable fillers; resins compatible with cellulose based membrane forming materials, and the like.

Other materials that can be used to form membrane 20 for imparting flexibility and elongation properties to the wall, for making the membrane less-to-nonbrittle and to render greater tear strength, include, for example, phthalate plasticizers such as dibenzyl phthalate, dihexyl phthalate, butyl octyl phthalate, straight chain phthalates of six to eleven carbons, di-isononyl phthalte, di-isodecyl phthalate, and the like. The plasticizers include nonphthalates such as triacetin, dioctyl azelate, epoxidized tallate, tri-isoctyl trimellitate, tri-isononyl trimellitate, sucrose acetate isobutyrate, epoxidized soybean oil, and the like. The amount of plasticizer in a membrane when incorporated therein is about 0.01 % to 20% weight, or higher.

Push layer 50 comprises an expandable layer in contacting layered arrangement with the second drug layer 40 as illustrated in Figure 2. Push layer 50 comprises a polymer that imbibes an aqueous or biological fluid and swells to push the drug composition through the exit of the device.

The expandable layer comprises in one embodiment a hydroactivated composition that swells in the presence of water, such as that present in gastric fluids. Conveniently, it can comprise an osmotic composition comprising an osmotic solute that exhibits an osmotic pressure gradient across the semipermeable membrane against an external fluid present in the environment of use. In another embodiment, the hydro-activated layer comprises a hydrogel that imbibes and/or absorbs fluid into the layer through the outer semipermeable membrane. The semipermeable membrane is non-toxic. It maintains its physical and chemical integrity during operation and it is essentially free of interaction with the expandable layer.

The expandable layer in one preferred embodiment comprises a hydroactive layer comprising a hydrophilic polymer, also known as an osmopolymer. The osmopolymers exhibit fluid imbibition properties. The osmopolymers are swellable, hydrophilic polymers, which osmopolymers interact with water and biological aqueous fluids and swell or expand to an equilibrium state. The osmopolymers exhibit the ability to swell in water and biological fluids and retain a significant portion of the imbibed fluid within the polymer structure. The osmopolymers swell or expand to a very high degree, usually exhibiting a 2 to 50 fold volume increase. The osmopolymers can be non-cross-linked or cross-linked. The swellable, hydrophilic polymers are in one embodiment lightly cross-linked, such cross-links being formed by covalent or ionic bonds or residue crystalline regions after swelling. The osmopolymers can be of plant, animal or synthetic origin.

The osmopolymers are hydrophilic polymers. Hydrophilic polymers suitable for the present purpose include poly (hydroxy-alkyl methacrylate) having a molecular weight of from 30,000 to 5,000,000; poly (vinylpyrrolidone) having a molecular weight of from 10,000 to 360,000; anionic and cationic hydrogels; polyelectrolytes complexes; poly (vinyl alcohol) having a low acetate residual, cross-linked with glyoxal, formaldehyde, or glutaraldehyde and having a degree of polymerization of from 200 to 30,000; a mixture of methyl cellulose, cross-linked agar and carboxymethyl cellulose; a mixture of hydroxypropyl methylcellulose and sodium carboxymethylcellulose; a mixture of hydroxypropyl ethylcellulose and sodium carboxymethyl cellulose, a mixture of sodium carboxymethylcellulose and methylcellulose, sodium carboxymethylcellulose; potassium carboxymethylcellulose; a water insoluble, water swellable copolymer formed from a dispersion of finely divided copolymer of maleic anhydride with styrene, ethylene, propylene, butylene or isobutylene crosslinked with from 0.001 to about 0.5 moles of saturated cross-linking agent per mole of maleic anhydride per copolymer; water swellable polymers of N-vinyl lactams; polyoxyethylene-polyoxypropylene gel; carob gum; polyacrylic gel; polyester gel; polyuria gel; polyether gel, polyamide gel; polycellulosic gel; polygum gel; initially dry hydrogels that imbibe and absorb water which penetrates the glassy hydrogel and lowers its glass temperature; and the like.

Representative of other osmopolymers are polymers that form hydrogels such as Carbopol™. acidic carboxypolymer, a polymer of acrylic acid cross-linked with a polyallyl sucrose, also known as carboxypolymethylene, and carboxyvinyl polymer having a molecular weight of 250,000 to 4,000,000; Cyanamer™ polyacrylamides; cross-linked water swellable indenemaleic anhydride polymers; Good-rite™ polyacrylic acid having a molecular weight of 80,000 to 200,000; Polyox™ polyethylene oxide polymer having a molecular weight of 100,000 to 5,000,000 and higher; starch graft copolymers; Aqua-Keeps™ acrylate polymer polysaccharides composed of condensed glucose units such as diester cross-linked polygluran; and the like. Representative polymers that form hydrogels are known to the prior art in U.S. Pat. No. 3,865,108; U.S. Pat. No. 4,002,173; U.S. Pat. No. 4,207,893; and in Handbook of Common Polymers, by Scott and Roff, published by the Chemical Rubber Co., Cleveland, Ohio. The amount of osmopolymer comprising a hydro-activated layer can be from about 5% to 100%.

The expandable layer in another manufacture can comprise an osmotically effective compound that comprises inorganic and organic compounds that exhibit an osmotic pressure gradient across a semipermeable membrane against an external fluid. The osmotically effective compounds, as with the osmopolymers, imbibe fluid into the osmotic system, thereby making available fluid to push against the inner wall, i.e., in some embodiments, the barrier layer and/or the membrane of the soft or hard capsule for pushing active agent from the dosage form. The osmotically effective compounds are known also as osmotically effective solutes, and also as osmagents. Osmotically effective solutes that can be used comprise magnesium sulfate, magnesium chloride, potassium sulfate, sodium sulfate, lithium sulfate, potassium acid phosphate, mannitol, urea, inositol, magnesium succinate, tartaric acid, carbohydrates such as raffinose, sucrose, glucose, lactose, sorbitol, and mixtures therefore. The amount of osmagent can be from about 5% to 100% of the weight of the layer. The expandable layer optionally comprises an osmopolymer and an osmagent with the total amount of osmopolymer and osmagent equal to 100%. Osmotically effective solutes are known to the prior art as described in U.S. Pat. No. 4,783,337.

Protective subcoat, inner wall 90, is permeable to the passage of fluid entering the compartment defined by membrane 20. Wall 90 provides a lubricating function that facilitates the movement of first drug layer 30, second drug layer 40 and push layer 50 toward exit 60. Wall 90 may be formed from hydrophilic materials and excipients. Wall 90 promotes release of the drug composition from the compartment and reduces the amount of residual drug composition remaining in the compartment at the end of the delivery period, particularly when the slurry, suspension or solution of the drug composition that is being dispensed is highly viscous during the period of time in which it is being dispensed. In dosage forms with hydrophobic agents and no inner wall, it has been observed that significant residual amounts of drug may remain in the device after the period of delivery has been completed. In some instances, amounts of 20% or greater may remain in the dosage form at the end of a twenty-four hour period when tested in a release rate assay. Particularly in the case of active compounds having a high cost, such an improvement presents substantial economic advantages since it is not necessary to load the drug layer with an excess of drug to insure that the minimum amount of drug required will be delivered. Inner membrane 90 may be formed as a coating applied over the compressed core.

Wall 90 typically may be 0.01 to 5 mm thick, more typically 0.5 to 5mm thick, and it comprises a member selected from hydrogels, gelatin, low molecular weight polyethylene oxides, e.g., less than 100,000 MW, hydroxyalkylcelluloses, e.g., hydroxyethylcellulose, hydroxypropylcellulose, hydroxyisopropylcelluose, hydroxybutylcellulose and hydroxyphenylcellulose, and hydroxyalkyl alkylcelluloses, e.g., hydroxypropyl methylcellulose, and mixtures thereof. The hydroxyalkylcelluloses comprise polymers having a 9,500 to 1,250,000 number-average molecular weight. For example, hydroxypropyl celluloses having number average molecular weights of 80,000 to 850,000 are useful. The wall 90 may be prepared from conventional solutions or suspensions of the aforementioned materials in aqueous solvents or inert organic solvents.

Preferred materials for the wall 90 include hydroxypropyl cellulose, hydroxyethyl cellulose, hydroxypropyl methyl cellulose, povidone [poly(vinylpyrrolidone)], polyethylene glycol, and mixtures thereof.

Most preferred are mixtures of hydroxypropyl cellulose and povidone, prepared in organic solvents, particularly organic polar solvents such as lower alkanols having 1-8 carbon atoms, preferably ethanol, mixtures of hydroxyethyl cellulose and hydroxypropyl methyl cellulose prepared in aqueous solution; and mixtures of hydroxyethyl cellulose and polyethylene glycol prepared in aqueous solution. Most preferably, the wall 90 comprises a mixture of hydroxypropyl cellulose and providone prepared in ethanol.

It is preferred that wall 90 comprises between about 50% and about 90% hydroxypropylcellulose identified as EF having an average molecular weight of about 80,000 and between about 10% and about 50% polyvinylpyrrolidone identified as K29-32.

Conveniently, the weight of the wall 90 applied to the compressed core may be correlated with the thickness of the wall 90 and residual drug remaining in a dosage form in a release rate assay such as described herein. As such, during manufacturing operations, the thickness of the wall 90 may be controlled by controlling the weight of the wall 90 taken up in the coating operation.

When wall 90 is formed as a subcoat, i.e., by coating onto the tableted composite including one or all of the first drug layer, second drug layer and push layer, the wall 90 can fill in surface irregularities formed on the core by the tableting process. The resulting smooth external surface facilitates slippage between the coated composite core and the semipermeable membrane during dispensing of the drug, resulting in a lower amount of residual drug composition remaining in the device at the end of the dosing period. When wall 90 is fabricated of a gel-forming material, contact with water in the environment of use facilitates formation of the gel or gel-like inner coat having a viscosity that may promote and enhance slippage between membrane 20 and drug layer 30 and drug layer 40.

Pan coating may be conveniently used to provide the completed dosage form, except for the exit orifice. In the pan coating system, the wall-forming composition for the wall or the membrane, as the case may be, is deposited by successive spraying of the appropriate membrane composition onto the compressed trilayered or multilayered core comprising the drug layers, optional barrier layer and push layer, accompanied by tumbling in a rotating pan. A pan coater is used because of its availability at commercial scale. Other techniques can be used for coating the compressed core. Once coated, the membrane is dried in a forced-air oven or in a temperature and humidity controlled oven to free the dosage form of solvent(s) used in the manufacturing. Drying conditions will be conventionally chosen on the basis of available equipment, ambient conditions, solvents, coatings, coating thickness, and the like.

Other coating techniques can also be employed. For example, the membrane or walls of the dosage form may be formed in one technique using the air-suspension procedure. This procedure consists of suspending and tumbling the compressed core in a current of air and the semipermeable membrane forming composition, until the membrane is applied to the core. The air-suspension procedure is well suited for independently forming the membrane of the dosage form. The air-suspension procedure is described in U.S. Patent No. 2,799,241; in J. Am. Pharm. Assoc., Vol. 48, pp. 451-459 (1959); and, ibid., Vol. 49, pp. 82-84 (1960). The dosage form also can be coated with a Wurster® air-suspension coater using, for example, methylene dichloride methanol as a cosolvent for the membrane forming material. An Aeromatic® air-suspension coater can be used employing a cosolvent.

In an embodiment, the sustained release dosage form of the invention is provided with at least one exit 60 as shown in Figure 2. Exit 60 cooperates with the compressed core for the uniform release of drug from the dosage form. The exit can be provided during the manufacture of the dosage form or during drug delivery by the dosage form in a fluid environment of use.

One or more exit orifices are drilled in the drug layer end of the dosage form, and optional water soluble overcoats, which may be colored (e.g., Opadry colored coatings) or clear (e.g., Opadry Clear), may be coated on the dosage form to provide the finished dosage form.

Exit 60 may include an orifice that is formed or formable from a substance or polymer that erodes, dissolves or is leached from the outer membrane to thereby form an exit orifice. The substance or polymer may include, for example, an erodible poly(glycolic) acid or poly(lactic) acid in the semipermeable wall; a gelatinous filament; a water-removable poly(vinyl alcohol); a leachable compound, such as a fluid removable pore-former selected from the group consisting of inorganic and organic salt, oxide and carbohydrate.

An exit, or a plurality of exits, can be formed by leaching a member selected from the group consisting of sorbitol, lactose, fructose, glucose, mannose, galactose, talose, sodium chloride, potassium chloride, sodium citrate and mannitol to provide a uniform-release dimensioned pore-exit orifice.

The exit can have any shape, such as round, triangular, square, elliptical and the like for the uniform metered dose release of a drug from the dosage form. The sustained release dosage form can be constructed with one or more exits in spaced-apart relation in one or more surfaces of the sustained release dosage form.

Drilling, including mechanical and laser drilling, through the semipermeable membrane can be used to form the exit orifice. Such exits and equipment for forming such exits are disclosed in U.S. Patents Nos. 3,916,899, by Theeuwes and Higuchi and in U.S. Patent No. 4,088,864, by Theeuwes, et al. It is presently preferred to utilize two exits of equal diameter. In a preferred embodiment, exit 60 penetrates through subcoat 90, if present, to drug layer 30.

Dosage forms in accordance with the embodiments depicted in Figure 1 are manufactured by standard techniques. For example, the dosage form may be manufactured by the wet granulation technique. In the wet granulation technique, the drug and carrier are blended using an organic solvent, such as denatured anhydrous ethanol, as the granulation fluid. The remaining ingredients can be dissolved in a portion of the granulation fluid, such as the solvent described above, and this latter prepared wet blend is slowly added to the drug blend with continual mixing in the blender. The granulating fluid is added until a wet blend is produced, which wet mass blend is then forced through a predetermined screen onto oven trays. The blend is dried for 18 to 24 hours at 24°C to 35°C in a forced-air oven. The dried granules are then sized.

Next, magnesium stearate, or another suitable lubricant, is added to the drug granulation, and the granulation is put into milling jars and mixed on a jar mill for 10 minutes. The composition is pressed into a layer, for example, in a Manesty® press or a Korsch LCT press. For a trilayered core, granules or powders of the drug layer compositions and push layer composition are sequentially placed in an appropriately-sized die with intermediate compression steps being applied to each of the first two layers, followed by a final compression step after the last layer is added to the die to form the trilayered core. The intermediate compression typically takes place under a force of about 50-100 newtons. Final stage compression typically takes place at a force of 3500 newtons or greater, often 3500-5000 newtons. The compressed cores are fed to a dry coater press, e.g., Kilian® Dry Coater press, and subsequently coated with the membrane materials as described above.

In another embodiment, the drug and other ingredients comprising the drug layer are blended and pressed into a solid layer. The layer possesses dimensions that correspond to the internal dimensions of the area the layer is to occupy in the dosage form, and it also possesses dimensions corresponding to the push layer, if included, for forming a contacting arrangement therewith. The drug and other ingredients can also be blended with a solvent and mixed into a solid or semisolid form by conventional methods, such as ballmilling, calendering, stirring or roll milling, and then pressed into a preselected shape. Next, if included, a layer of osmopolymer composition is placed in contact with the layer of drug in a like manner. The layering of the drug formulation and the osmopolymer layer can be fabricated by conventional two-layer press techniques. An analogous procedure may be followed for the preparation of the trilayered core. The compressed cores then may be coated with the wall material and the semipermeable membrane material as described above.

Another manufacturing process that can be used comprises blending the powdered ingredients for each layer in a fluid bed granulator. After the powdered ingredients are dry blended in the granulator, a granulating fluid, for example, poly(vinylpyrrolidone) in water, is sprayed onto the powders. The coated powders are then dried in the granulator. This process granulates all the ingredients present therein while adding the granulating fluid. After the granules are dried, a lubricant, such as stearic acid or magnesium stearate, is mixed into the granulation using a blender e.g., V-blender or tote blender. The granules are then pressed in the manner described above.

Exemplary solvents suitable for manufacturing the dosage form components comprise aqueous or inert organic solvents that do not adversely harm the materials used in the system. The solvents broadly include members selected from the group consisting of aqueous solvents, alcohols, ketones, esters, ethers, aliphatic hydrocarbons, halogenated solvents, cycloaliphatics, aromatics, heterocyclic solvents and mixtures thereof. Typical solvents include acetone, diacetone alcohol, methanol, ethanol, isopropyl alcohol, butyl alcohol, methyl acetate, ethyl acetate, isopropyl acetate, n-butyl acetate, methyl isobutyl ketone; methyl propyl ketone, n-hexane, n-heptane, ethylene glycol monoethyl ether, ethylene glycol monoethyl acetate, methylene dichloride, ethylene dichloride, propylene dichloride, carbon tetrachloride nitroethane, nitropropane tetrachloroethane, ethyl ether, isopropyl ether, cyclohexane, cyclooctane, benzene, toluene, naphtha, 1,4-dioxane, tetrahydrofuran, diglyme, water, aqueous solvents containing inorganic salts such as sodium chloride, calcium chloride, and the like, and mixtures thereof such as acetone and water, acetone and methanol, acetone and ethyl alcohol, methylene dichloride and methanol, and ethylene dichloride and methanol.

One important consideration in the practice of this invention is the physical state of the opioid to be delivered by the dosage form. In certain embodiments, the opioids may be in a paste or liquid state. In such cases solid dosage forms may not be suitable for use in the practice of this invention. Instead, dosage forms capable of delivering substances in a paste or liquid state should be used.

The present invention provides a liquid formulation of substances for use with oral osmotic devices. Oral osmotic devices for delivering liquid formulations and methods of using them are known in the art, for example, as described and claimed in the following U.S. Patents owned by ALZA corporation: 6,419,952; 6,174,547; 6,551,613; 5,324,280; 4,111,201; and 6,174,547. Methods of using oral osmotic devices for delivering therapeutic agents at an ascending rate of release can be found in International Application Numbers WO 98/06380, WO 98/23263, and WO 99/62496.

Exemplary liquid carriers for the present invention include lipophilic solvents (e.g., oils and lipids), surfactants, and hydrophilic solvents. Exemplary lipophilic solvents, for example, include, but are not limited to, Capmul PG-8, Caprol MPGO, Capryol 90, Plurol Oleique CC 497, Capmul MCM, Labrafac PG, N-Decyl Alcohol, Caprol 10G100, Oleic Acid, Vitamin E, Maisine 35-1, Gelucire 33/01, Gelucire 44/14, Lauryl Alcohol, Captex 355EP, Captex 500, Capylic/Caplic Triglyceride, Peceol, Caprol ET, Labrafil M2125 CS, Labrafac CC, Labrafil M 1944 CS, Captex 8277, Myvacet 9-45, Isopropyl Nyristate, Caprol PGE 860, Olive Oil, Plurol Oleique, Peanut Oil, Captex 300 Low C6, and Capric Acid.

Exemplary surfactants for example, include, but are not limited to, Vitamin E TPGS, Cremophor (grades EL, EL-P, and RH40), Labrasol, Tween (grades 20, 60, 80), Pluronic (grades L-31, L-35, L-42, L-64, and L-121), Acconon S-35, Solutol HS-15, and Span (grades 20, and 80). Exemplary hydrophilic solvents for example; include, but are not limited to, Isosorbide Dimethyl Ether, Polyethylene Glycol (PEG grades 300, 400, 600, 3000, 4000, 6000, and 8000) and Propylene Glycol (PG).

The skilled practitioner will understand that any formulation comprising a sufficient dosage of opioid solubilized in a liquid carrier suitable for administration to a subject and for use in an osmotic device can be used in the present invention. In one exemplary embodiment of the present invention, the liquid carrier is PG, Solutol, Cremophor EL, or a combination thereof.

The liquid formulation according to the present invention can also comprise, for example, additional excipients such as an antioxidant, permeation enhancer and the like. Antioxidants can be provided to slow or effectively stop the rate of any autoxidizable material present in the capsule. Representative antioxidants can comprise a member selected from the group of ascorbic acid; alpha tocopherol; ascorbyl palmitate; ascorbates; isoascorbates; butylated hydroxyanisole; butylated hydroxytoluene; nordihydroguiaretic acid; esters of garlic acid comprising at least 3 carbon atoms comprising a member selected from the group consisting of propyl gallate, octyl gallate, decyl gallate, decyl gallate; 6-ethoxy-2,2,4-trimethyl-1,2-dihydroguinoline; N-acetyl-2,6-di-t-butyl-p-aminophenol; butyl tyrosine; 3-tertiarybutyl-4-hydroxyanisole; 2-tertiary-butyl-4-hydroxyanisole; 4-chloro-2,6-ditertiary butyl phenol; 2,6-ditertiary butyl p-methoxy phenol; 2,6-ditertiary butyl-p-cresol: polymeric antioxidants; trihydroxybutyro-phenone physiologically acceptable salts of ascorbic acid, erythorbic acid, and ascorbyl acetate; calcium ascorbate; sodium ascorbate; sodium bisulfite; and the like. The amount of antioxidant used for the present purposes, for example, can be about 0.001% to 25% of the total weight of the composition present in the lumen. Antioxidants are known to the prior art in U.S. Pat. Nos. 2,707,154; 3,573,936; 3,637,772; 4,038,434; 4,186,465 and 4,559,237, each of which is hereby incorporated by reference in its entirety for all purposes.

The inventive liquid formulation can comprise permeation enhancers that facilitate absorption of the drug in the environment of use. Such enhancers can, for example, open the so-called "tight junctions" in the gastrointestinal tract or modify the effect of cellular components, such a p-glycoprotein and the like. Suitable enhancers can include alkali metal salts of salicyclic acid, such as sodium salicylate, caprylic or capric acid, such as sodium caprylate or sodium caprate, and the like. Enhancers can include, for example, the bile salts, such as sodium deoxycholate. Various p-glycoprotein modulators are described in U.S. Pat. Nos. 5,112,817 and 5,643,909. Various other absorption enhancing compounds and materials are described in U.S. Pat. No. 5,824,638. Enhancers can be used either alone or as mixtures in combination with other enhancers.

In certain embodiments, the inventive substances are administered as a self-emulsifying formulation. Like the other liquid carriers, the surfactant functions to prevent aggregation, reduce interfacial tension between constituents, enhance the free-flow of constituents, and lessen the incidence of constituent retention in the dosage form. The emulsion formulation of this invention comprises a surfactant that imparts emulsification. Exemplary surfactants can also include, for example, in addition to the surfactants listed above, a member selected from the group consisting of polyoxyethylenated castor oil comprising ethylene oxide in the concentration of 9 to 15 moles, polyoxyethylenated sorbitan monopalmitate, mono and tristearate comprising 20 moles of ethylene oxide, polyoxyethylenated sorbitan monostearate comprising 4 moles of ethylene oxide, polyoxyethylenated sorbitan trioleate comprising 20 moles of ethylene oxide, polyoxyethylene lauryl ether, polyoxyethylenated stearic acid comprising 40 to 50 moles of ethylene oxide, polyoxyethylenated stearyl alcohol comprising 2 moles of ethylene oxide, and polyoxyethylenated oleyl alcohol comprising 2 moles of ethylene oxide. The surfactants may be available from Atlas Chemical Industries.

The drug emulsified formulations of the present invention can initially comprise an oil and a non-ionic surfactant. The oil phase of the emulsion comprises any pharmaceutically acceptable oil that is not immiscible with water. The oil can be an edible liquid such as a non-polar ester of an unsaturated fatty acid, derivatives of such esters, or mixtures of such esters. The oil can be vegetable, mineral, animal or marine in origin. Examples of non-toxic oils can also include, for example, in addition to the surfactants listed above, a member selected from the group consisting of peanut oil, cottonseed oil, sesame oil, corn oil, almond oil, mineral oil, castor oil, coconut oil, palm oil, cocoa butter, safflower, a mixture of mono- and diglycerides of 16 to 18 carbon atoms, unsaturated fatty acids, fractionated triglycerides derived from coconut oil, fractionated liquid triglycerides derived from short chain 10 to 15 carbon atoms fatty acids, acetylated monoglycerides, acetylated diglycerides, acetylated triglycerides, olein known also as glyceral trioleate, palmitin known as glyceryl tripalmitate, stearin known also as glyceryl tristearate, lauric acid hexylester, oleic acid oleylester, glycolyzed ethoxylated glycerides of natural oils, branched fatty acids with 13 molecules of ethyleneoxide, and oleic acid decylester. The concentration of oil, or oil derivative in the emulsion formulation can be from about 1 wt % to about 40 wt %, with the wt % of all constituents in the emulsion preparation equal to 100 wt %. The oils are disclosed in Pharmaceutical Sciences by Remington, 17th Ed., pp. 403-405, (1985) published by Mark Publishing Co., in Encyclopedia of Chemistry, by Van Nostrand Reinhold, 4th Ed., pp. 644-645, (1984) published by Van Nostrand Reinhold Co.; and in U.S. Pat. No. 4,259,323.

The amount of opioid incorporated in the dosage forms of the present invention is generally from about 10% to about 90% by weight of the composition depending upon the therapeutic indication and the desired administration period, e.g., every 12 hours, every 24 hours, and the like. Depending on the dose of opioid desired to be administered, one or more of the dosage forms can be administered.

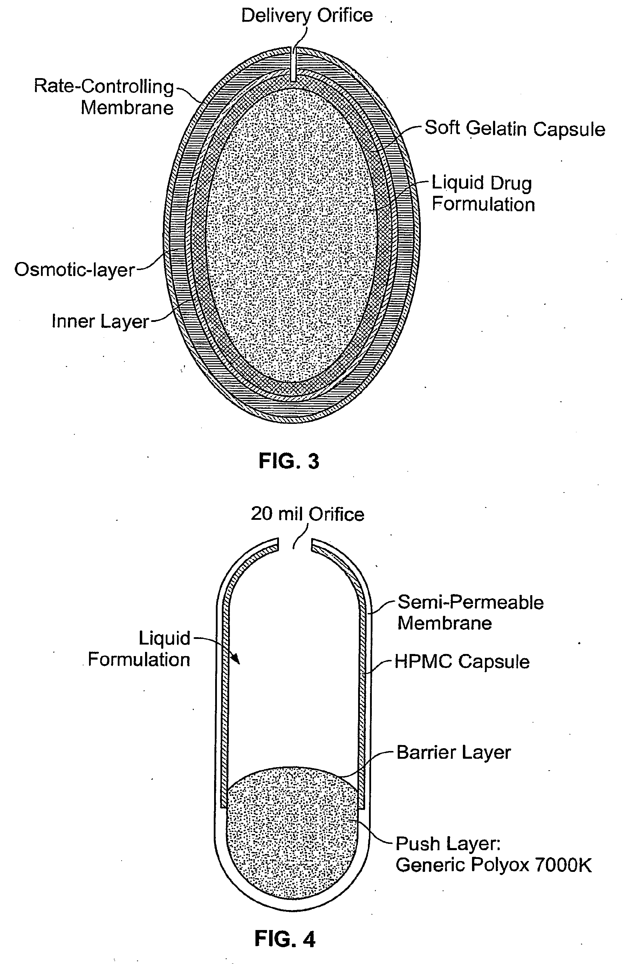

The osmotic dosage forms of the present invention can possess two distinct forms, a soft capsule form (shown in Fig. 3) and a hard capsule form (shown in Fig. 4). The soft capsule, as used by the present invention, preferably in its final form comprises one piece. The one-piece capsule is of a sealed construction encapsulating the drug formulation therein. The capsule can be made by various processes including the plate process, the rotary die process, the reciprocating die process, and the continuous process. An example of the plate process is as follows. The plate process uses a set of molds. A warm sheet of a prepared capsule lamina-forming material is laid over the lower mold and the formulation poured on it. A second sheet of the lamina-forming material is placed over the formulation followed by the top mold. The mold set is placed under a press and a pressure applied, with or without heat, to form a unit capsule. The capsules are washed with a solvent for removing excess agent formulation from the exterior of the capsule, and the air-dried capsule is encapsulated with a semipermeable wall. The rotary die process uses two continuous films of capsule lamina-forming material that are brought into convergence between a pair of revolving dies and an injector wedge. The process fills and seals the capsule in dual and coincident operations. In this process, the sheets of capsule lamina-forming material are fed over guide rolls, and then down between the wedge injector and the die rolls.

The agent formulation to be encapsulated flows by gravity into a positive displacement pump. The pump meters the agent formulation through the wedge injector and into the sheets between the die rolls. The bottom of the wedge contains small orifices lined up with the die pockets of the die rolls. The capsule is about half-sealed when the pressure of pumped agent formulation forces the sheets into the die pockets, wherein the capsules are simultaneously filled, shaped, hermetically sealed and cut from the sheets of lamina-forming materials. The sealing of the capsule is achieved by mechanical pressure on the die rolls and by heating of the sheets of lamina-forming materials by the wedge. After manufacture, the agent formulation-filled capsules are dried in the presence of forced air, and a semipermeable lamina encapsulated thereto.

The reciprocating die process produces capsules by leading two films of capsule lamina-forming material between a set of vertical dies. The dies as they close, open, and close perform as a continuous vertical plate forming row after row of pockets across the film. The pockets are filled with an inventive formulation, and as the pockets move through the dies, they are sealed, shaped, and cut from the moving film as capsules filled with agent formulation. A semipermeable encapsulating lamina is coated thereon to yield the capsule. The continuous process is a manufacturing system that also uses rotary dies, with the added feature that the process can successfully fill active agent in dry powder form into a soft capsule, in addition to encapsulating liquids. The filled capsule of the continuous process is encapsulated with a semipermeable polymeric material to yield the capsule. Procedures for manufacturing soft capsules are disclosed in U.S. Pat. No. 4,627,850 and U.S. Patent No. 6,419,952.

The dosage forms of the present invention can also be made from an injection-moldable composition by an injection-molding technique. Injection-moldable compositions provided for injection-molding into the semipermeable membrane comprise a thermoplastic polymer, or the compositions comprise a mixture of thermoplastic polymers and optional injection-molding ingredients. The thermoplastic polymer that can be used for the present purpose comprise polymers that have a low softening point, for example, below 200°C, preferably within the range of 40°C to 180°C. The polymers, are preferably synthetic resins, addition polymerized resins, such as polyamides, resins obtained from diepoxides and primary alkanolamines, resins of glycerine and phthalic anhydrides, polymethane, polyvinyl resins, polymer resins with end-positions free or esterified carboxyl or caboxamide groups, for example with acrylic acid, acrylic amide, or acrylic acid esters, polycaprolactone, and its copolymers with dilactide, diglycolide, valerolactone and decalactone, a resin composition comprising polycaprolactone and polyalkylene oxide, and a resin composition comprising polycaprolactone, a polyalkylene oxide such as polyethylene oxide, poly(cellulose) such as poly(hydroxypropylmethylcellulose), poly(hydroxyethylmethylcellulose), and poly(hydroxypropylcellulose). The membrane forming composition can comprise optional membrane-forming ingredients such as polyethylene glycol, talcum, polyvinylalcohol, lactose, or polyvinyl pyrrolidone. The compositions for forming an injection-molding polymer composition can comprise 100% thermoplastic polymer. The composition in another embodiment comprises 10% to 99% of a thermoplastic polymer and 1% to 90% of a different polymer with the total equal to 100%. The invention provides also a thermoplastic polymer composition comprising 1% to 98% of a first thermoplastic polymer, 1% to 90% of a different, second polymer and 1% to 90% of a different, third polymer with all polymers equal to 100%.

Representative compositions comprises 20% to 90% of thermoplastic polycaprolactone and 10% to 80% of poly(alkylene oxide); a composition comprising 20% to 90% polycaprolactone and 10% to 60% of poly(ethylene oxide) with the ingredients equal to 100%; a composition comprising 10% to 97% of polycaprolactone, 10% to 97% poly(alkylene oxide), and 1% to 97% of poly(ethylene glycol) with all ingredients equal to 100%; a composition comprising 20% to 90% polycaprolactone and 10% to 80% of poly(hydroxypropylcellulose) with all ingredients equal to 100%; and a composition comprising 1% to 90% polycaprolactone, 1% to 90% poly(ethylene oxide), 1% to 90% poly(hydroxypropylcellulose) and 1% to 90% poly(ethylene glycol) with all ingredients equal to 100%. The percent expressed is weight percent wt %.

In another embodiment of the invention, a composition for injection-molding to provide a membrane can be prepared by blending a composition comprising a polycaprolactone 63 wt %, polyethylene oxide 27 wt %, and polyethylene glycol 10 wt % in a conventional mixing machine, such as a Moriyama™ Mixer at 65°C to 95°C, with the ingredients added to the mixer in the following addition sequence, polycaprolactone, polyethylene oxide and polyethylene glycol. In one example, all the ingredients are mixed for 135 minutes at a rotor speed of 10 to 20 rpm. Next, the blend is fed to a Baker Perkins Kneader™ extruder at 80°C to 90°C, at a pump speed of 10 rpm and a screw speed of 22 rpm, and then cooled to 10°C to 12°C, to reach a uniform temperature. Then, the cooled extruded composition is fed to an Albe Pelletizer, converted into pellets at 250°C, and a length of 5 mm. The pellets next are fed into an injection-molding machine, an Arburg Allrounder™ at 200°F. to 350°C (93°C to 177°C), heated to a molten polymeric composition, and the liquid polymer composition forced into a mold cavity at high pressure and speed until the mold is filled and the composition comprising the polymers are solidified into a preselected shape. The parameters for the injection-molding consists of a band temperature through zone 1 to zone 5 of the barrel of 195°F. (91°C) to 375°F., (191°C), an injection-molding pressure of 1818 bar, a speed of 55 cm3 /s, and a mold temperature of 75°C. The injection-molding compositions and injection-molding procedures are disclosed in U.S. Pat. No. 5,614,578.

Alternatively, the capsule can be made conveniently in two parts, with one part (the "cap") slipping over and capping the other part (the "body") as long as the capsule is deformable under the forces exerted by the expandable layer and seals to prevent leakage of the liquid, active agent formulation from between the telescoping portions of the body and cap. The two parts completely surround and capsulate the internal lumen that contains the liquid, active agent formulation, which can contain useful additives. The two parts can be fitted together after the body is filled with a preselected formulation. The assembly can be done by slipping or telescoping the cap section over the body section, and sealing the cap and body, thereby completely surrounding and encapsulating the formulation of active agent.

Soft capsules typically have a wall thickness that is greater than the wall thickness of hard capsules. For example, soft capsules can, for example, have a wall thickness on the order of 10-40 mils, about 20 mils being typical, whereas hard capsules can, for example, have a wall thickness on the order of 2-6 mils, about 4 mils being typical.

In one embodiment of the dosage system, a soft capsule can be of single unit construction and can be surrounded by an unsymmetrical hydro-activated layer as the expandable layer. The expandable layer will generally be unsymmetrical and have a thicker portion remote from the exit orifice. As the hydro-activated layer imbibes and/or absorbs external fluid, it expands and applies a push pressure against the wall of the capsule and optional barrier layer and forces active agent formulation through the exit orifice. The presence of an unsymmetrical layer functions to assure that the maximum dose of agent is delivered from the dosage form, as the thicker section of layer distant from passageway swells and moves towards the orifice.

In yet another configuration, the expandable layer can be formed in discrete sections that do not entirely encompass an optionally barrier layer-coated capsule. The expandable layer can be a single element that is formed to fit the shape of the capsule at the area of contact. The expandable layer can be fabricated conveniently by tableting to form the concave surface that is complementary to the external surface of the barrier-coated capsule.

Appropriate tooling such as a convex punch in a conventional tableting press can provide the necessary complementary shape for the expandable layer. In this case, the expandable layer is granulated and compressed, rather than formed as a coating. The methods of formation of an expandable layer by tableting are well known, having been described, for example in U.S. Pat. Nos. 4,915,949; 5,126,142; 5,660,861; 5,633,011; 5,190,765; 5,252,338; 5;620,705; 4,931,285; 5,006,346; 5,024,842; and 5,160,743.

In some embodiments, a barrier layer can be first coated onto the capsule and then the tableted, expandable layer is attached to the barrier-coated capsule with a biologically compatible adhesive. Suitable adhesives include, for example, starch paste, aqueous gelatin solution, aqueous gelatin/glycerin solution, acrylate-vinylacetate based adhesives such as Duro-Tak adhesives (National Starch and Chemical Company), aqueous solutions of water soluble hydrophilic polymers such as hydroxypropyl methyl cellulose, hydroxymethyl cellulose, hydroxyethyl cellulose, and the like. That intermediate dosage form can be then coated with a semipermeable layer. The exit orifice is formed in the side or end of the capsule opposite the expandable layer section. As the expandable layer imbibes fluid, it will swell. Since it is constrained by the semipermeable layer, as it expands it will compress the barrier-coated capsule and express the liquid, active agent formulation from the interior of the capsule into the environment of use.

The hard capsules are typically composed of two parts, a cap and a body, which are fitted together after the larger body is filled with a preselected appropriate formulation. This can be done by slipping or telescoping the cap section over the body section, thus completely surrounding and encapsulating the useful agent formulation. Hard capsules can be made, for example, by dipping stainless steel molds into a bath containing a solution of a capsule lamina-forming material to coat the mold with the material. Then, the molds are withdrawn, cooled, and dried in a current of air. The capsule is stripped from the mold and trimmed to yield a lamina member with an internal lumen. The engaging cap that telescopically caps the formulation receiving body is made in a similar manner. Then, the closed and filled capsule can be encapsulated with a semipermeable lamina. The semipermeable lamina can be applied to capsule parts before or after parts and are joined into the final capsule. In another embodiment, the hard capsules can be made with each part having matched locking rings near their opened end that permit joining and locking together the overlapping cap and body after filling with formulation. In this embodiment, a pair of matched locking rings are formed into the cap portion and the body portion, and these rings provide the locking means for securely holding together the capsule. The capsule can be manually filled with the formulation, or they can be machine filled with the formulation. In the final manufacture, the hard capsule is encapsulated with a semipermeable lamina permeable to the passage of fluid and substantially impermeable to the passage of useful agent. Methods of forming hard cap dosage forms are described in U.S. Patent No. 6,174,547, U.S. Patent Nos. 6,596,314, 6,419,952, and 6,174,547.

The hard and soft capsules can comprise, for example, gelatin; gelatin having a viscosity of 15 to 30 millipoises and a bloom strength up to 150 grams; gelatin having a bloom value of 160 to 250; a composition comprising gelatin, glycerine, water and titanium dioxide; a composition comprising gelatin, erythrosin, iron oxide and titanium dioxide; a composition comprising gelatin, glycerine, sorbitol, potassium sorbate and titanium dioxide; a composition comprising gelatin, acacia glycerine, and water; and the like. Materials useful for forming capsule membrane are known in U.S. Pat. Nos. 4,627,850; and in 4,663,148. Alternatively, the capsules can be made out of materials other than gelatin (see for example, products made by BioProgres plc).

The capsules typically can be provided, for example, in sizes from about 3 to about 22 minims (1 minim being equal to 0.0616 ml) and in shapes of oval, oblong or others. They can be provided in standard shape and various standard sizes, conventionally designated as (000), (00), (0), (1), (2), (3), (4), and (5). The largest number corresponds to the smallest size. Non-standard shapes can be used as well. In either case of soft capsule or hard capsule, non-conventional shapes and sizes can be provided if required for a particular application.

The osmotic devices of the present invention may comprise a semipermeable membrane permeable to the passage of exterior biological fluid and substantially impermeable to the passage of opioid formulation. The selectively permeable compositions used for forming the membrane are essentially non-erodible and they are insoluble in biological fluids during the life of the osmotic system. The semipermeable membrane comprises a composition that does not adversely affect the host, the opioid formulation, an osmopolymer, osmagent and the like. Materials useful in the formation of a semipermeable membrane are disclosed elsewhere herein.

The semipermeable membrane can also comprise a flux regulating agent. Materials useful as flux regulating agents are disclosed elsewhere herein. Other materials that can be used to form the semipermeable membrane for imparting flexibility and elongation properties to the semipermeable membrane are also disclosed elsewhere herein.

The semipermeable membrane surrounds and forms a compartment containing one or a plurality of layers, one of which is an expandable layer which in some embodiments, can contain osmotic agents. The composition of such expandable layers is disclosed elsewhere herein.

In certain solid and liquid embodiments, the dosage forms further can comprise a barrier layer. The barrier layer in certain embodiments is deformable under the pressure exerted by the expandable layer and will be impermeable (or less permeable) to fluids and materials that can be present in the expandable layer, the liquid active agent formulation and in the environment of use, during delivery of the active agent formulation. A certain degree of permeability of the barrier layer can be permitted if the delivery rate of the active agent formulation is not detrimentally affected. However, it is preferred that the barrier layer not completely transport through it fluids and materials in the dosage form and the environment of use during the period of delivery of the active agent. The barrier layer can be deformable under forces applied by the expandable layer so as to permit compression of the capsule to force the liquid, active agent formulation from the exit orifice. In some embodiments, the barrier layer will be deformable to such an extent that it creates a seal between the expandable layer and the semipermeable layer in the area where the exit orifice is formed. In that manner, the barrier layer will deform or flow to a limited extent to seal the initially, exposed areas of the expandable layer and the semipermeable layer when the exit orifice is being formed, such as by drilling or the like, or during the initial stages of operation. When sealed, the only avenue for liquid permeation into the expandable layer is through the semipermeable layer, and there is no back-flow of fluid into the expandable layer through the exit orifice.

Suitable materials for forming the barrier layer can include, for example, polyethylene, polystyrene, ethylene-vinyl acetate copolymers, polycaprolactone and Hytrel™ polyester elastomers (Du Pont), cellulose acetate, cellulose acetate pseudolatex (such as described in U.S. Pat. No. 5,024,842), cellulose acetate propionate, cellulose acetate butyrate, ethyl cellulose, ethyl cellulose pseudolatex (such as Surelease™ as supplied by 10 Colorcon, West Point, Pa. or Aquacoat™ as supplied by FMC Corporation, Philadelphia, Pa.), nitrocellulose, polylactic acid, poly- glycolic acid, polylactide glycolide copolymers, collagen, polyvinyl alcohol, polyvinyl acetate, polyethylene vinylacetate, polyethylene teraphthalate, polybutadiene styrene, polyisobutylene, polyisobutylene isoprene copolymer, polyvinyl chloride, polyvinylidene chloride-vinyl chloride copolymer, copolymers of acrylic acid and methacrylic acid esters, copolymers of methylmethacrylate and ethylacrylate, latex of acrylate esters (such as Eudragit™ supplied by RohmPharma, Darmstaat, Germany), polypropylene, copolymers of propylene oxide and ethylene oxide, propylene oxide ethylene oxide block copolymers, ethylenevinyl alcohol copolymer, polysulfone, ethylene vinylalcohol copolymer, polyxylylenes, polyalkoxysilanes, polydimethyl siloxane, polyethylene glycol-silicone elastomers, electromagnetic irradiation crosslinked acrylics, silicones, or polyesters, thermally crosslinked acrylics, silicones, or polyesters, butadiene-styrene rubber, and blends of the above.

Preferred materials can include cellulose acetate, copolymers of acrylic acid and methacrylic acid esters, copolymers of methylmethacrylate and ethylacrylate, and latex of acrylate esters. Preferred copolymers can include poly (butyl methacrylate), (2-dimethylaminoethyl)methacrylate, methyl methacrylate) 1:2:1, 150,000, sold under the trademark EUDRAGIT E; poly (ethyl acrylate, methyl methacrylate) 2:1, 800,000, sold under the trademark EUDRAGIT NE 30 D; poly (methacrylic acid, methyl methacrylate) 1:1,135,000, sold under the trademark EUDRAGIT L; poly (methacrylic acid, ethyl acrylate) 1:1, 250,000, sold under the trademark EUDRAGIT L; poly (methacrylic acid, methyl methacrylate) 1:2, 135,000, sold under the trademark EUDRAGIT S; poly (ethyl acrylate, methyl methacrylate, trimethylammonioethyl methacrylate chloride) 1:2:0.2, 150,000, sold under the trademark EUDRAGIT RL; poly (ethyl acrylate, methyl methacrylate, trimethylammonioethyl methacrylate chloride) 1:2:0.1, 150,000, sold as EUDRAGIT RS. In each case, the ratio x:y:z indicates the molar proportions of the monomer units and the last number is the number average molecular weight of the polymer. Especially preferred are cellulose acetate containing plasticizers such as acetyl tributyl citrate and ethylacrylate methylmethylacrylate copolymers such as Eudragit NE.

The foregoing materials for use as the barrier layer can be formulated with plasticizers to make the barrier layer suitably deformable such that the force exerted by the expandable layer will collapse the compartment formed by the barrier layer to dispense the liquid, active agent formulation. Examples of typical plasticizers are as follows: polyhydric alcohols, triacetin, polyethylene glycol, glycerol, propylene glycol, acetate esters, glycerol triacetate, triethyl citrate, acetyl triethyl citrate, glycerides, acetylated monoglycerides, oils, mineral oil, castor oil and the like. The plasticizers can be blended into the material in amounts of 10-50 weight percent based on the weight of the material.

The various layers forming the barrier layer, expandable layer and semipermeable layer can be applied by conventional coating methods such as described in U.S. Pat. No. 5,324,280. While the barrier layer, expandable layer and semipermeable membrane have been illustrated and described for convenience as single layers, each of those layers can be composites of several layers. For example, for particular applications it may be desirable to coat the capsule with a first layer of material that facilitates coating of a second layer having the permeability characteristics of the barrier layer. In that instance, the first and second layers comprise the barrier layer. Similar considerations would apply to the semipermeable layer and the expandable layer.

The exit orifice can be formed by mechanical drilling, laser drilling, eroding an erodible element, extracting, dissolving, bursting, or leaching a passageway former from the composite wall. The exit orifice can be a pore formed by leaching sorbitol, lactose or the like from a membrane or layer as disclosed in U.S. Pat. No. 4,200,098. This patent discloses pores of controlled-size porosity formed by dissolving, extracting, or leaching a material from a wall, such as sorbitol from cellulose acetate. A preferred form of laser drilling is the use of a pulsed laser that incrementally removes material from the composite membrane to the desired depth to form the exit orifice.

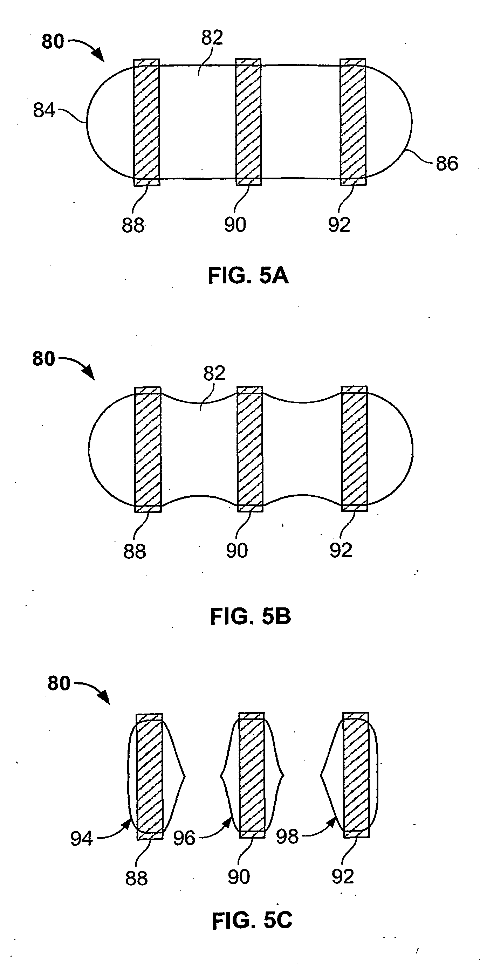

Figures 5A-5C illustrate another exemplary dosage form, known in the art and described in U.S. Patents Nos. 5,534,263; 5,667,804; and 6,020,000. Briefly, a cross-sectional view of a dosage form 80 is shown prior to ingestion into the gastrointestinal tract in Fig. 5A. The dosage form is comprised of a cylindrically shaped matrix 82 comprising an inventive substance. Ends 84, 86 of matrix 82 are preferably rounded and convex in shape in order to ensure ease of ingestion. Bands 88, 90, and 92 concentrically surround the cylindrical matrix and are formed of a material that is relatively insoluble in an aqueous environment. Suitable materials are set forth in the patents noted above and elsewhere herein.

After ingestion of dosage form 80, regions of matrix 82 between bands 88, 90, 92 begin to erode, as illustrated in Fig. 5B. Erosion of the matrix initiates release of the inventive substance into the fluidic environment of the G.I. tract. As the dosage form continues transit through the G.I. tract, the matrix continues to erode, as illustrated in Fig. 5C. Here, erosion of the matrix has progressed to such an extent that the dosage form breaks into three pieces, 94, 96, 98. Erosion will continue until the matrix portions of each of the pieces have completely eroded. Bands 94, 96, 98 will thereafter be expelled from the G.I. tract.

Other approaches to achieving sustained release of drugs from oral dosage forms are known in the art. For example, diffusion systems such as reservoir devices and matrix devices, dissolution systems such as encapsulated dissolution systems (including, for example, "tiny time pills") and matrix dissolution systems, combination diffusion/dissolution systems and ion-exchange resin systems are known and are disclosed in Remington's Pharmaceutical Sciences, 1990 ed., pp. 1682-1685. Dosage forms that operate in accord with these other approaches are encompassed by the scope of the disclosure herein to the extent that the drug release characteristics and/or the blood plasma concentration characteristics as recited herein and in the claims describe those dosage forms either literally or equivalently.

In other embodiments of the inventive methods, the recited sustained release dosage forms can be protected from the effects of ethanol in the gastrointestinal tract through the use of an enteric coating. Alcohol, especially ethanol, tends to be absorbed in the upper gastrointestinal tract, particularly the stomach. Accordingly, use of an enteric coating can mitigate the effects of coadministered alcohol on an inventive sustained release dosage form by retarding the initial release of drug in the upper GI tract..

In a preferable embodiment, the enteric coating comprises an enteric polymer. Preferably, the enteric polymer should not dissolve quickly in ethanol, but may swell or dissolve very slowly. Other polymers or materials may be mixed with the enteric polymer, so long as their addition does not compromise the enteric coating's performance in ethanol. In certain embodiments, the polymer or material that may be mixed with the enteric polymer may be chosen to enhance the performance of the enteric polymer in aqueous alcohol. For instance, in an embodiment a polymer or material that has little or no swell/solubility in aqueous alcohol may be advantageously mixed with the enteric polymer. A plasticizer such as PEG 6000 at 1-20% level, may be needed to prevent brittleness. Enteric polymers suitable for use in the invention comprise cellulose acetate phthalates, such as those made by Eastman Chemical. In certain embodiments, enteric polymers may be applied from solvent systems, such as acetone or acetone/ethanol mixtures, or from aqueous dispersions. In some cases, the enteric coatings may be applied using compression molding techniques.

In other embodiments of the present invention, non-enteric polymers may be used to coat the sustained release dosage forms, and thus reduce susceptibility to alcohol-induced dose dumping, particularly ethanol-induced dose dumping. In an embodiment, Eudragit® RS100 and Eudragit® RL100 may be used. These polymers are reported in the literature to be insoluble in water and to have slow dissolution in ethanol/water mixtures. They are further reported to offer low and moderate water permeability, respectively. Applied to a sustained release tablet matrix, these polymers would be expected to be at least reasonably effective rate-limiting films in water and in ethanol/water mixtures. In terms of mechanism of operation, such structures may perform according to diffusion-controlled release principles. These films are typically applied from aqueous dispersions and formulated with a plasticizer, such as triethyl citrate, and an anti-adherent such as talc. In another embodiment, cellulose acetate with 24-28% acetyl content may be used. This material is reportedly soluble in water and less soluble in ethanol/water mixtures, thus reducing the likelihood of dose dumping when a dosage form is coadministered with alcohol, especially with ethanol. The non-enteric polymers may be solution coated or applied using compression molding techniques.

In an embodiment, the sustained release dosage form of the inventive methods may be a matrix dosage form. A matrix dosage form typically contains a gelling component, a hydrophobic excipient to control initial burst, drug, and diluent. Typically, the gelling component is 20-60 wt% and the hydrophobic excipient is 5-20 wt%, based on total dry weight of the dosage form. These dosage forms can be produced using granulation or dry-blending and compression into tablets. Alternatively, formulations could be hot-melt extruded into strands that are chopped and filled into capsules, thus producing dosage forms according to the present invention.

Suitable gelling components comprise:
1. Blends of different grades of HPMC (K4M, K100, E5) to obtain desired swelling and viscosity. HPMC is insoluble in ethanol, and therefore would be expected to release slower in alcohol/water than in water. HPC (Klucel® from Hercules-Aqualon) can be added to retard hydration rate.
2. Blends of different grades of polyethylene oxide (Polyox®, available from Dow Chemical). Polyox swells much less in ethanol/water than in water. Suggested grades are POLYOX WSR-205 NF, WSR-1105 NF, WSR N-12K NF, WSR N-60K NF, WSR-301 NF, WSR-303 NF, WSR Coagulant NF. These comprise usually 20-55% of the formulation
3. NaCMC (sodium carboxymethylcellulose) is insoluble in ethanol, so would probably be less susceptible to dose dumping in ethanol/water mixtures.
4. Alginic acid is insoluble in ethanol, swells in water, and therefore would be expected to swells less in ethanol/water.
5. Xanthum gum and guar gum matrices
6. Polyvinyl alcohol is reportedly soluble in water, but insoluble in ethanol.

The following hydrophobic excipients for burst control should be as or more effective in ethanol/water mixtures, due to low ethanol solubility:
1. MC (methylcellulose, Methocel-A Premium ® from Dow Chemical)
2. Glycerol palmitostearate (Precirol® ATO-5, Gattefosse)
3. Glycerol behenate (Compritol® 888-ATO, Gattefosse)
4. Calcium stearate
5. waxes
6. vegetable and mineral oils
7. aliphatic alcohols
8. polycaprolactone
9. PLGA
10. rosin

In an embodiment, the hydrophobic excipients comprise hydrophobic excipients that have a melting temperature greater than or equal to about 55 Deg C. Such hydrophobic excipients include, but are not limited to, white paraffin wax, stearyl alcohol, bee's wax, Lubritab® (vegetable oil), rosin, carnauba wax, and hydrogenated caster oil.

Diluents or fillers used in matrix formulations typically do not affect the release profile significantly. However care should be exercised in selection of these excipients as in the presence of alcohol the diluents can significantly affect the start up and release profile from the controlled release matrices. In an embodiment, a diluent may be usefully selected to have lower solubility in aqueous alcohol than in water such that the core hydration, and hence drug dissolution, may be limited in aqueous alcohol environments. In a preferable embodiment, a useful diluent comprises mannitol.

The following hydrophobic excipients are less preferred to be used in the present invention:
1. EC (ethylcellulose, from Dow Chemical) is typically used, but is soluble in ethanol.
2. Hydrogenated polyoxyl 60 castor oil.

U.S. Patents Nos. 5,871,778 and 5,656,299 disclose sustained microsphere formulations having almost zero order rate of release of active component when administered to a patient. U.S. Patents Nos. 5,654,008; 5,650,173; 5,770,231; 6,077,843; 6,368,632; and 5,965,168 disclose sustained-release microparticle compositions and their use for controlled delivery of active agents.

In another embodiment, osmotic beads may be used in the practice of the present invention. The opioid can be Wurster-coated onto nonpareil seeds or other substrates having sufficient osmotic activity. Subsequently, a semi-permeable film is deposited through another Wurster-coating process. For the latter, product is removed at varying times or extents of coating so that a wide distribution of coating thicknesses is achieved. Upon hydration, the systems pull in water due to osmosis and burst to release the drug. The bursting time should be proportional to the membrane thickness on each bead. These beads, optionally including some without any semi-permeable coating to act as a immediate release component, may be loaded into capsules to form an embodiment of the sustained release dosage forms of the present invention.

If drug loading using osmotic beads is too limiting, then beads can be made with extrusion-spheronization techniques. An advantage of this approach is that more of the drug can incorporated in the bead and there is one less coating process. Preferred carriers for extrusion-spheronization techniques might include, but are not limited to PLGA R208, rosin, and other high molecular weight materials. Other bead manufacturing techniques, such as coating non-drug containing cores, may also be used. The drug-containing beads can alternatively be coated with films not semi-permeable to water and release would be governed by a combination of diffusion and osmosis. In embodiments, stiffening agents and/or hydrophobic materials may be incorporated into the sustained release dosing structure to prevent alcohol-induced dosing dumping. Preferable stiffening agents and/or hydrophobic materials include, but are not limited to, fatty alcohols, waxes, oils and biodegradable materials; more preferably such materials include, but are not limited to, stearyl alcohol, carnauba wax, castor wax, and rosin.

In embodiments, gastric retention systems may also be used. Conventional gastro-retentive systems achieve gastric retention by virtue of their size (i.e. larger than the pyloric opening) and density (lighter than GI contents enabling floatation). The systems may use polymers including but not limited to polyethylene oxide (Polyox), HPC, HPMC, Crosspovidone, Sodium CMC, Ethyl cellulose, and the like. Addition of hydrophobic materials or waxes may improve the performance of such materials (which tend to form weaker gels in aqueous alcohol and thus may provide unsatisfactory performance). However hydrophobic materials might significantly increase the risk of deployment of such gastroretentive system further downstream from the stomach.

Other types of gastroretentive systems comprise rigid frames with attached and/or integral controlled release portions. These frames /or integral controlled release portions are preferably comprised of materials that are relatively insensitive to aqueous alcohol so to maintain gastroretentive and controlled release properties.

It will be appreciated that the dosage forms and formulation strategies described herein are merely exemplary of a variety of dosage forms intended to achieve administration of the inventive substance(s). Those of skill in the pharmaceutical arts can identify other formulation strategies that would be suitable, especially because not all formulation strategies will necessarily work for all opioids. Optimization within the skill of one of ordinary skill may be useful in the practice of the present invention.

### IV. Examples

### Example 1

### Hydromorphone Tablet, Bilayer 16 mg System

An inventive hydromorphone sustained release dosage form adapted, designed and shaped as an osmotic drug delivery device was manufactured as follows: First, a drug composition was prepared. 8.98 kg of hydromorphone hydrochloride, 2.2 kg of povidone (polyvinylpyrrolidone) identified as K29-32, and 67.06 kg of polyethylene oxide with average molecular weight of 200,000 were added to a fluid bed granulator bowl. Then, 6.0 kg of povidone (polyvinylpyrrolidone) identified as K29-32 and having an average molecular weight of 40,000 was dissolved in 54.0 kg of water to prepare the binder solution. The dry materials were fluid bed granulated by spraying with 18.0 kg of binder solution. Next, the wet granulation was dried in the granulator to an acceptable moisture content, and sized using a mill fitted with a 7-mesh screen. The granulation was then transferred to a blender and mixed with 16 g of butylated hydroxytoluene as an antioxidant and lubricated with 0.20 kg of magnesium stearate.

Next, a push composition was prepared as follows: 24.0 kg of sodium chloride and 0.32 kg of black iron oxide were sized using a Quadro Comil with a 21-mesh screen. The screened materials, 1.6 kg of hydroxypropylmethyl cellulose identified as 2910, and 51.44 kg of polyethylene oxide with an average molecular weight of approximately 7,000,000 were added to a fluid bed granulator bowl. Then a binder solution was prepared. Then, 6.0 kg of hydroxypropylmethyl cellulose identified as 2910 and having an average viscosity of 5 cps was dissolved in 54.0 kg of water to prepare the binder solution. The dry materials were fluid bed granulated by spraying 24.0 kg of binder solution. Next, the wet granulation was dried in the granulator to an acceptable moisture content, and sized using a mill fitted with a 0.094 inch screen. The granulation was then transferred to a blender and mixed with 40 g of butylated hydroxytoluene and lubricated with 0.20 kg of magnesium stearate.

Next, the hydromorphone drug composition and the push composition were compressed into bilayer cores. First, 150 mg of the hydromorphone drug composition was added to the die cavity and pre-compressed, then 130 mg of the push composition was added and the layers were pressed into 11/32" diameter, standard concave, bilayer arrangements.

The bilayer arrangements were coated with a semi-permeable wall. The wall forming composition comprised of 99% cellulose acetate identified as 398-10 and having an average acetyl content of 39.8%, and 1% polyethylene glycol identified as 3350 and having an average molecular weight of 3350. The wall-forming composition was dissolved in a 96% acetone and 4% water mixture to make a 6% solids solution. The wall-forming composition was sprayed onto and around the bilayered arrangements in a pan coater until approximately 30 mg of membrane was applied to each tablet.

One 0.64 mm exit passageway was laser drilled through the semi-permeable wall to connect the drug layer with the exterior of the dosage system. The residual solvent was removed by drying for 72 hours at 45°C and 45% relative humidity. After humidity drying, the tablets were dried for 4 hours at 45°C and ambient humidity.

### Example 2

### In vitro release study - 16 mg hydromorphone

A series of dissolution experiments using the hydromorphone tablets of Example 1 were conducted to evaluate the effect of alcohol on the in vitro release characteristics of hydromorphone sustained release dosage forms according to the invention that comprise 16mg of hydromorphone as hydromorphone HCl. Hydromorphone hydrochloride release was measured over 24 hours in aqueous solutions containing 0, 4, 20, and 40% ethanol by volume using a Type VII dissolution bath.

Hydromorphone HCl 16 mg tablets according to Example 1 were used to determine the release rate and cumulative release profiles in 0%, 4%, 20% and 40% ethanol. Release rate results from the 0 month stability time point were used for the 0% ethanol (water) condition. Results for the 4%, 20%, and 40% ethanol conditions were generated using extra samples from a 0 month stability time point. The release rate conditions were as follows: Apparatus: USP Type VII; Medium: Aqueous solutions containing 0% , 4%, 20% and 40% ethanol by volume; Volume: 50 mL; Temperature: 37 ± 0.5 Deg C; Time points: 2, 4, 6, 8, 10, 12, 14, 16, 18 and 24 hours.

Note: Precautions were taken to minimize release rate media evaporation. As known in the art, for a USP Type VII tester, the dosage form moves from tube to tube during the testing procedure. Accordingly, to minimize the effects of media evaporation, for the first 6 intervals and the last interval (2 - 12 hours and 24 hour), release rate media were added to the release rate tube approximately 30 minutes before each interval and the release rate tubes were taken out of the release rate bath immediately after each interval was done. For the 14, 16 and 18 intervals, media were placed into the tube at the same time so the release rate tubes were in the bath for approximately 6 and half hours.

The media was prepared as follows:
4% Ethanol by volume: a volume of 140 mL of pure ethanol (Sigma-Aldrich, 200 proof) was added to 3360 mL of water and mixed well.
20% Ethanol by volume: a volume of 700 mL of pure ethanol was added to 2800 mL of water and mixed well.
40% Ethanol by volume: a volume of 1400 mL of pure ethanol was added to 2100 mL of water and mixed well.

Samples were prepared as follows: Sample solutions in 4% and 20% ethanol were injected as is after mixing. A brief study was done to demonstrate the validity of this approach. Two standards prepared in water at different concentrations were diluted using 20% and 40% ethanol and analyzed by HPLC respectively, %recovery and peak shape was evaluated. Since peak splitting was observed for samples in the presence of 40% ethanol but not in the other sample solutions, and sample solutions in 40% ethanol have to be further treated while sample solutions in 4% and 20% ethanol were injected as is.

To avoid peak splitting, sample solutions in 40% ethanol were prepared as follows: After cooling to room temperature, solutions in the release rate tubes were adjusted back to 50 ml with 40% ethanol solution and mixed well. A volume of 2 mL of sample solution was then added to a scintillation vial. Sample solution was evaporated to dryness using an evaporator at 45 Deg C (SPD SpeedVac, SPD131DDA, RVT4104 Refrigerated Vapor Trap, OFP-400, Thermo Savant). A volume of 2 mL of water was added back to the scintillation vial and mixed well. Sample solution was then injected onto HPLC.

**HPLC Conditions**

| | |
|---|---|
| Column: | Varian Inertsil Phenyl-3, 5mm, 4.6x150 mm |
| Mobile Phase: | 35% Methanol 65% Buffer (0.1 % Sodium Phosphate, 0.2% Octanesulfonic acid, sodium salt, pH = 2.2) |
| Flow Rate: | 1.5 mL/min |
| Temperature: | 45 Deg C |
| Injection Volume: | 50 mL |
| Wavelength: | 280 nm |
| Run Time: | 7 min |

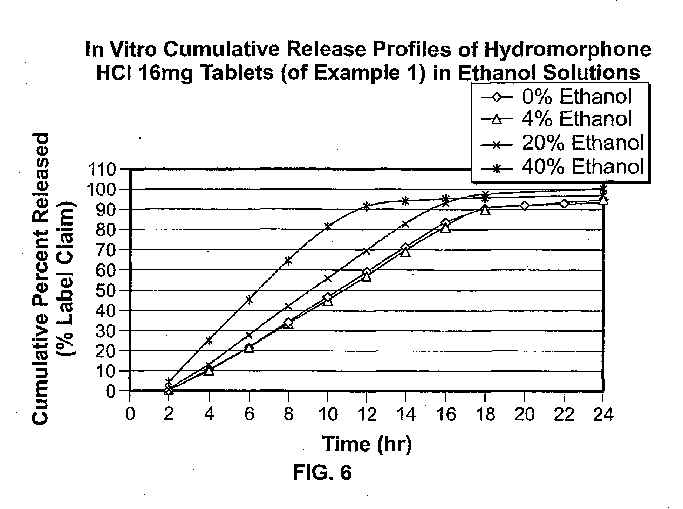

The results of this testing can be seen in Figure 6. For hydromorphone sustained release dosage forms according to the invention, the various ethanol solutions did not cause dose dumping or uncontrolled release. However, a trend of increasing release rate was observed as the concentration of ethanol in the dissolution media increased. The average release rate was greatest (approximately 10% label claim/hr) in the 40% ethanol media and was unaffected (approximately 6% label claim/hr) in the 4% ethanol media relative to the 0% control (6% label claim/hr). Correspondingly, the time to deliver 90% of the drug (T90) was unaffected in the 4% media relative to the control and most affected in the 40% media as shown in Table 1. Even for the 40% ethanol condition, T90 was at 12 h. In addition, there was minimal impact on the 2-hour cumulative release time interval (start-up time) for the tablet that reflects the lack of dose dumping across all ethanol concentrations evaluated.

### Example 3

### In vitro release comparison study

As a comparison, hydromorphone hydrochloride release from Palladone XL® 32 mg capsules was evaluated in vodka (27% v/v ethanol) and water using a Type II dissolution bath, as compared to the hydromorphone tablets of Example 1.

Dissolution parameters are as follows: Dissolution Apparatus:Varian VK7010 Dissolution unit and VK8000 Autosampler; Medium: Water and vodka (Pavlova, 40% alcohol/vol) respectively; Volume: 900 mL; Paddle Speed: 50 rpm; Pull Volume: 5 mL; Temperature: 37 ± 0.5 Deg C; Time points: T=1, 2, 4, 6, 10, 14, 18 and 24 hours. Note: testing results indicated that the alcohol content for Pavlova is only 27%.

Due to the chromatographic interference of vodka, sample solutions in vodka were evaporated before analysis, the detailed procedures were as follows: A volume of 5 mL sample solution was pulled using the auto-sampler into a test tube. After cooling to room temperature, a volume of 2 mL of sample solution was added to a scintillation vial. Sample solution was evaporated to dryness using an evaporator at 45 Deg C (SPD SpeedVac, SPD131DDA, RVT4104 Refrigerated Vapor Trap, OFP-400, Thermo Savant). A volume of 2 mL of water was added back to the scintillation vial and mixed well. Sample solution was then injected onto HPLC. Sample solutions in water were cooled to room temperature and injected onto HPLC.

Since sample solutions in water were injected as is, while sample solutions in vodka were evaporated and reconstituted back with water as part of the sample preparation, a brief validation study was conducted to show that there was no difference between the two sample preparations. For sample solutions in water, two standards at 100.04 and 180.07 mg/mL were evaporated to dryness, 2 mL of water was added back separately and mixed well followed by HPLC analysis. For sample solutions in vodka, a standard at 250.13 mg/mL was diluted to 50.03 mg/mL with vodka in triplicate, evaporated to dryness, 2 mL of water was added back and mixed well followed by HPLC analysis. Recovery was evaluated for the equivalency between two sample preparations. The two sample preparation techniques were shown to produce comparable results in the validation study.

Sample solution volume for both water and vodka was measured after 24 hours, and evaporation rate was calculated by the following formula based on linear evaporation: Evaporation rate = (900 - final volume - 8 x 5)/24 hour (8 x 5 = 5 mL per pull for 8 time points). Evaporation was corrected in the dissolution profile calculations. Sample pull volume was verified as a separate experiment in both water and vodka in triplicate measurements.

The HPLC conditions were as follows:

| | |
|---|---|
| Column: | Varian Inersil Phenyl-3, 5mm, 4.6x150 mm |
| Mobile Phase: | 35% Methanol 65% Buffer (0.1% Sodium Phosphate, 0.2% Octanesulfonic acid, sodium salt, pH = 2.2) |
| Flow Rate: | 1.5 mL/min |
| Temperature: | 45 Deg C |
| Injection Volume: | 100 mL |
| Wavelength: | 280 nm |
| Run Time: | 6.5 min |

The injection volume was increased to 100mL due to the low concentration of the sample solutions at earlier time points.

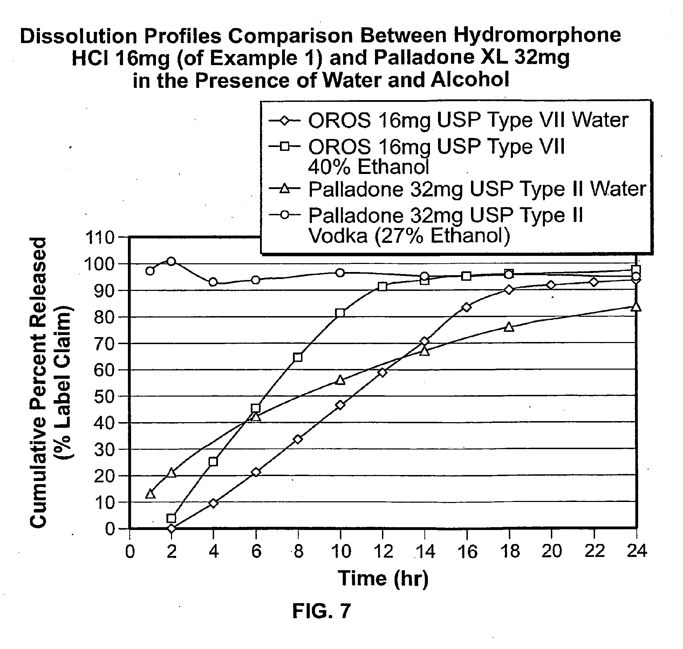

When exposed to 27% ethanol, Palladone XL delivered 100% label claim within 2 hours compared to 21% label claim in water as laid out in Table 2 and shown in Figure 7.

### Example 4

### Hydromorphone Tablet, Bilayer 16 mg System

A inventive hydromorphone sustained release dosage form adapted, designed and shaped as an osmotic drug delivery device was manufactured as follows: First, a drug composition was prepared. 8.98 kg of hydromorphone hydrochloride, 2.2 kg of povidone (polyvinylpyrrolidone) identified as K29-32, and 67.06 kg of polyethylene oxide with average molecular weight of 200,000 were added to a fluid bed granulator bowl. Then, 6.0 kg of povidone (polyvinylpyrrolidone) identified as K29-32 and having an average molecular weight of 40,000 was dissolved in 54.0 kg of water to prepare the binder solution. The dry materials were fluid bed granulated by spraying with 18.0 kg of binder solution. Next, the wet granulation was dried in the granulator to an acceptable moisture content, and sized using a mill fitted with a 7-mesh screen. The granulation was then transferred to a blender and mixed with 16 g of butylated hydroxytoluene as an antioxidant and lubricated with 0.20 kg of magnesium stearate.

Next, a push composition was prepared as follows: 24.0 kg of sodium chloride and 0.32 kg of black iron oxide were sized using a Quadro Comil with a 21-mesh screen. The screened materials, 1.6 kg of hydroxypropylmethyl cellulose identified as 2910, and 51.44 kg of polyethylene oxide with an average molecular weight of approximately 7,000,000 were added to a fluid bed granulator bowl. Then a binder solution was prepared. Then, 6.0 kg of hydroxypropylmethyl cellulose identified as 2910 and having an average viscosity of 5 cps was dissolved in 54.0 kg of water to prepare the binder solution. The dry materials were fluid bed granulated by spraying 24.0 kg of binder solution. Next, the wet granulation was dried in the granulator to an acceptable moisture content, and sized using a mill fitted with a 0.094 inch screen. The granulation was then transferred to a blender and mixed with 40 g of butylated hydroxytoluene and lubricated with 0.20 kg of magnesium stearate.

Next, the hydromorphone drug composition and the push composition were compressed into bilayer cores. First, 150 mg of the hydromorphone drug composition was added to the die cavity and pre-compressed, then 130 mg of the push composition was added and the layers were pressed into 11/32" diameter, standard concave, bilayer arrangements.

The bilayer arrangements were coated with a semi-permeable wall. The wall forming composition comprised of 99% cellulose acetate identified as 398-10 and having an average acetyl content of 39.8%, and 1% polyethylene glycol identified as 3350 and having an average molecular weight of 3350. The wall-forming composition was dissolved in a 96% acetone and 4% water mixture to make a 6% solids solution. The wall-forming composition was sprayed onto and around the bilayered arrangements in a pan coater until approximately 33 mg of membrane was applied to each tablet.

One 0.64 mm exit passageway was laser drilled through the semi-permeable wall to connect the drug layer with the exterior of the dosage system. The residual solvent was removed by drying for 72 hours at 45°C and 45% relative humidity. After humidity drying, the tablets were dried for 4 hours at 45°C and ambient humidity.

The dried tablets were then coated with a color and clear coating. The yellow Opadry II color coat was identified as Y-30-12863-A. 14.4 kg of the yellow Opadry II color was mixed in 105.6 kg of water to form a color suspension. The color suspension was sprayed onto and around the dried tablets in a pan coater until approximately 18 mg was applied to each tablet. Then, a clear coat solution was prepared by mixing 2.4 kg of Opadry clear identified as YS-1-19025 in 45.6 kg of water. The clear solution was sprayed onto and around the dried tablets in a pan coater until approximately 1.5 mg was applied to each tablet.

After color and clear coating, HM 16 was printed on each tablet with an Opacode water-based black ink identified as NS-78-17715. Printing was performed on a ramp printer.

### Example 5

### In vivo study

A Phase I study was performed to evaluate the effect of alcohol on the pharmacokinetics of hydromorphone tablets according to Example 4 under a fasted and a fed state in healthy subjects.

Two groups of 24 healthy, adult males and females, aged 21-45 years (inclusive) weighing at least 70 kg and within 25% of normal weight for height and body built were enrolled in the study. The study was a single-center, single-dose, open-label, four-treatment, four-period, four-sequence crossover study in two groups of subjects.

In Group 1 each subject received the following treatments in the fasting state:
Treatment A - 16 mg hydromorphone tablets according to Example 4 with 240 mL of orange juice
Treatment B - 16 mg hydromorphone tablets according to Example 4 with 240 mL of 4% v/v alcohol in orange juice
Treatment C - 16 mg hydromorphone tablets according to Example 4 with 240 mL of 20% v/v alcohol in orange juice
Treatment D - 16 mg hydromorphone tablets according to Example 4 with 240 mL of 40% v/v alcohol in orange juice

In Group 2 each subject received the following treatments after a standard breakfast:
Treatment E - 16 mg hydromorphone tablets according to Example 4 with 240 mL of orange juice
Treatment F - 16mg hydromorphone tablets according to Example 4 with 240 mL of 4% v/v alcohol in orange juice
Treatment G - 16 mg hydromorphone tablets according to Example 4 with 240 mL of 20% v/v alcohol in orange juice
Treatment H - 16 mg hydromorphone tablets according to Example 4 with 240 mL of 40% v/v alcohol in orange juice

The alcohol in treatments B, C, D, F, G, and H were consumed in approximately 30 minutes generally without gulping. For each treatment, the subject received about 50 mg of naltrexone as the opioid antagonist starting about 14 hours before dosing and twice daily during dosing and through about 48 hours post dose. There was a washout period of about 6 to 14 days between treatment periods, with the washout period beginning about 24 hours after dosing.

During each treatment, blood samples were collected from each subject to measure plasma hydromorphone concentration at about 0 (pre-dose), 2, 4, 6, 8, 10, 12, 16, 20, 24, 27, 30, 36, 42, and 48 hours post dose.

Plasma samples were analyzed using a validated liquid chromatography-tandem mass spectrometry (LC/MS/MS) method developed at CEDRA Corporation. Human plasma containing hydromorphone and the internal standard hydromorphone-D3 was extracted with an ethyl acetate/hexane solution, and the organic layer was removed and back-extracted before being evaporated to dryness. The extract was reconstituted, and an aliquot was injected onto a SCIEX API 4000 LC/MS/MS equipped with an HPLC column. Positive ions were monitored in the multiple-reaction-monitoring (MRM) mode.

This method was validated with a minimum quantifiable hydromorphone concentration of 0.05 ng/mL. During the validation, calibration curves for the analyte were constructed by plotting the analyte:internal standard ratio versus known concentrations of analyte. A calibration curve was constructed using peak area ratios (PAR) of the calibration standards by applying a 1/concentration2, linear-weighted regression algorithm. The calibration curve for hydromorphone was linear in the range of 0.05 to 10.0 ng/mL.

The following pharmacokinetic parameters were determined, based on plasma concentrations of hydromorphone:
Cmax - maximum observed plasma concentrations
Tmax - time to maximum concentration
k - apparent elimination rate constant was estimated by linear regression of the log-transformed plasma concentrations during the terminal log-linear decline phase.
t1/2 - apparent half-life (t1/2) values were calculated as 0.693/k.
AUCt - the area under the plasma concentration time profile from hour 0 to the last detectable concentration at time t was determined by the linear trapezoidal method.
AUCinf - the AUC value extrapolated to infinity was calculated as the sum of AUCt, and the area extrapolated to infinity, calculated by the concentration at time t (Ct) divided by k.

In both fed and fasted groups, plasma concentrations were close to limit of quantification at the first timepoint post dose at 2 hours; thereafter plasma concentrations rose slowly in all 4 treatments. Each group had some subjects with no concentration values for some treatments (dropouts) or low values with no clinical explanation; these subjects with low values were excluded from the analysis. Median Tmax was between 12 and 16 hours. Cmax values in the 3 alcohol treatments were slightly higher versus the value in the 0% alcohol treatment with ratios of 117, 131, and 128% in the 4, 20, and 40% alcohol treatments, respectively in the fasting state. In the fed state plasma hydromorphone concentration profiles were similar for the 4 treatments and resulted in lower Cmax ratios compared to the fasted state. Cmax ratios did not show any relation to the percent of alcohol (114, 114, and 110% in the 4, 20, and 40% alcohol treatments, respectively, versus 0% alcohol treatment).

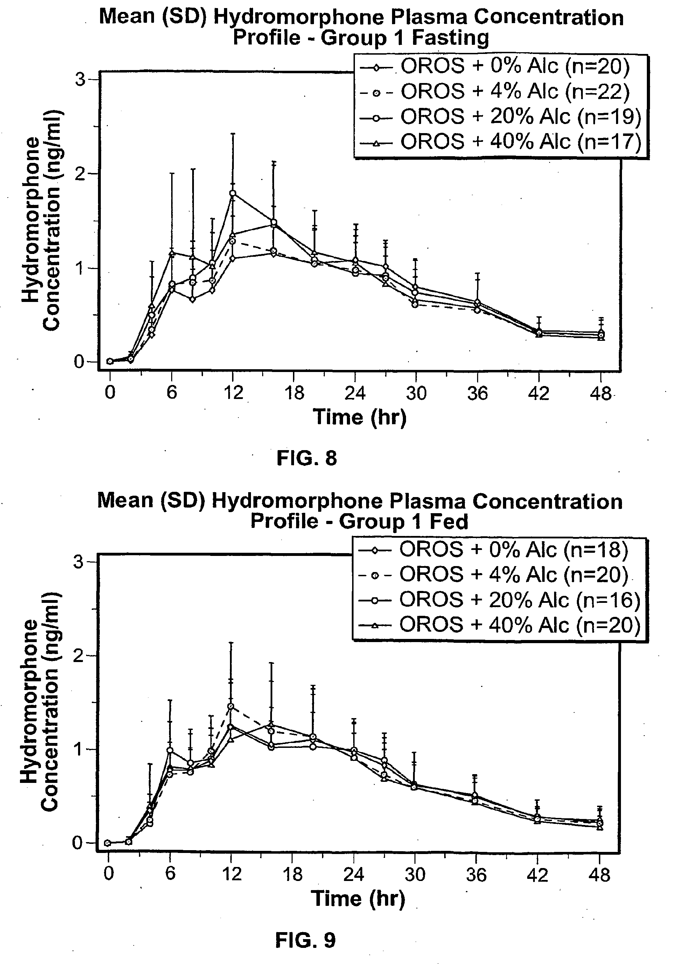

AUC values for the 3 alcohol treatments in relation to the 0% alcohol treatment met the 80 to 125% bioequivalence criteria for the confidence interval in both the fed and fasted states. Figure 8 presents the mean concentration profile following the 4 treatments that were administered in the fasting state (Group 1). Table 3 summarizes the pharmacokinetic parameters. Figure 9 presents the mean concentration profile following the 4 treatments in Group 2 in which all treatments were administered after a standard breakfast. Table 4 summarizes the PK parameters.

### Example 6

### Individual Ratios Comparison: Alcohol Study vs. Replicate Dosing Study

A study was conducted to evaluate bioequivalence between lots produced in two different sites (Lot A vs Lot B). This was a four period, replicate study design in which each of the two lots was administered on two different occasions with washout between treatments to characterize the inter- and intra-subject variability pharmacokinetic variability of dosing in healthy subjects.

Drug supplies for Lot A and Lot B were manufactured as oral osmotic sustained release dosage forms according to the invention, generally according to the methods and techniques laid out in Examples 1 and 4.

Each subject received each of the following treatments twice, in a four period sequence determined by a randomization schedule:
Treatment A: Lot A, with naltrexone HCI 50 mg
Treatment B: Lot B, with naltrexone HCI 50 mg

Naltrexone 50 mg was administered 12 hours prior to and at the time that the inventive hydromorphone dosage forms were administered. Additional 50 mg doses of naltrexone was administered 12 and 24 hours following hydromorphone administration as needed. There was a minimum of a seven day washout period between doses.

Plasma from the timed blood samples collected after drug administration were assayed for hydromorphone concentrations from which Cmax, Tmax, terminal half-life (t1/2), and area under the concentration-time curve (AUC0-72 and AUC0-inf) were determined.

Ten millimeter samples of venous blood were drawn into sample tubes containing anticoagulants at each sampling time. Samples were centrifuged within 1 hour of collection and stored at -40 Deg C until assayed. Blood samples were to be drawn during each dosing period at 0 (prior to dosing), 2, 4, 6, 8, 10, 12, 16, 20, 24, 36, 42, 48, 56, 64, and 72 hours after each dosing of hydromorphone dosage forms according to the invention. Plasma samples were analyzed using a validated liquid chromatography-tandem mass spectrometry (LC/MS/MS) method developed at CEDRA Corporation.

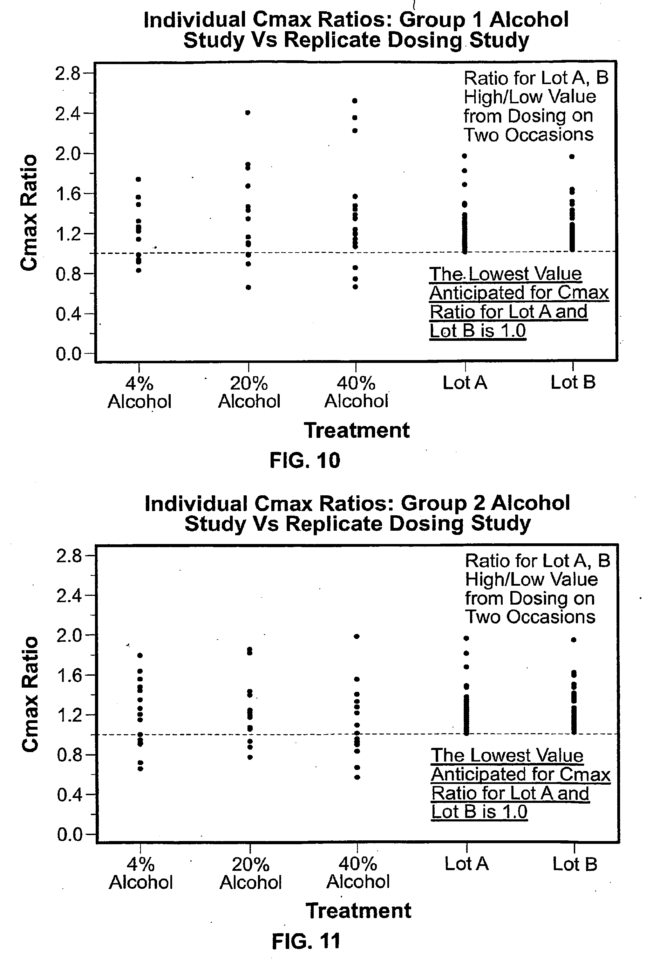

The Cmax ratio from this replicate administration represents intra-individual variability. From this study the ratio of Cmax values (high value /low value) was estimated for each individual and compared to the ratio of Cmax values (alcohol / no alcohol treatment) from the previous Example. Figures 10 and 11 present this comparison for Group 1 and 2, respectively, from Example 5. As shown in these figures the range of Cmax ratio observed with alcohol vs no alcohol treatment is in the same range of ratios that represent intra-individual variability. In addition, these figures show a ratio of an individual test subject's single dose maximum plasma hydromorphone concentration following co-ingestion of the dosage form with alcohol to the same test subject's single dose maximum plasma hydromorphone concentration following co-ingestion of the dosage form with the same aqueous solution (i.e., orange juice) without alcohol that is less than about 2.5:1.

### Example 7

### Study as to the Effect of Stiffening Agents and Acrylic Resin on Oxycodone Release in Water and in 40/60 (% Volume) Ethanol/Water

Ten grams each of formulations with and without stearyl alcohol were prepared via wet granulation technique. The amounts set forth in Tables 5 and 6 of oxycodone hydrochloride, lactose and Eudragit® RS PO were combined in an appropriate vessel and blended for 5 minutes., The powder mixture was granulated with water until a moist mass was produced. The wet mass was then passed through a 16-mesh size screen and was allowed to dry overnight at ambient conditions. In a small vessel, the required amount of stearyl alcohol (a stiffening agent) was melted over a water bath. Keeping the molten stearyl alcohol in the water bath, the desired amount of dried granules were added and mixed until the granules were sufficiently coated with the molten stearyl alcohol. The mixture was removed from the water bath and allowed to cool at ambient conditions before sizing through a 16-mesh screen. Talc and magnesium stearate were added to the coated granules and blended using a suitable blender. The granules were then compressed to 375 mg tablets using an appropriate tabletting machine, such as a Carver press. For granules that were not coated with stearyl alcohol, granules were compressed to 300 mg tablets.

### Example 8

### Hydromorphone HCl Formulations with and without Stearyl Alcohol

The same manufacturing procedure as Example 7 was used, with the substitution of hydromorphone HCl instead of oxycodone HCl. The compositions used are set forth in Tables 7 and 8.

### Example 9

### Release Functionalities of Opioids From Formulations with and without Stearyl Alcohol

Samples for this test came from Examples 7 and 8. Tablets were release tested via USP Type VII. The release media used were as follows: Ethanol data: Ethanol = 40% EtOH / Water = 0 - 4 hrs, and then water = 4 - 24 hrs; Water Data: water used as media for all Intervals. Drug assay was performed in an analytical laboratory via HPLC methods (LAR 007411, AAM1.773v1, AAM1.585v50).

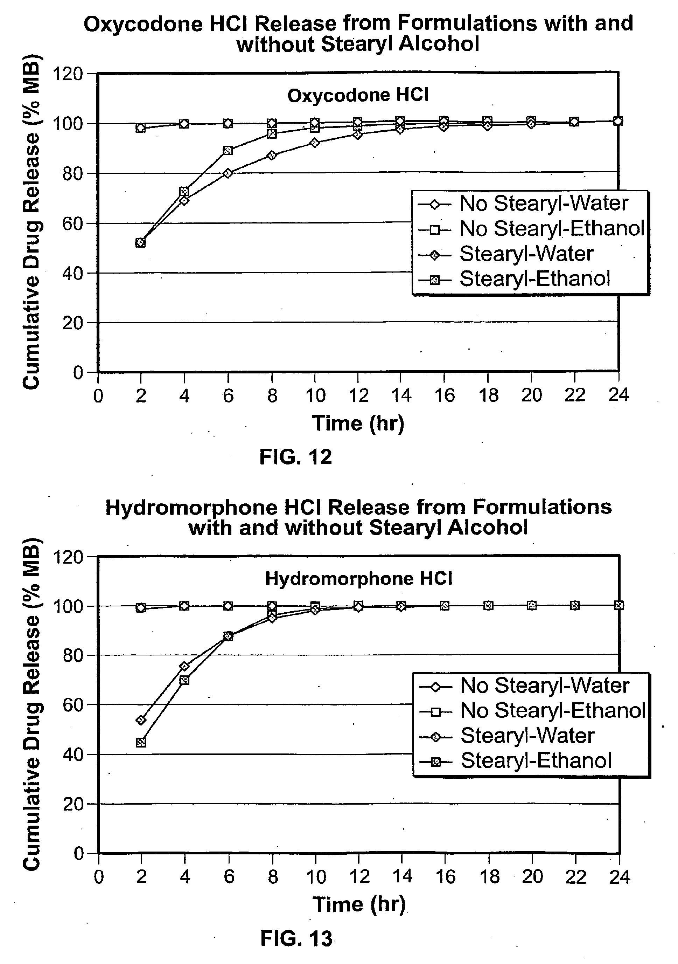

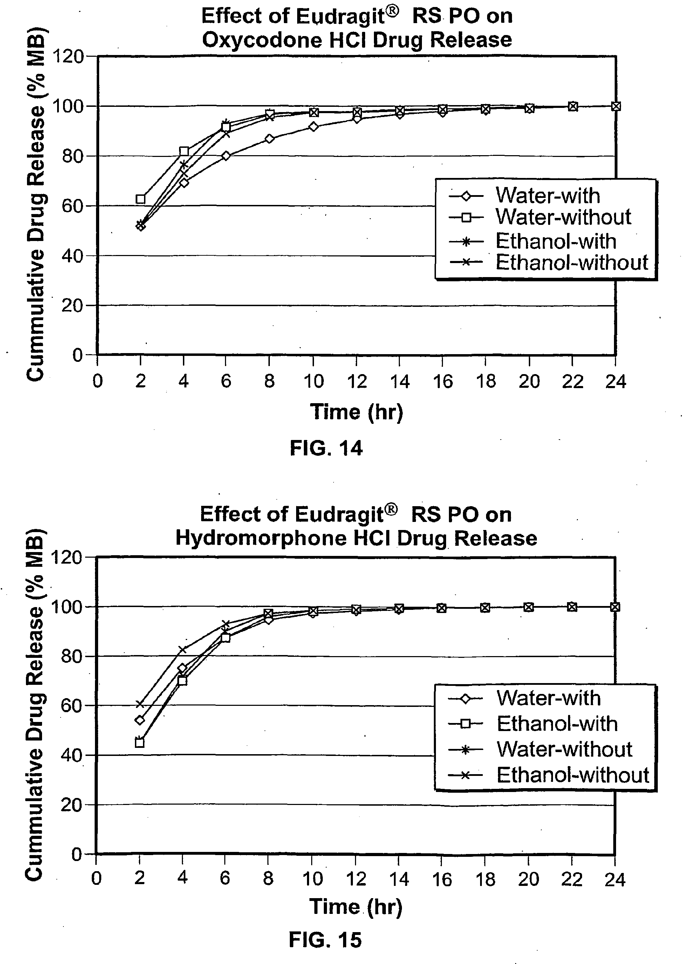

Result: Stearyl alcohol suppressed ethanol effect on opioid functionality, as seen in Figures 12 and 13. The abbreviation "% MB" which appears in the labels for the y-axes of these figures and Figures 14-16 indicates that the drug release data was normalized to 100%.

### Example 10

### Effect of Eudragit® RS PO on Opioid Functionality

The same wet granulation method as detailed in Example 7 was followed to make the granulations. However, Eudragit RS PO was omitted from the powder blends. The tablet weight was adjusted so as to obtain 25 mg of opioids in each tablet. The formulations are as shown in Tables 9 and 10.

Results: As shown in Figure 14, the absence of Eudragit® RS PO in the formulation had no effect on oxycodone HCl functionality in either water or water/ethanol media. As shown in Figure 15, the absence of Eudragit® RS PO in the formulation had no effect on hydromorphone HCl functionality in either water or water/ethanol media.

### Example 11

### Relative Effects of Stearyl Alcohol, Hydrogenated Polyoxyl 60 Castor Oil and Carnauba Wax on Oxycodone HCl Functionality

The same wet granulation method as detailed in Example 7 was followed to make the granulations. However, stearyl alcohol was replaced with either hydrogenated polyoxyl 60 castor oil or carnauba wax. The tablet weight was maintained at 375 mg to obtain 30 mg of opioids in each tablet. The formulations are as shown in Table 11. The tablets are released in the following media; Ethanol Data: 40% EtOH / Water = 0 - 4 hrs, water = 4 - 24 hrs, Water Data: Water used as Media for all Intervals

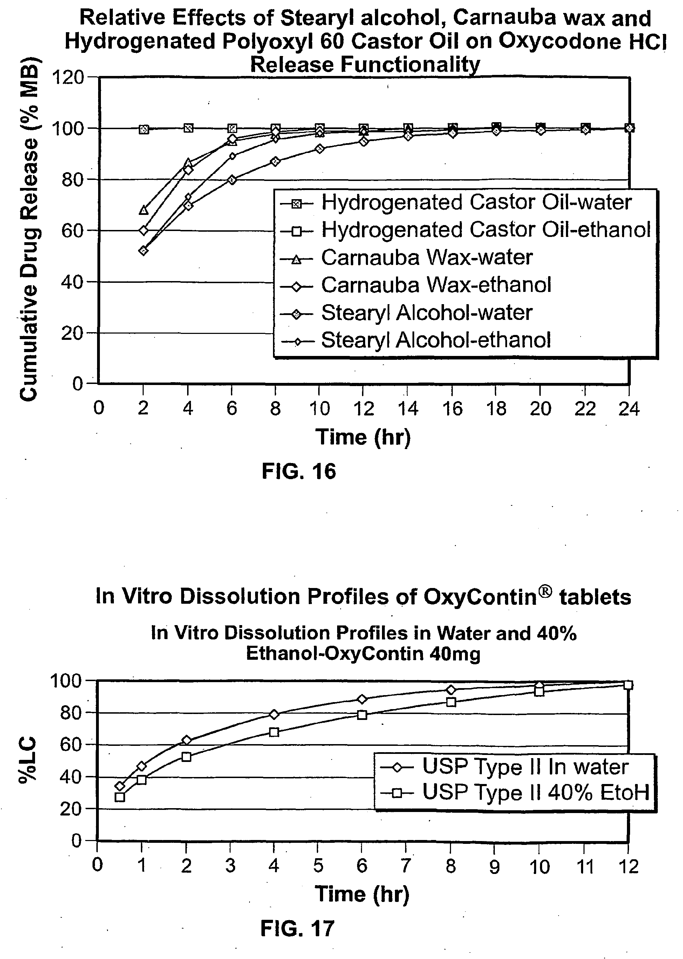

Result: As shown in Figure 16, carnauba wax could be used as a replacement for stearyl alcohol, but not hydrogenated polyoxyl castor oil.

### Example 12

### Testing of OxyContin® Dosage Forms

In vitro dissolution testing of OxyContin® dosage forms was performed substantially under the following conditions:

**Dissolution Conditions:**

| | |
|---|---|
| Apparatus: | USP Type II |
| Paddle Speed: | 50 rpm |
| Volume: | 900 mL |
| Bath Temperature: | 37 ± 0.5 °C |
| Sample Volume: | 5 mL |
| Dissolution Media: | Analytical Grade Water and 40% Ethanol respectively (n=6 tablets per medium) |
| Sampling Interval: | T=0.5, 1, 2, 4, 6, 8, 10 and 12 hours |

Sample solutions were analyzed using a C18 column and detected by UV at 286nm wavelength. Quantitation was performed by linearity curve ranging from 1.05 - 100.53 µg/mL to accommodate sample concentrations. The detailed HPLC conditions for this particular analysis were substantially as follows.

**HPLC Conditions:**

| | |
|---|---|
| Column: | Zorbax Extended C18, 5µ, 50 x 4.6 mm |
| Mobile Phase: | THF:Acetonitrile:34mM Phosphate Buffer |
| | (3:25:72, v/v/v) |
| Flow Rate: | 1.2 mL/min |
| Detector Wavelength: | 286 nm |
| Injection Volume: | 30 µL |
| Column Temperature: | 50°C |
| Run Time: | 4 min |
| The results are shown in Figure 17. | |

### Example 13

### 16-mg Hydromorphone Matrix

A 100-gram blend containing 6 g hydromorphone HCl, 25 g HPMC K100M, 15 g HPMC K3, 5 g PVP K29-32, 2 g magnesium stearate, and 47 g microcrystalline cellulose is dry blended in a roller mill for 3 minutes. 267-mg samples are weighed out and then compressed on a Carver press with 11/32" standard round tooling and ½ ton compression force to produce extended release tablets.

### Example 14

### 40-mg Oxycodone Matrix

A 100-gram blend containing 15 g oxycodone HCl, 25 g HPMC K100M, 15 g HPMC K3, 5 g PVP K29-32, 2 g magnesium stearate, and 38 g microcrystalline cellulose is dry blended in a roller mill for 3 minutes. 267-mg samples are weighed out and then compressed on a Carver press with 11/32" standard round tooling and ½ ton compression force to produce extended release tablets.

### Example 15

### 90-mg Morphine Sulphate Matrix

A 100-gram blend containing 18 g morphine sulphate, 25 g HPMC K100M, 15 g HPMC K3, 5 g PVP K29-32, 2 g magnesium stearate, and 35 g microcrystalline cellulose is dry blended in a roller mill for 3 minutes. 500-mg samples are weighed out and then compressed on a Carver press with 13/32" standard round tooling and 3/4 ton compression force to produce extended release tablets.

### Example 16

### 40 mg Oxymorphone HCl Matrix

A 100-gram blend containing 15 g oxymorphone HCl, 25 g HPMC K100M, 15 g HPMC K3, 5 g PVP K29-32, 2 g magnesium stearate, and 38 g microcrystalline cellulose is dry blended in a roller mill for 3 minutes. 267-mg samples are weighed out and then compressed on a Carver press with 11/32" standard round tooling and ½ ton compression force to produce extended release tablets.

### Example 17

### 40 mg Hydrocodone Bitratrate Matrix

A 100-gram blend containing 15 g hydrocodone bitartrate, 25 g HPMC K100M, 15 g HPMC K3, 5 g PVP K29-32, 2 g magnesium stearate, and 38 g microcrystalline cellulose is dry blended in a roller mill for 3 minutes. 267-mg samples are weighed out and then compressed on a Carver press with 11/32" standard round tooling and ½ ton compression force to produce extended release tablets.

### Example 18

### OROS® Oxycodone 40 mg System

First, a drug composition is prepared by dry blending the following materials: 135.6 g of polyethylene oxide N-150, 54 g of oxycodone hydrochloride, and 8 g povidone (polyvinylpyrrolidone). While mixing in a KitchenAid planetary mixer, 70 g of ethanol is slowly added. The resulting wet granulation is sized through a 16-mesh box sieve, spread into a pan, dried in air at room temperature, and then sized a second time through a 16-mesh box sieve. Finally, the material is returned to the mixer and 0.5 g of magnesium stearate is blended into it for 1 minute.

Next, a push composition is prepared by dry blending the following materials in a KitchenAid planetary mixer: 147.5 g of polyethylene oxide having a molecular weight of 7000K, 40 g sodium chloride powder, 8 g of povidone K29-32, and 2 g of green iron oxide. While mixing, 100 g of ethanol is slowly added. The resulting wet granulation is sized through a 16-mesh box sieve, spread into a pan, dried in air at room temperature and then sized a second time through a 16-mesh box sieve. Finally, the material is placed in the mixer and 0.5 g of magnesium stearate is blending into it for 1 minute.

Next, the oxycodone drug composition and the push composition are compressed into bilayer cores. First, 148 mg of the oxycodone drug composition is added to the die cavity and pre-compressed, then 123 mg of the push composition is added and the layers were pressed into 11/32" diameter, standard concave, bilayer arrangements.

The bilayer arrangements are coated with a semi-permeable wall. The wall forming composition comprised of 99% cellulose acetate identified as 398-10 and having an average acetyl content of 39.8%, and 1% polyethylene glycol identified as 3350 and having an average molecular weight of 3350. The wall-forming composition is dissolved in a 96% acetone and 4% water mixture to make a 6% solids solution. The wall-forming composition is sprayed onto and around the bilayered arrangements in a pan coater until approximately 43 mg of membrane is applied to each tablet.

One 1.0 mm exit passageway is drilled through the semi-permeable wall to connect the drug layer with the exterior of the dosage system. The residual solvent is removed by drying for 72 hours at 45°C and 45% relative humidity. After humidity drying, the tablets are dried for 4 hours at 45°C and ambient humidity.

### Example 19

### 90 mg OROS® Morphine Sulphate

First, a drug composition is prepared by dry blending the following materials: 135.6 g of polyethylene oxide N-80, 54 g of morphine sulphate, and 8 g povidone (polyvinylpyrrolidone). While mixing in a KitchenAid planetary mixer, 70 g of ethanol is slowly added. The resulting wet granulation is sized through a 16-mesh box sieve, spread into a pan, dried in air at room temperature, and then sized a second time through a 16-mesh box sieve. Finally, the material is returned to the mixer and 0.5 g of magnesium stearate is blended into it for 1 minute.

Next, a push composition is prepared by dry blending the following materials in a KitchenAid planetary mixer: 147.5 g of polyethylene oxide having a molecular weight of 7000K, 40 g sodium chloride powder, 8 g of povidone K29-32, and 2 g of green iron oxide. While mixing, 100 g of ethanol is slowly added. The resulting wet granulation is sized through a 16-mesh box sieve, spread into a pan, dried in air at room temperature and then sized a second time through a 16-mesh box sieve. Finally, the material is placed in the mixer and 0.5 g of magnesium stearate is blending into it for 1 minute.

Next, the morphine sulphate drug composition and the push composition are compressed into bilayer cores. First, 333 mg of the morphine sulphate drug composition is added to the die cavity and pre-compressed, then 280 mg of the push composition is added and the layers were pressed into 7/16" diameter, standard concave, bilayer arrangements.

The bilayer arrangements are coated with a semi-permeable wall. The wall forming composition comprised of 95% cellulose acetate identified as 398-10 and having an average acetyl content of 39.8%, and 5% polyethylene glycol identified as 3350 and having an average molecular weight of 3350. The wall-forming composition is dissolved in a 96% acetone and 4% water mixture to make a 6% solids solution. The wall-forming composition is sprayed onto and around the bilayered arrangements in a pan coater until approximately 33 mg of membrane is applied to each tablet.

One 1.0 mm exit passageway is drilled through the semi-permeable wall to connect the drug layer with the exterior of the dosage system. The residual solvent is removed by drying for 72 hours at 45°C and 45% relative humidity. After humidity drying, the tablets are dried for 4 hours at 45°C and ambient humidity.

### Example 20

### 40 mg OROS® Oxymorphone HCl

First, a drug composition is prepared by dry blending the following materials: 135.6 g of polyethylene oxide N-80, 54 g of oxymorphone hydrochloride, and 8 g povidone (polyvinylpyrrolidone). While mixing in a KitchenAid planetary mixer, 70 g of ethanol is slowly added. The resulting wet granulation is sized through a 16-mesh box sieve, spread into a pan, dried in air at room temperature, and then sized a second time through a 16-mesh box sieve. Finally, the material is returned to the mixer and 0.5 g of magnesium stearate is blended into it for 1 minute.

Next, a push composition is prepared by dry blending the following materials in a KitchenAid planetary mixer: 147.5 g of polyethylene oxide having a molecular weight of 7000K, 40 g sodium chloride powder, 8 g of povidone K29-32, and 2 g of green iron oxide. While mixing, 100 g of ethanol is slowly added. The resulting wet granulation is sized through a 16-mesh box sieve, spread into a pan, dried in air at room temperature and then sized a second time through a 16-mesh box sieve. Finally, the material is placed in the mixer and 0.5 g of magnesium stearate is blending into it for 1 minute.

Next, the oxymorphine HCl drug composition and the push composition are compressed into bilayer cores. First, 148 mg of the oxycodone drug composition is added to the die cavity and pre-compressed, then 123 mg of the push composition is added and the layers were pressed into 11/32" diameter, standard concave, bilayer arrangements.

The bilayer arrangements are coated with a semi-permeable wall. The wall forming composition comprised of 99% cellulose acetate identified as 398-10 and having an average acetyl content of 39.8%, and 1% polyethylene glycol identified as 3350 and having an average molecular weight of 3350. The wall-forming composition is dissolved in a 96% acetone and 4% water mixture to make a 6% solids solution. The wall-forming composition is sprayed onto and around the bilayered arrangements in a pan coater until approximately 43 mg of membrane is applied to each tablet.

One 1.0 mm exit passageway is drilled through the semi-permeable wall to connect the drug layer with the exterior of the dosage system. The residual solvent is removed by drying for 72 hours at 45°C and 45% relative humidity. After humidity drying, the tablets are dried for 4 hours at 45°C and ambient humidity.

### Example 21

### 40 mg OROS® Hydrocodone Bitartrate

First, a drug composition is prepared by dry blending the following materials: 135.6 g of polyethylene oxide N-80, 54 g of hydrocodone bitartrate, and 8 g povidone (polyvinylpyrrolidone). While mixing in a KitchenAid planetary mixer, 70 g of ethanol is slowly added. The resulting wet granulation is sized through a 16-mesh box sieve, spread into a pan, dried in air at room temperature, and then sized a second time through a 16-mesh box sieve. Finally, the material is returned to the mixer and 0.5 g of magnesium stearate is blended into it for 1 minute.

Next, a push composition is prepared by dry blending the following materials in a KitchenAid planetary mixer: 147.5 g of polyethylene oxide having a molecular weight of 7000K, 40 g sodium chloride powder, 8 g of povidone K29-32, and 2 g of green iron oxide. While mixing, 100 g of ethanol is slowly added. The resulting wet granulation is sized through a 16-mesh box sieve, spread into a pan, dried in air at room temperature and then sized a second time through a 16-mesh box sieve. Finally, the material is placed in the mixer and 0.5 g of magnesium stearate is blending into it for 1 minute.

Next, the hydrocodone bitartrate drug composition and the push composition are compressed into bilayer cores. First, 148 mg of the oxycodone drug composition is added to the die cavity and pre-compressed, then 123 mg of the push composition is added and the layers were pressed into 11/32" diameter, standard concave, bilayer arrangements.

The bilayer arrangements are coated with a semi-permeable wall. The wall forming composition comprised of 99% cellulose acetate identified as 398-10 and having an average acetyl content of 39.8%, and 1% polyethylene glycol identified as 3350 and having an average molecular weight of 3350. The wall-forming composition is dissolved in a 96% acetone and 4% water mixture to make a 6% solids solution. The wall-forming composition is sprayed onto and around the bilayered arrangements in a pan coater until approximately 43 mg of membrane is applied to each tablet.

One 1.0 mm exit passageway is drilled through the semi-permeable wall to connect the drug layer with the exterior of the dosage system. The residual solvent is removed by drying for 72 hours at 45°C and 45% relative humidity. After humidity drying, the tablets are dried for 4 hours at 45°C and ambient humidity.

### V. FEATURE OF THE INVENTION

From the foregoing, it can be seen that the features of the invention include, without limitation, the following:
A1. A method comprising:
   providing a once-per-day hydromorphone sustained release dosage form comprising hydromorphone and a sustained release dosing structure that provides once-per-day dosing;
   coadministering the once-per-day hydromorphone sustained release dosage form with aqueous alcohol to a patient;
   releasing hydromorphone from the once-per-day hydromorphone sustained release dosage form;
   wherein the aqueous alcohol comprises alcohol at concentrations equal to or greater than about 20% volume/volume; and
   wherein a ratio of a mean single dose maximum plasma hydromorphone concentration achieved when the once-per-day hydromorphone sustained release dosage form is coadministered to the patient with the aqueous alcohol to a mean single dose maximum plasma hydromorphone concentration achieved when the once-per-day hydromorphone sustained release dosage form is administered to a patient without coadministration of the aqueous alcohol is equal to or less than about 1.8:1.
A2. The method of Feature A1, wherein the sustained release dosing structure comprises an osmotic oral sustained release dosing structure.
A3. The method of Feature A1, wherein the alcohol comprises ethanol.
A4. The method of Feature A1, wherein the ratio of a mean single dose maximum plasma hydromorphone concentration achieved when the once-per-day hydromorphone sustained release dosage form is coadministered to the patient with the aqueous alcohol to a mean single dose maximum plasma hydromorphone concentration achieved when the once-per-day hydromorphone sustained release dosage form is administered to a patient without coadministration of the aqueous alcohol is equal to or less than about 1.6:1.
A5. The method of Feature A4, wherein the ratio of a mean single dose maximum plasma hydromorphone concentration achieved when the once-per-day hydromorphone sustained release dosage form is coadministered to the patient with the aqueous alcohol to a mean single dose maximum plasma hydromorphone concentration achieved when the once-per-day hydromorphone sustained release dosage form is administered to a patient without coadministration of the aqueous alcohol is equal to or less than about 1.4:1.
A6. The method of Feature A1, wherein the once-per-day hydromorphone sustained release dosage form comprises hydromorphone in an amount ranging from about 1 mg to about 100 mg.
A7. The method of Feature A6, wherein the once-per-day hydromorphone sustained release dosage form comprises hydromorphone in an amount ranging from about 1 mg to about 50 mg.
A8. The method of Feature A1, wherein the once-per-day hydromorphone sustained release dosage form further comprises an immediate release component for immediate release of the hydromorphone.
A9. The method of Feature A1, wherein the once-per-day hydromorphone sustained release dosage form further comprises an opioid antagonist.
A10. The method of Feature A1, wherein releasing hydromorphone from the hydromorphone sustained release dosage form comprises sustainably releasing hydromorphone from the sustained release dosing structure that provides once-per-day dosing.
A11. The method of Feature A1, wherein the aqueous alcohol comprises alcohol at concentrations equal to or greater than about 30% volume/volume.
A12. The method of Feature A11, wherein the aqueous alcohol comprises alcohol at concentrations equal to or greater than about 40% volume/volume.
A13. A method comprising:
   providing a once-per-day hydromorphone sustained release dosage form comprising hydromorphone and a sustained release dosing structure that provides once-per-day dosing;
   coadministering the once-per-day hydromorphone sustained release dosage form with aqueous alcohol to a patient;
   releasing hydromorphone from the once-per-day hydromorphone sustained release dosage form;
   wherein the aqueous alcohol comprises alcohol at concentrations equal to or greater than about 20% volume/volume; and
   wherein a ratio of an individual patient single dose maximum plasma hydromorphone concentration achieved when the once-per-day hydromorphone sustained release dosage form is coadministered to the patient with the aqueous alcohol to an individual patient single dose maximum plasma hydromorphone concentration achieved when the once-per-day hydromorphone sustained release dosage form is administered to a patient without coadministration of the aqueous alcohol is equal to or less than about 5:1.
A14. The method of Feature A13, wherein the sustained release dosing structure comprises an osmotic oral sustained release dosing structure.
A15. The method of Feature A13, wherein the alcohol comprises ethanol.
A16. The method of Feature A13, wherein the ratio of an individual patient single dose maximum plasma hydromorphone concentration achieved when the once-per-day hydromorphone sustained release dosage form is coadministered to the patient with the aqueous alcohol to an individual patient single dose maximum plasma hydromorphone concentration achieved when the once-per-day hydromorphone sustained release dosage form is administered to a patient without coadministration of the aqueous alcohol is equal to or less than about 4:1.
A17. The method of Feature A16, wherein the ratio of an individual patient single dose maximum plasma hydromorphone concentration achieved when the once-per-day hydromorphone sustained release dosage form is coadministered to the patient with the aqueous alcohol to an individual patient single dose maximum plasma hydromorphone concentration achieved when the once-per-day hydromorphone sustained release dosage form is administered to a patient without coadministration of the aqueous alcohol is equal to or less than about 3:1.
A18. The method of Feature A13, wherein the once-per-day hydromorphone sustained release dosage form comprises hydromorphone in an amount ranging from about 1 mg to about 100 mg.
A19. The method of Feature A18, wherein the once-per-day hydromorphone sustained release dosage form comprises hydromorphone in an amount ranging from about 1 mg to about 50 mg.
A20. The method of Feature A13, wherein the once-per-day hydromorphone sustained release dosage form further comprises an immediate release component for immediate release of the hydromorphone.
A21. The method of Feature A13, wherein the once-per-day hydromorphone sustained release dosage form further comprises an opioid antagonist.
A22. The method of Feature A 13, wherein releasing hydromorphone from the hydromorphone sustained release dosage form comprises sustainably releasing hydromorphone from the sustained release dosing structure that provides once-per-day dosing.
A23. The method of Feature A13, wherein the aqueous alcohol comprises alcohol at concentrations equal to or greater than about 30% volume/volume.
A24. The method of Feature A23, wherein the aqueous alcohol comprises alcohol at concentrations equal to or greater than about 40% volume/volume.
A25. A method comprising:
   providing a once-per-day hydromorphone sustained release dosage form comprising hydromorphone and a sustained release dosing structure that provides once-per-day dosing;
   coadministering the once-per-day hydromorphone sustained release dosage form with aqueous alcohol to a patient;
   releasing hydromorphone from the once-per-day hydromorphone sustained release dosage form;
   wherein the once-per-day hydromorphone sustained release dosage form releases less than or equal to about 80 weight percent of the dose of hydromorphone from the once-per-day hydromorphone sustained release dosage form as measured (a) using an in vitro test method that comprises test media and (b) in a period of about 2 hours following initiation of the in vitro test method; and
   wherein the test media comprises aqueous alcohol that comprises alcohol at concentrations equal to or greater than about 20% volume/volume.
A26. The method of Feature A25, wherein the sustained release dosing structure comprises an osmotic oral sustained release dosing structure.
A27. The method of Feature A25, wherein the alcohol comprises ethanol.
A28. The method of Feature A25, wherein the once-per-day hydromorphone sustained release dosage form releases less than or equal to about 50 weight percent of the dose of hydromorphone from the once-per-day hydromorphone sustained release dosage form.
A29. The method of Feature A28, wherein the once-per-day hydromorphone sustained release dosage form releases less than or equal to about 25 weight percent of the dose of hydromorphone from the once-per-day hydromorphone sustained release dosage form.
A30. The method of Feature A25, wherein the once-per-day hydromorphone sustained release dosage form comprises hydromorphone in an amount ranging from about 1 mg to about 100 mg.
A31. The method of Feature A30, wherein the once-per-day hydromorphone sustained release dosage form comprises hydromorphone in an amount ranging from about 1 mg to about 50 mg.
A32. The method of Feature A25, wherein the once-per-day hydromorphone sustained release dosage form further comprises an immediate release component for immediate release of the hydromorphone.
A33. The method of Feature A25, wherein the once-per-day hydromorphone sustained release dosage form further comprises an opioid antagonist.
A34. The method of Feature A25, wherein releasing hydromorphone from the hydromorphone sustained release dosage form comprises sustainably releasing hydromorphone from the sustained release dosing structure that provides once-per-day dosing.
A35. The method of Feature A25, wherein the aqueous alcohol comprises alcohol at concentrations equal to or greater than about 30% volume/volume.
A36. The method of Feature A35, wherein the aqueous alcohol comprises alcohol at concentrations equal to or greater than about 40% volume/volume.
A37. A method comprising:
   providing a hydromorphone sustained release dosage form comprising hydromorphone and a sustained release dosing structure;
   coadministering the hydromorphone sustained release dosage form with aqueous alcohol to a patient;
   releasing hydromorphone from the hydromorphone sustained release dosage form;
   wherein the aqueous alcohol comprises alcohol at concentrations equal to or greater than about 20% volume/volume; and
   wherein a ratio of a mean single dose maximum plasma hydromorphone concentration achieved when the dosage form is coadministered to the patient with the aqueous alcohol to a mean single dose maximum plasma hydromorphone concentration achieved when the dosage form is administered to a patient without coadministration of the aqueous alcohol is equal to or less than about 1.8:1.
A38. The method of Feature A37, wherein the sustained release dosing structure comprises an osmotic oral sustained release dosing structure.
A39. The method of Feature A37, wherein the alcohol comprises ethanol.
A40. The method of Feature A37, wherein the ratio of a mean single dose maximum plasma hydromorphone concentration achieved when the hydromorphone sustained release dosage form is coadministered to the patient with the aqueous alcohol to a mean single dose maximum plasma hydromorphone concentration achieved when the hydromorphone sustained release dosage form is administered to a patient without coadministration of the aqueous alcohol is equal to or less than about 1.6:1.
A41. The method of Feature A40, wherein the ratio of a mean single dose maximum plasma hydromorphone concentration achieved when the hydromorphone sustained release dosage form is coadministered to the patient with the aqueous alcohol to a mean single dose maximum plasma hydromorphone concentration achieved when the hydromorphone sustained release dosage form is administered to a patient without coadministration of the aqueous alcohol is equal to or less than about 1.4:1.
A42. The method of Feature A37, wherein the hydromorphone sustained release dosage form comprises hydromorphone in an amount ranging from about 1 mg to about 100 mg.
A43. The method of Feature A42, wherein the hydromorphone sustained release dosage form comprises hydromorphone in an amount ranging from about 1 mg to about 50 mg.
A44. The method of Feature A37, wherein the hydromorphone sustained release dosage form comprises a once-per-day hydromorphone sustained release dosage form.
A45. The method of Feature A37, wherein the hydromorphone sustained release dosage form comprises a twice-per-day hydromorphone sustained release dosage form.
A46. The method of Feature A37, wherein the hydromorphone sustained release dosage form further comprises an immediate release component for immediate release of the hydromorphone.
A47. The method of Feature A37, wherein the hydromorphone sustained release dosage form further comprises an opioid antagonist.
A48. The method of Feature A37, wherein releasing hydromorphone from the hydromorphone sustained release dosage form comprises sustainably releasing hydromorphone from the sustained release dosing structure.
A49. The method of Feature A37, wherein the aqueous alcohol comprises alcohol at concentrations equal to or greater than about 30% volume/volume.
A50. The method of Feature A49, wherein the aqueous alcohol comprises alcohol at concentrations equal to or greater than about 40% volume/volume.
A51. A method comprising:
   providing a hydromorphone sustained release dosage form comprising hydromorphone and a sustained release dosing structure;
   coadministering the hydromorphone sustained release dosage form with aqueous alcohol to a patient;
   releasing hydromorphone from the hydromorphone sustained release dosage form;
   wherein the aqueous alcohol comprises alcohol at concentrations equal to or greater than about 20% volume/volume; and
   wherein a ratio of an individual patient single dose maximum plasma hydromorphone concentration achieved when the dosage form is coadministered to the patient with the aqueous alcohol to an individual patient single dose maximum plasma hydromorphone concentration achieved when the dosage form is administered to a patient without coadministration of the aqueous alcohol is equal to or less than about 5:1.
A52. The method of Feature A51, wherein the sustained release dosing structure comprises an osmotic oral sustained release dosing structure.
A53. The method of Feature A51, wherein the alcohol comprises ethanol.
A54. The method of Feature A51, wherein the ratio of an individual patient single dose maximum plasma hydromorphone concentration achieved when the hydromorphone sustained release dosage form is coadministered to the patient with the aqueous alcohol to an individual patient single dose maximum plasma hydromorphone concentration achieved when the hydromorphone sustained release dosage form is administered to a patient without coadministration of the aqueous alcohol is equal to or less than about 4:1.
A55. The method of Feature A54, wherein the ratio of an individual patient single dose maximum plasma hydromorphone concentration achieved when the hydromorphone sustained release dosage form is coadministered to the patient with the aqueous alcohol to an individual patient single dose maximum plasma hydromorphone concentration achieved when the hydromorphone sustained release dosage form is administered to a patient without coadministration of the aqueous alcohol is equal to or less than about 3:1.
A56. The method of Feature A51, wherein the hydromorphone sustained release dosage form comprises hydromorphone in an amount ranging from about 1 mg to about 100 mg.
A57. The method of Feature A56, wherein the hydromorphone sustained release dosage form comprises hydromorphone in an amount ranging from about 1 mg to about 50 mg.
A58. The method of Feature A51, wherein hydromorphone sustained release dosage form further comprises an immediate release component for immediate release of the hydromorphone.
A59. The method of Feature A51, wherein hydromorphone sustained release dosage form further comprises an opioid antagonist.
A60. The method of Feature A51, wherein releasing hydromorphone from the hydromorphone sustained release dosage form comprises sustainably releasing hydromorphone from the sustained release dosing structure.
A61. The method of Feature A51, wherein the aqueous alcohol comprises alcohol at concentrations equal to or greater than about 30% volume/volume.
A62. The method of Feature A61, wherein the aqueous alcohol comprises alcohol at concentrations equal to or greater than about 40% volume/volume.
A63. The method of Feature A51, wherein the hydromorphone sustained release dosage form comprises a once-per-day hydromorphone sustained release dosage form.
A64. The method of Feature A51, wherein the hydromorphone sustained release dosage form comprises a twice-per-day hydromorphone sustained release dosage form.
A65. A method comprising:
   providing a hydromorphone sustained release dosage form comprising a dose of hydromorphone and a sustained release dosing structure;
   coadministering the hydromorphone sustained release dosage form with aqueous alcohol to a patient;
   releasing the dose of hydromorphone from the hydromorphone sustained release dosage form;
   wherein the hydromorphone sustained release dosage form releases less than about 80 weight percent of the dose of hydromorphone from the hydromorphone sustained release dosage form as measured (a) using an in vitro test method that comprises test media and (b) in a period of about 2 hours following initiation of the in vitro test method; and
   wherein the test media comprises aqueous alcohol that comprises alcohol at concentrations equal to or greater than about 20% volume/volume.
A66. The method of Feature A65, wherein the sustained release dosing structure comprises an osmotic oral sustained release dosing structure.
A67. The method of Feature A65, wherein the alcohol comprises ethanol.
A68. The method of Feature A65, wherein the hydromorphone sustained release dosage form releases less than or equal to about 50 weight percent of the dose of hydromorphone from the hydromorphone sustained release dosage form.
A69. The method of Feature A68, wherein the hydromorphone sustained release dosage form releases less than or equal to about 25 weight percent of the dose of hydromorphone from the hydromorphone sustained release dosage form.
A70. The method of Feature A65, wherein the hydromorphone sustained release dosage form comprises hydromorphone in an amount ranging from about 1 mg to about 100 mg.
A71. The method of Feature A70, wherein the hydromorphone sustained release dosage form comprises hydromorphone in an amount ranging from about 1 mg to about 50 mg.
A72. The method of Feature A65, wherein the hydromorphone sustained release dosage form further comprises an immediate release component for immediate release of the hydromorphone.
A73. The method of Feature A65, wherein the hydromorphone sustained release dosage form further comprises an opioid antagonist.
A74. The method of Feature A65, wherein releasing hydromorphone from the hydromorphone sustained release dosage form comprises sustainably releasing hydromorphone from the sustained release dosing structure.
A75. The method of Feature A65, wherein the aqueous alcohol comprises alcohol at concentrations equal to or greater than about 30% volume/volume.
A76. The method of Feature A65, wherein the aqueous alcohol comprises alcohol at concentrations equal to or greater than about 40% volume/volume.
A77. The method of Feature A65, wherein the hydromorphone sustained release dosage form comprises a once-per-day hydromorphone sustained release dosage form.
A78. The method of Feature A65, wherein the hydromorphone sustained release dosage form comprises a twice-per-day hydromorphone sustained release dosage form.
A79. A method comprising:
   providing a once-per-day opioid sustained release dosage form comprising opioid and a sustained release dosing structure that provides once-per-day dosing;
   coadministering the once-per-day opioid sustained release dosage form with aqueous alcohol to a patient;
   releasing opioid from the once-per-day opioid sustained release dosage form;
   wherein the aqueous alcohol comprises alcohol at concentrations equal to or greater than about 20% volume/volume; and
   wherein a ratio of a mean single dose maximum plasma opioid concentration achieved when the once-per-day opioid sustained release dosage form is coadministered to the patient with the aqueous alcohol to a mean single dose maximum plasma opioid concentration achieved when the once-per-day opioid sustained release dosage form is administered to a patient without coadministration of the aqueous alcohol is equal to or less than about 1.8:1.
A80. The method of Feature A79, wherein the sustained release dosing structure comprises an osmotic oral sustained release dosing structure.
A81. The method of Feature A79, wherein the alcohol comprises ethanol.
A82. The method of Feature A79, wherein the ratio of a mean single dose maximum plasma opioid concentration achieved when the once-per-day opioid sustained release dosage form is coadministered to the patient with the aqueous alcohol to a mean single dose maximum plasma opioid concentration achieved when the once-per-day opioid sustained release dosage form is administered to a patient without coadministration of the aqueous alcohol is equal to or less than about 1.6:1.
A83. The method of Feature A82, wherein the ratio of a mean single dose maximum plasma opioid concentration achieved when the once-per-day opioid sustained release dosage form is coadministered to the patient with the aqueous alcohol to a mean single dose maximum plasma opioid concentration achieved when the once-per-day opioid sustained release dosage form is administered to a patient without coadministration of the aqueous alcohol is equal to or less than about 1.4:1.
A84. The method of Feature A79, wherein the once-per-day opioid sustained release dosage form comprises opioid in an amount ranging from about 1 mg to about 100 mg.
A85. The method of Feature A84, wherein the once-per-day opioid sustained release dosage form comprises opioid in an amount ranging from about 1 mg to about 50 mg.
A86. The method of Feature A79, wherein once-per-day opioid sustained release dosage form further comprises an immediate release component for immediate release of the opioid.
A87. The method of Feature A79, wherein once-per-day opioid sustained release dosage form further comprises an opioid antagonist.
A88. The method of Feature A79, wherein releasing opioid from the opioid sustained release dosage form comprises sustainably releasing opioid from the sustained release dosing structure that provides once-per-day dosing.
A89. The method of Feature A79, wherein the aqueous alcohol comprises alcohol at concentrations equal to or greater than about 30% volume/volume.
A90. The method of Feature A89, wherein the aqueous alcohol comprises alcohol at concentrations equal to or greater than about 40% volume/volume.
A91. A method comprising:
   providing a once-per-day opioid sustained release dosage form comprising opioid and a sustained release dosing structure that provides once-per-day dosing;
   coadministering the once-per-day opioid sustained release dosage form with aqueous alcohol to a patient;
   releasing opioid from the once-per-day opioid sustained release dosage form;
   wherein the aqueous alcohol comprises alcohol at concentrations equal to or greater than about 20% volume/volume; and
   wherein a ratio of an individual patient single dose maximum plasma opioid concentration achieved when the once-per-day opioid sustained release dosage form is coadministered to the patient with the aqueous alcohol to an individual patient single dose maximum plasma opioid concentration achieved when the once-per-day opioid sustained release dosage form is administered to a patient without coadministration of the aqueous alcohol is equal to or less than about 5:1.
A92. The method of Feature A91, wherein the sustained release dosing structure comprises an osmotic oral sustained release dosing structure.
A93. The method of Feature A91, wherein the alcohol comprises ethanol.
A94. The method of Feature A91, wherein the ratio of an individual patient single dose maximum plasma opioid concentration achieved when the once-per-day opioid sustained release dosage form is coadministered to the patient with the aqueous alcohol to an individual patient single dose maximum plasma opioid concentration achieved when the once-per-day opioid sustained release dosage form is administered to a patient without coadministration of the aqueous alcohol is equal to or less than about 4:1.
A95. The method of Feature A94, wherein the ratio of an individual patient single dose maximum plasma opioid concentration achieved when the once-per-day opioid sustained release dosage form is coadministered to the patient with the aqueous alcohol to an individual patient single dose maximum plasma opioid concentration achieved when the once-per-day opioid sustained release dosage form is administered to a patient without coadministration of the aqueous alcohol is equal to or less than about 3:1.
A96. The method of Feature A91, wherein the once-per-day opioid sustained release dosage form comprises opioid in an amount ranging from about 1 mg to about 100 mg.
A97. The method of Feature A96, wherein the once-per-day opioid sustained release dosage form comprises opioid in an amount ranging from about 1 mg to about 50 mg.
A98. The method of Feature A91, wherein once-per-day opioid sustained release dosage form further comprises an immediate release component for immediate release of the opioid.
A99. The method of Feature A91, wherein once-per-day opioid sustained release dosage form further comprises an opioid antagonist.
A100. The method of Feature A91, wherein releasing opioid from the opioid sustained release dosage form comprises sustainably releasing opioid from the sustained release dosing structure that provides once-per-day dosing.
A101. The method of Feature A91, wherein the aqueous alcohol comprises alcohol at concentrations equal to or greater than about 30% volume/volume.
A102. The method of Feature A101, wherein the aqueous alcohol comprises alcohol at concentrations equal to or greater than about 40% volume/volume.
A103. A method comprising:
   providing a once-per-day opioid sustained release dosage form comprising opioid and a sustained release dosing structure that provides once-per-day dosing;
   coadministering the once-per-day opioid sustained release dosage form with aqueous alcohol to a patient;
   releasing opioid from the once-per-day opioid sustained release dosage form;
   wherein the once-per-day opioid sustained release dosage form releases less than or equal to about 80 weight percent of the dose of opioid from the once-per-day opioid sustained release dosage form as measured (a) using an in vitro test method that comprises test media and (b) in a period of about 2 hours following initiation of the in vitro test method; and
   wherein the test media comprises aqueous alcohol that comprises alcohol at concentrations equal to or greater than about 20% volume/volume.
A104. The method of Feature A103, wherein the sustained release dosing structure comprises an osmotic oral sustained release dosing structure.
A105. The method of Feature A103, wherein the alcohol comprises ethanol.
A106. The method of Feature A103, wherein the once-per-day opioid sustained release dosage form releases less than or equal to about 50 weight percent of the dose of opioid from the once-per-day opioid sustained release dosage form.
A107. The method of Feature A106, wherein the once-per-day opioid sustained release dosage form releases less than or equal to about 25 weight percent of the dose of opioid from the once-per-day opioid sustained release dosage form.
A108. The method of Feature A103, wherein the once-per-day opioid sustained release dosage form comprises opioid in an amount ranging from about 1 mg to about 100 mg.
A109. The method of Feature A108, wherein the once-per-day opioid sustained release dosage form comprises opioid in an amount ranging from about 1 mg to about 50 mg.
A110. The method of Feature A103, wherein the once-per-day opioid sustained release dosage form further comprises an immediate release component for immediate release of the opioid.
A111. The method of Feature A103, wherein the once-per-day opioid sustained release dosage form further comprises an opioid antagonist.
A112. The method of Feature A103, wherein releasing opioid from the opioid sustained release dosage form comprises sustainably releasing opioid from the sustained release dosing structure that provides once-per-day dosing.
A113. The method of Feature A103, wherein the aqueous alcohol comprises alcohol at concentrations equal to or greater than about 30% volume/volume.
A114. The method of Feature A103, wherein the aqueous alcohol comprises alcohol at concentrations equal to or greater than about 40% volume/volume.
A115. A method comprising:
   providing a opioid sustained release dosage form comprising opioid and a sustained release dosing structure;
   coadministering the opioid sustained release dosage form with aqueous alcohol to a patient;
   releasing opioid from the opioid sustained release dosage form;
   wherein the aqueous alcohol comprises alcohol at concentrations equal to or greater than about 20% volume/volume; and
   wherein a ratio of a mean single dose maximum plasma opioid concentration achieved when the dosage form is coadministered to the patient with the aqueous alcohol to a mean single dose maximum plasma opioid concentration achieved when the dosage form is administered to a patient without coadministration of the aqueous alcohol is equal to or less than about 1.8:1.
A116. The method of Feature A115, wherein the sustained release dosing structure comprises an osmotic oral sustained release dosing structure.
A117. The method of Feature A115, wherein the alcohol comprises ethanol.
A118. The method of Feature A115, wherein the ratio of a mean single dose maximum plasma opioid concentration achieved when the opioid sustained release dosage form is coadministered to the patient with the aqueous alcohol to a mean single dose maximum plasma opioid concentration achieved when the opioid sustained release dosage form is administered to a patient without coadministration of the aqueous alcohol is equal to or less than about 1.6:1.
A119. The method of Feature A118, wherein the ratio of a mean single dose maximum plasma opioid concentration achieved when the opioid sustained release dosage form is coadministered to the patient with the aqueous alcohol to a mean single dose maximum plasma opioid concentration achieved when the opioid sustained release dosage form is administered to a patient without coadministration of the aqueous alcohol is equal to or less than about 1.4:1.
A120. The method of Feature A115, wherein the opioid sustained release dosage form comprises opioid in an amount ranging from about 1 mg to about 100 mg.
A121. The method of Feature A120, wherein the opioid sustained release dosage form comprises opioid in an amount ranging from about 1 mg to about 50 mg.
A122. The method of Feature A115, wherein the opioid sustained release dosage form comprises a once-per-day opioid sustained release-dosage form.
A123. The method of Feature A115, wherein the opioid sustained release dosage form comprises a twice-per-day opioid sustained release dosage form.
A124. The method of Feature A115, wherein the opioid sustained release dosage form further comprises an immediate release component for immediate release of the opioid.
A125. The method of Feature A115, wherein the opioid sustained release dosage form further comprises an opioid antagonist.
A126. The method of Feature A115, wherein releasing opioid from the opioid sustained release dosage form comprises sustainably releasing opioid from the sustained release dosing structure.
A127. The method of Feature A115, wherein the aqueous alcohol comprises alcohol at concentrations equal to or greater than about 30% volume/volume.
A128. The method of Feature A127, wherein the aqueous alcohol comprises alcohol at concentrations equal to or greater than about 40% volume/volume.
A129. A method comprising:
   providing a opioid sustained release dosage form comprising opioid and a sustained release dosing structure;
   coadministering the opioid sustained release dosage form with aqueous alcohol to a patient;
   releasing opioid from the opioid sustained release dosage form;
   wherein the aqueous alcohol comprises alcohol at concentrations equal to or greater than about 20% volume/volume; and
   wherein a ratio of an individual patient single dose maximum plasma opioid concentration achieved when the dosage form is coadministered to the patient with the aqueous alcohol to an individual patient single dose maximum plasma opioid concentration achieved when the dosage form is administered to a patient without coadministration of the aqueous alcohol is equal to or less than about 5:1.
A130. The method of Feature A129, wherein the sustained release dosing structure comprises an osmotic oral sustained release dosing structure.
A131. The method of Feature A129, wherein the alcohol comprises ethanol.
A132. The method of Feature A129, wherein the ratio of an individual patient single dose maximum plasma opioid concentration achieved when the opioid sustained release dosage form is coadministered to the patient with the aqueous alcohol to an individual patient single dose maximum plasma opioid concentration achieved when the opioid sustained release dosage form is administered to a patient without coadministration of the aqueous alcohol is equal to or less than about 4:1.
A133. The method of Feature A132, wherein the ratio of an individual patient single dose maximum plasma opioid concentration achieved when the opioid sustained release dosage form is coadministered to the patient with the aqueous alcohol to an individual patient single dose maximum plasma opioid concentration achieved when the opioid sustained release dosage form is administered to a patient without coadministration of the aqueous alcohol is equal to or less than about 3:1.
A134. The method of Feature A129, wherein the opioid sustained release dosage form comprises opioid in an amount ranging from about 1 mg to about 100 mg.
A135. The method of Feature A134, wherein the opioid sustained release dosage form comprises opioid in an amount ranging from about 1 mg to about 50 mg.
A136. The method of Feature A129, wherein opioid sustained release dosage form further comprises an immediate release component for immediate release of the opioid.
A137. The method of Feature A129, wherein opioid sustained release dosage form further comprises an opioid antagonist.
A138. The method of Feature A129, wherein releasing opioid from the opioid sustained release dosage form comprises sustainably releasing opioid from the sustained release dosing structure.
A139. The method of Feature A129, wherein the aqueous alcohol comprises alcohol at concentrations equal to or greater than about 30% volume/volume.
A140. The method of Feature A139, wherein the aqueous alcohol comprises alcohol at concentrations equal to or greater than about 40% volume/volume.
A141. The method of Feature A129, wherein the opioid sustained release dosage form comprises a once-per-day opioid sustained release dosage form.
A142. The method of Feature A129, wherein the opioid sustained release dosage form comprises a twice-per-day opioid sustained release dosage form.
A143. A method comprising
   providing a opioid sustained release dosage form comprising a dose of opioid and a sustained release dosing structure;
   coadministering the opioid sustained release dosage form with aqueous alcohol to a patient;
   releasing the dose of opioid from the opioid sustained release dosage form;
   wherein the opioid sustained release dosage form releases less than about 80 weight percent of the dose of opioid from the opioid sustained release dosage form as measured (a) using an in vitro test method that comprises test media and (b) in a period of about 2 hours following initiation of the in vitro test method; and
   wherein the test media comprises aqueous alcohol that comprises alcohol at concentrations equal to or greater than about 20% volume/volume.
A144. The method of Feature A143, wherein the sustained release dosing structure comprises an osmotic oral sustained release dosing structure.
A 145. The method of Feature A 143, wherein the alcohol comprises ethanol.
A146. The method of Feature A143, wherein the opioid sustained release dosage form releases less than or equal to about 50 weight percent of the dose of opioid from the opioid sustained release dosage form.
A147. The method of Feature A146, wherein the opioid sustained release dosage form releases less than or equal to about 25 weight percent of the dose of opioid from the opioid sustained release dosage form.
A148. The method of Feature A129, wherein the opioid sustained release dosage form comprises the opioid in an amount ranging from about 1 mg to about 100 mg.
A 149. The method of Feature A 148, wherein the opioid sustained release dosage form comprises opioid in an amount ranging from about 1 mg to about 50 mg.
A150. The method of Feature A129, wherein the opioid sustained release dosage form further comprises an immediate release component for immediate release of the opioid.
A 151. The method of Feature A 129, wherein the opioid sustained release dosage form further comprises an opioid antagonist.
A152. The method of Feature A129, wherein releasing opioid from the opioid sustained release dosage form comprises sustainably releasing opioid from the sustained release dosing structure.
A153. The method of Feature A129, wherein the aqueous alcohol comprises alcohol at concentrations equal to or greater than about 30% volume/volume.
A154. The method of Feature A153, wherein the aqueous alcohol comprises alcohol at concentrations equal to or greater than about 40% volume/volume.
A155. The method of Feature A129, wherein the opioid sustained release dosage form comprises a once-per-day opioid sustained release dosage form.
A156. The method of Feature A129, wherein the opioid sustained release dosage form comprises a twice-per-day opioid sustained release dosage form.
A157. A method comprising:
   providing a once-per-day hydromorphone sustained release dosage form comprising hydromorphone and a sustained release dosing structure that provides once-per-day dosing;
   coadministering the once-per-day hydromorphone sustained release dosage form with aqueous alcohol to a patient;
   releasing hydromorphone from the once-per-day hydromorphone sustained release dosage form;
   wherein the aqueous alcohol comprises alcohol at concentrations equal to or greater than about 20% volume/volume; and
   wherein a ratio of the median single dose, time to maximum plasma concentration achieved when the dosage form is coadministered to the patient with the aqueous alcohol to the median single dose, time to maximum plasma concentration achieved when the once-per-day hydromorphone sustained release dosage form is administered to a patient without coadministration of the aqueous alcohol ranges from about 0.5 to about 1.0.
A158. A method comprising:
   providing a hydromorphone sustained release dosage form comprising hydromorphone and a sustained release dosing structure;
   coadministering the hydromorphone sustained release dosage form with aqueous alcohol to a patient;
   releasing hydromorphone from the hydromorphone sustained release dosage form;
   wherein the aqueous alcohol comprises alcohol at concentrations equal to or greater than about 20% volume/volume; and
   wherein a ratio of the median single dose, time to maximum plasma concentration achieved when the dosage form is coadministered to the patient with the aqueous alcohol to the median single dose, time to maximum plasma concentration achieved when the hydromorphone sustained release dosage form is administered to a patient without coadministration of the aqueous alcohol ranges from about 0.5 to about 1.0.
A159. A method comprising:
   providing a once-per-day opioid sustained release dosage form comprising opioid and a sustained release dosing structure that provides once-per-day dosing;
   coadministering the once-per-day opioid sustained release dosage form with aqueous alcohol to a patient;
   releasing opioid from the once-per-day opioid sustained release dosage form;
   wherein the aqueous alcohol comprises alcohol at concentrations equal to or greater than about 20% volume/volume; and
   wherein a ratio of the median single dose, time to maximum plasma concentration achieved when the dosage form is coadministered to the patient with the aqueous alcohol to the median single dose, time to maximum plasma concentration achieved when the once-per-day opioid sustained release dosage form is administered to a patient without coadministration of the aqueous alcohol ranges from about 0.5 to about 1.0.
A160. A method comprising:
   providing a opioid sustained release dosage form comprising opioid and a sustained release dosing structure;
   coadministering the opioid sustained release dosage form with aqueous alcohol to a patient;
   releasing opioid from the opioid sustained release dosage form;
   wherein the aqueous alcohol comprises alcohol at concentrations equal to or greater than about 20% volume/volume; and
   wherein a ratio of the median single dose, time to maximum plasma concentration achieved when the dosage form is coadministered to the patient with the aqueous alcohol to the median single dose, time to maximum plasma concentration achieved when the opioid sustained release dosage form is administered to a patient without coadministration of the aqueous alcohol ranges from about 0.5 to about 1.0.

Additionally, the features of the invention include, without limitation, the following:
B 1. The use of a once-per-day hydromorphone sustained release dosage form comprising hydromorphone and a sustained release dosing structure that provides once-per-day dosing in combination with aqueous alcohol, for reducing alcohol-induced dose dumping;
   wherein the aqueous alcohol comprises alcohol at concentrations equal to or greater than about 20% volume/volume; and
   wherein a ratio of (i) a mean single dose maximum plasma hydromorphone concentration achieved when the once-per-day hydromorphone sustained release dosage form is used in combination with aqueous alcohol to (ii) a mean single dose maximum plasma hydromorphone concentration achieved when the once-per-day hydromorphone sustained release dosage form is used alone, is equal to or less than about 1.8:1.
B2. The use according to Feature B1, wherein the sustained release dosing structure comprises an osmotic oral sustained release dosing structure.
B3. The use according to Feature B1, wherein the alcohol comprises ethanol.
B4. The use according to Feature B 1, wherein the ratio of (i) a mean single dose maximum plasma hydromorphone concentration achieved when the once-per-day hydromorphone sustained release dosage form is used in combination with aqueous alcohol to (ii) a mean single dose maximum plasma hydromorphone concentration achieved when the once-per-day hydromorphone sustained release dosage form is used alone, is equal to or less than about 1.6:1.
B5. The use according to Feature B4, wherein the ratio of (i) a mean single dose maximum plasma hydromorphone concentration achieved when the once-per-day hydromorphone sustained release dosage form is used in combination with aqueous alcohol to (ii) a mean single dose maximum plasma hydromorphone concentration achieved when the once-per-day hydromorphone sustained release dosage form is used alone, is equal to or less than about 1.4:1.
B6. The use according to Feature B1, wherein the once-per-day hydromorphone sustained release dosage form comprises hydromorphone in an amount ranging from about 1 mg to about 100 mg.
B7. The use according to Feature B6, wherein the once-per-day hydromorphone sustained release dosage form comprises hydromorphone in an amount ranging from about 1 mg to about 50 mg.
B8. The use according to Feature B1, wherein once-per-day hydromorphone sustained release dosage form further comprises an immediate release component for immediate release of the hydromorphone.
B9. The use according to Feature B1, wherein once-per-day hydromorphone sustained release dosage form further comprises an opioid antagonist.
B10. The use according to Feature B1, wherein releasing hydromorphone from the hydromorphone sustained release dosage form comprises sustainably releasing hydromorphone from the sustained release dosing structure that provides once-per-day dosing.
B11. The use according to Feature B 1, wherein the aqueous alcohol comprises alcohol at concentrations equal to or greater than about 30% volume/volume.
B12. The use according to Feature B 11, wherein the aqueous alcohol comprises alcohol at concentrations equal to or greater than about 40% volume/volume.
B13. The use of a once-per-day hydromorphone sustained release dosage form comprising hydromorphone and a sustained release dosing structure, that provides once-per-day dosing, in combination with aqueous alcohol, for reducing alcohol-induced dose dumping;
   wherein the aqueous alcohol comprises alcohol at concentrations equal to or greater than about 20% volume/volume; and
   wherein a ratio of (i) an individual patient single dose maximum plasma hydromorphone concentration achieved when the once-per-day hydromorphone sustained release dosage form is used in combination with the aqueous alcohol to (ii) an individual patient single dose maximum plasma hydromorphone concentration achieved when the once-per-day hydromorphone sustained release dosage form is used alone, is equal to or less than about 5:1.
B14. The use according to Feature B13, wherein the sustained release dosing structure comprises an osmotic oral sustained release dosing structure.
B15. The use according to Feature B13, wherein the alcohol comprises ethanol.
B 16. The use according to Feature B 13, wherein the ratio of (i) an individual patient single dose maximum plasma hydromorphone concentration achieved when the once-per-day hydromorphone sustained release dosage form is used in combination with the aqueous alcohol to (ii) an individual patient single dose maximum plasma hydromorphone concentration achieved when the once-per-day hydromorphone sustained release dosage form is used alone, is equal to or less than about 4:1.
B 17. The use according to Feature B 16, wherein the ratio of (i) an individual patient single dose maximum plasma hydromorphone concentration achieved when the once-per-day hydromorphone sustained release dosage form is used in combination with the aqueous alcohol to (ii) an individual patient single dose maximum plasma hydromorphone concentration achieved when the once-per-day hydromorphone sustained release dosage form is used alone, is equal to or less than about 3:1.
B18. The use according to Feature B 13, wherein the once-per-day hydromorphone sustained release dosage form comprises hydromorphone in an amount ranging from about 1 mg to about 100 mg.
B 19. The use according to Feature B 18, wherein the once-per-day hydromorphone sustained release dosage form comprises hydromorphone in an amount ranging from about 1 mg to about 50 mg.
B20. The use according to Feature B13, wherein once-per-day hydromorphone sustained release dosage form further comprises an immediate release component for immediate release of the hydromorphone.
B21. The use according to Feature B 13, wherein once-per-day Hydromorphone sustained release dosage form further comprises an opioid antagonist.
B22. The use according to Feature B13, wherein releasing hydromorphone from the hydromorphone sustained release dosage form comprises sustainably releasing hydromorphone from the sustained release dosing structure that provides once-per-day dosing.
B23. The use according to Feature B 13, wherein the aqueous alcohol comprises alcohol at concentrations equal to or greater than about 30% volume/volume.
B24. The use according to Feature B23, wherein the aqueous alcohol comprises alcohol at concentrations equal to or greater than about 40% volume/volume.
B25. The use of a once-per-day hydromorphone sustained release dosage form comprising hydromorphone and a sustained release dosing structure that provides once-per-day dosing in combination with aqueous alcohol, for reducing alcohol-induced dose dumping;
   wherein the once-per-day hydromorphone sustained release dosage form releases less than or equal to about 80 weight percent of the dose of hydromorphone from the once-per-day hydromorphone sustained release dosage form as measured (a) using an in vitro test method that comprises test media and (b) in a period of about 2 hours following initiation of the in vitro test method; and
   wherein the test media comprises aqueous alcohol that comprises alcohol at concentrations equal to or greater than about 20% volume/volume.
B26. The use according to Feature B25, wherein the sustained release dosing structure comprises an osmotic oral sustained release dosing structure.
B27. The use according to Feature B25, wherein the alcohol comprises ethanol.
B28. The use according to Feature B25, wherein the once-per-day hydromorphone sustained release dosage form releases less than or equal to about 50 weight percent of the dose of hydromorphone from the once-per-day hydromorphone sustained release dosage form.
B29. The use according to Feature B28, wherein the once-per-day hydromorphone sustained release dosage form releases less than or equal to about 25 weight percent of the dose of hydromorphone from the once-per-day hydromorphone sustained release dosage form.
B30. The use according to Feature B25, wherein the once-per-day hydromorphone sustained release dosage form comprises hydromorphone in an amount ranging from about 1 mg to about 100 mg.
B31. The use according to Feature B30, wherein the once-per-day hydromorphone sustained release dosage form comprises hydromorphone in an amount ranging from about 1 mg to about 50 mg.
B32. The use according to Feature B25, wherein the once-per-day hydromorphone sustained release dosage form further comprises an immediate release component for immediate release of the hydromorphone.
B33. The use according to Feature B25, wherein the once-per-day hydromorphone sustained release dosage form further comprises an opioid antagonist.
B34. The use according to Feature B25, wherein releasing hydromorphone from the hydromorphone sustained release dosage form comprises sustainably releasing hydromorphone from the sustained release dosing structure that provides once-per-day dosing.
B35. The use according to Feature B25, wherein the aqueous alcohol comprises alcohol at concentrations equal to or greater than about 30% volume/volume.
B36. The use according to Feature B25, wherein the aqueous alcohol comprises alcohol at concentrations equal to or greater than about 40% volume/volume.
B37. The use of an hydromorphone sustained release dosage form comprising hydromorphone and a sustained release dosing structure in combination with aqueous alcohol, for reducing alcohol-induced dose dumping;
   wherein the aqueous alcohol comprises alcohol at concentrations equal to or greater than about 20% volume/volume; and
   wherein a ratio of (i) a mean single dose maximum plasma hydromorphone concentration achieved when the dosage form is used in combination with the aqueous alcohol to (ii) a mean single dose maximum plasma hydromorphone concentration achieved when the dosage form is used alone, is equal to or less than about 1.8:1.
B38. The use according to Feature B37, wherein the sustained release dosing structure comprises an osmotic oral sustained release dosing structure.
B39. The use according to Feature B37, wherein the alcohol comprises ethanol.
B40. The use according to Feature B37, wherein the ratio of (i) a mean single dose maximum plasma hydromorphone concentration achieved when the hydromorphone sustained release dosage form is used in combination with the aqueous alcohol to (ii) a mean single dose maximum plasma hydromorphone concentration achieved when the hydromorphone sustained release dosage form is used alone, is equal to or less than about 1.6:1.
B41. The use according to Feature B40, wherein the ratio of (i) a mean single dose maximum plasma hydromorphone concentration achieved when the hydromorphone sustained release dosage form is used in combination with aqueous alcohol to (ii) a mean single dose maximum plasma hydromorphone concentration achieved when the hydromorphone sustained release dosage form is used alone, is equal to or less than about 1.4:1.
B42. The use according to Feature B37, wherein the hydromorphone sustained release dosage form comprises hydromorphone in an amount ranging from about 1 mg to about 100 mg.
B43. The use according to Feature B42, wherein the hydromorphone sustained release dosage form comprises hydromorphone in an amount ranging from about 1 mg to about 50 mg.
B44. The use according to Feature B37, wherein the hydromorphone sustained release dosage form comprises a once-per-day hydromorphone sustained release dosage form.
B45. The use according to Feature B37, wherein the hydromorphone sustained release dosage form comprises a twice-per-day hydromorphone sustained release dosage form.
B46. The use according to Feature B37, wherein the hydromorphone sustained release dosage form further comprises an immediate release component for immediate release of the hydromorphone.
B47. The use according to Feature B37, wherein the hydromorphone sustained release dosage form further comprises an opioid antagonist.
B48. The use according to Feature B37, wherein releasing hydromorphone from the hydromorphone sustained release dosage form comprises sustainably releasing hydromorphone from the sustained release dosing structure.
B49. The use according to Feature B37, wherein the aqueous alcohol comprises alcohol at concentrations equal to or greater than about 30% volume/volume.
B50. The use according to Feature B49, wherein the aqueous alcohol comprises alcohol at concentrations equal to or greater than about 40% volume/volume.
B51. The use of an hydromorphone sustained release dosage form comprising hydromorphone and a sustained release dosing structure in combination with aqueous alcohol, for reducing alcohol-induced dose dumping;
   wherein the aqueous alcohol comprises alcohol at concentrations equal to or greater than about 20% volume/volume; and
   wherein a ratio of (i) an individual patient single dose maximum plasma hydromorphone concentration achieved when the dosage form is used in combination with the aqueous alcohol to (ii) an individual patient single dose maximum plasma hydromorphone concentration achieved when the dosage form is used alone, is equal to or less than about 5:1.
B52. The use according to Feature B51, wherein the sustained release dosing structure comprises an osmotic oral sustained release dosing structure.
B53. The use according to Feature B51, wherein the alcohol comprises ethanol.
B54. The use according to Feature B51, wherein the ratio of (i) an individual patient single dose maximum plasma hydromorphone concentration achieved when the hydromorphone sustained release dosage form is used in combination with the aqueous alcohol to (ii) an individual patient single dose maximum plasma hydromorphone concentration achieved when the hydromorphone sustained release dosage form is used alone, is equal to or less than about 4:1.
B55. The use according to Feature B54, wherein the ratio of (i) an individual patient single dose maximum plasma hydromorphone concentration achieved when the hydromorphone sustained release dosage form is used in combination with the aqueous alcohol to (ii) an individual patient single dose maximum plasma hydromorphone concentration achieved when the hydromorphone sustained release dosage form is used alone, is equal to or less than about 3:1.
B56. The use according to Feature B51, wherein the hydromorphone sustained release dosage form comprises hydromorphone in an amount ranging from about 1 mg to about 100 mg.
B57. The use according to Feature B56, wherein the hydromorphone sustained release dosage form comprises hydromorphone in an amount ranging from about 1 mg to about 50 mg.
B58. The use according to Feature B51, wherein hydromorphone sustained release dosage form further comprises an immediate release component for immediate release of the hydromorphone.
B59. The use according to Feature B51, wherein hydromorphone sustained release dosage form further comprises an opioid antagonist.
B60. The use according to Feature B51, wherein releasing hydromorphone from the hydromorphone sustained release dosage form comprises sustainably releasing hydromorphone from the sustained release dosing structure.
B61. The use according to Feature B51, wherein the aqueous alcohol comprises alcohol at concentrations equal to or greater than about 30% volume/volume.
B62. The use according to Feature B61, wherein the aqueous alcohol comprises alcohol at concentrations equal to or greater than about 40% volume/volume.
B63. The use according to Feature B51, wherein the hydromorphone sustained release dosage form comprises a once-per-day hydromorphone sustained release dosage form.
B64. The use according to Feature B65, wherein the hydromorphone sustained release dosage form comprises a twice-per-day hydromorphone sustained release dosage form.
B65. The use of an hydromorphone sustained release dosage form comprising hydromorphone and a sustained release dosing structure in combination with aqueous alcohol, for reducing alcohol-induced dose dumping;
   wherein the hydromorphone sustained release dosage form releases less than about 80 weight percent of the dose of hydromorphone from the hydromorphone sustained release dosage form as measured (a) using an in vitro test method that comprises test media and (b) in a period of about 2 hours following initiation of the in vitro test method; and
   wherein the test media comprises aqueous alcohol that comprises alcohol at concentrations equal to or greater than about 20% volume/volume.
B66. The use according to Feature B65, wherein the sustained release dosing structure comprises an osmotic oral sustained release dosing structure.
B67. The use according to Feature B65, wherein the alcohol comprises ethanol.
B68. The use according to Feature B67, wherein the hydromorphone sustained release dosage form releases less than or equal to about 50 weight percent of the dose of hydromorphone from the hydromorphone sustained release dosage form.
B69. The use according to Feature B68, wherein the hydromorphone sustained release dosage form releases less than or equal to about 25 weight percent of the dose of hydromorphone from the hydromorphone sustained release dosage form.
B70. The use according to Feature B65, wherein the hydromorphone sustained release dosage form comprises hydromorphone in an amount ranging from about 1 mg to about 100 mg.
B71. The use according to Feature B70, wherein the hydromorphone sustained release dosage form comprises hydromorphone in an amount ranging from about 1 mg to about 50 mg.
B72. The use according to Feature B65, wherein the hydromorphone sustained release dosage form further comprises an immediate release component for immediate release of the hydromorphone.
B73. The use according to Feature B65, wherein the hydromorphone sustained release dosage form further comprises an opioid antagonist.
B74. The use according to Feature B65, wherein releasing hydromorphone from the hydromorphone sustained release dosage form comprises sustainably releasing hydromorphone from the sustained release dosing structure.
B75. The use according to Feature B65, wherein the aqueous alcohol comprises alcohol at concentrations equal to or greater than about 30% volume/volume.
B76. The use according to Feature B75, wherein the aqueous alcohol comprises alcohol at concentrations equal to or greater than about 40% volume/volume.
B77. The use according to Feature B67, wherein the hydromorphone sustained release dosage form comprises a once-per-day hydromorphone sustained release dosage form.
B78. The use according to Feature B65, wherein the hydromorphone sustained release dosage form comprises a twice-per-day hydromorphone sustained release dosage form.
B79. The use of a once-per-day hydromorphone sustained release dosage form comprising hydromorphone and a sustained release dosing structure that provides once-per-day dosing in combination with aqueous alcohol, for reducing alcohol-induced dose dumping;
   wherein the aqueous alcohol comprises alcohol at concentrations equal to or greater than about 20% volume/volume; and
   wherein a ratio of (i) the median single dose, time to maximum plasma concentration achieved when the dosage form is used in combination with the aqueous alcohol to (ii) the median single dose, time to maximum plasma concentration achieved when the once-per-day hydromorphone sustained release dosage form is used alone, ranges from about 0.5 to about 1.0.
B80. The use of an hydromorphone sustained release dosage form comprising hydromorphone and a sustained release dosing structure in combination with aqueous alcohol, for reducing alcohol-induced dose dumping;
   wherein the aqueous alcohol comprises alcohol at concentrations equal to or greater than about 20% volume/volume; and
   wherein a ratio of (i) the median single dose, time to maximum plasma concentration achieved when the dosage form is used in combination with the aqueous alcohol to (ii) the median single dose, time to maximum plasma concentration achieved when the hydromorphone sustained release dosage form is used alone, ranges from about 0.5 to about 1.0.
B81. The use of a once-per-day hydromorphone sustained release dosage form in combination with aqueous alcohol, for reducing alcohol-induced dose dumping;
   wherein the once-per-day hydromorphone sustained release dosage form releases less than or equal to about 80 weight percent of the dose of hydromorphone from the once-per-day hydromorphone sustained release dosage form as measured (a) using an in vitro test method that comprises test media and (b) in a period of about 2 hours following initiation of the in vitro test method; and
   wherein the test media comprises aqueous alcohol that comprises alcohol at concentrations equal to or greater than about 20% volume/volume.
B82. The use according to Feature B81, wherein the sustained release dosage form comprises an osmotic oral sustained release dosing structure.
B83. The use according to Feature B81, wherein the alcohol comprises ethanol.
B84. The use according to Feature B81, wherein the once-per-day hydromorphone sustained release dosage form releases less than or equal to about 50 weight percent of the dose of hydromorphone from the once-per-day hydromorphone sustained release dosage form.
B85. The use according to Feature B84, wherein the once-per-day hydromorphone sustained release dosage form releases less than or equal to about 25 weight percent of the dose of hydromorphone from the once-per-day hydromorphone sustained release dosage form.
B86. The use according to Feature B81, wherein the once-per-day hydromorphone sustained release dosage form comprises hydromorphone in an amount ranging from about 1 mg to about 100 mg.
B87. The use according to Feature B86, wherein the once-per-day hydromorphone sustained release dosage form comprises hydromorphone in an amount ranging from about 1 mg to about 50 mg.
B88. The use according to Feature B81, wherein the once-per-day hydromorphone sustained release dosage form further comprises an immediate release component for immediate release of the hydromorphone.
B89. The use according to Feature B81, wherein the once-per-day hydromorphone sustained release dosage form further comprises an opioid antagonist.
B90. The use according to Feature B81, wherein releasing hydromorphone from the hydromorphone sustained release dosage form comprises sustainably releasing hydromorphone from the sustained release dosing structure that provides once-per-day dosing.
B91. The use according to Feature B81, wherein the aqueous alcohol comprises alcohol at concentrations equal to or greater than about 30% volume/volume.
B92. The use according to Feature B81, wherein the aqueous alcohol comprises alcohol at concentrations equal to or greater than about 40% volume/volume.
B93. The use of an hydromorphone sustained release dosage form in combination with aqueous alcohol, for reducing alcohol-induced dose dumping;
   wherein the hydromorphone sustained release dosage form releases less than about 80 weight percent of the dose of hydromorphone from the hydromorphone sustained release dosage form as measured (a) using an in vitro test method that comprises test media and (b) in a period of about 2 hours following initiation of the in vitro test method; and
   wherein the test media comprises aqueous alcohol that comprises alcohol at concentrations equal to or greater than about 20% volume/volume.
B94. The use according to Feature B93, wherein the sustained release dosage form comprises an osmotic oral sustained release dosing structure.
B95. The use according to Feature B93, wherein the alcohol comprises ethanol.
B96. The use according to Feature B93, wherein the hydromorphone sustained release dosage form releases less than or equal to about 50 weight percent of the dose of hydromorphone from the hydromorphone sustained release dosage form.
B97. The use according to Feature B96, wherein the hydromorphone sustained release dosage form releases less than or equal to about 25 weight percent of the dose of hydromorphone from the hydromorphone sustained release dosage form.
B98. The use according to Feature B93, wherein the hydromorphone sustained release dosage form comprises hydromorphone in an amount ranging from about 1 mg to about 100 mg.
B99. The use according to Feature B98, wherein the hydromorphone sustained release dosage form comprises hydromorphone in an amount ranging from about 1 mg to about 50 mg.
B 100. The use according to Feature B93, wherein the hydromorphone sustained release dosage form further comprises an immediate release component for immediate release of the hydromorphone.
B101. The use according to Feature B93, wherein the hydromorphone sustained release dosage form further comprises an opioid antagonist.
B102. The use according to Feature B93, wherein releasing hydromorphone from the hydromorphone sustained release dosage form comprises sustainably releasing hydromorphone from the sustained release dosing structure.
B103. The use according to Feature B93, wherein the aqueous alcohol comprises alcohol at concentrations equal to or greater than about 30% volume/volume.
B 104. The use according to Feature B 103, wherein the aqueous alcohol comprises alcohol at concentrations equal to or greater than about 40% volume/volume.
B105. The use according to Feature B93, wherein the hydromorphone sustained release dosage form comprises a once-per-day hydromorphone sustained release dosage form.
B106. The use according to Feature B93, wherein the hydromorphone sustained release dosage form comprises a twice-per-day hydromorphone sustained release dosage form.

Furthermore, the features of the invention include, without limitation, the following:
C1. The use of a once-per-day opioid sustained release dosage form comprising an opioid and a sustained release dosing structure that provides once-per-day dosing in combination with aqueous alcohol, for reducing alcohol-induced dose dumping;
   wherein the aqueous alcohol comprises alcohol at concentrations equal to or greater than about 20% volume/volume; and
   wherein a ratio of (i) a mean single dose maximum plasma opioid concentration achieved when the once-per-day opioid sustained release dosage form is used in combination with aqueous alcohol to (ii) a mean single dose maximum plasma opioid concentration achieved when the once-per-day opioid sustained release dosage form is used alone, is equal to or less than about 1.8:1.
C2. The use according to Feature C1, wherein the sustained release dosing structure comprises an osmotic oral sustained release dosing structure.
C3. The use according to Feature C1, wherein the alcohol comprises ethanol.
C4. The use according to Feature C1, wherein the ratio of (i) a mean single dose maximum plasma opioid concentration achieved when the once-per-day opioid sustained release dosage form is used in combination with aqueous alcohol to (ii) a mean single dose maximum plasma opioid concentration achieved when the once-per-day opioid sustained release dosage form is used alone, is equal to or less than about 1.6:1.
C5. The use according to Feature C4, wherein the ratio of (i) a mean single dose maximum plasma opioid concentration achieved when the once-per-day opioid sustained release dosage form is used in combination with aqueous alcohol to (ii) a mean single dose maximum plasma opioid concentration achieved when the once-per-day opioid sustained release dosage form is used alone, is equal to or less than about 1.4:1.
C6. The use according to Feature C1, wherein the once-per-day opioid sustained release dosage form comprises the opioid in an amount ranging from about 1 mg to about 100 mg.
C7. The use according to Feature C6, wherein the once-per-day opioid sustained release dosage form comprises the opioid in an amount ranging from about 1 mg to about 50 mg.
C8. The use according to Feature C1, wherein the once-per-day opioid sustained release dosage form further comprises an immediate release component for immediate release of the opioid.
C9. The use according to Feature C1, wherein the once-per-day opioid sustained release dosage form further comprises an opioid antagonist.
C10. The use according to Feature C1, wherein releasing the opioid from the opioid sustained release dosage form comprises sustainably releasing the opioid from the sustained release dosing structure that provides once-per-day dosing.
C11. The use according to Feature C1, wherein the aqueous alcohol comprises alcohol at concentrations equal to or greater than about 30% volume/volume.
C12. The use according to Feature C11, wherein the aqueous alcohol comprises alcohol at concentrations equal to or greater than about 40% volume/volume.
C13. The use of a once-per-day opioid sustained release dosage form comprising an opioid and a sustained release dosing structure, that provides once-per-day dosing, in combination with aqueous alcohol, for reducing alcohol-induced dose dumping;
   wherein the aqueous alcohol comprises alcohol at concentrations equal to or greater than about 20% volume/volume; and
   wherein a ratio of (i) an individual patient single dose maximum plasma opioid concentration achieved when the once-per-day opioid sustained release dosage form is used in combination with the aqueous alcohol to (ii) an individual patient single dose maximum plasma opioid concentration achieved when the once-per-day opioid sustained release dosage form is used alone, is equal to or less than about 5:1.
C14. The use according to Feature C13, wherein the sustained release dosing structure comprises an osmotic oral sustained release dosing structure.
C15. The use according to Feature C13, wherein the alcohol comprises ethanol.
C16. The use according to Feature C13, wherein the ratio of (i) an individual patient single dose maximum plasma opioid concentration achieved when the once-per-day opioid sustained release dosage form is used in combination with the aqueous alcohol to (ii) an individual patient single dose maximum plasma opioid concentration achieved when the once-per-day opioid sustained release dosage form is used alone, is equal to or less than about 4:1.
C17. The use according to Feature C16, wherein the ratio of (i) an individual patient single dose maximum plasma opioid concentration achieved when the once-per-day opioid sustained release dosage form is used in combination with the aqueous alcohol to (ii) an individual patient single dose maximum plasma opioid concentration achieved when the once-per-day opioid sustained release dosage form is used alone, is equal to or less than about 3:1.
C18. The use according to Feature C13, wherein the once-per-day opioid sustained release dosage form comprises the opioid in an amount ranging from about 1 mg to about 100 mg.
C19. The use according to Feature C18, wherein the once-per-day opioid sustained release dosage form comprises the opioid in an amount ranging from about 1 mg to about 50 mg.
C20. The use according to Feature C13, wherein the once-per-day opioid sustained release dosage form further comprises an immediate release component for immediate release of the opioid.
C21. The use according to Feature C13, wherein the once-per-day opioid sustained release dosage form further comprises an opioid antagonist.
C22. The use according to Feature C13, wherein releasing the opioid from the opioid sustained release dosage form comprises sustainably releasing the opioid from the sustained release dosing structure that provides once-per-day dosing.
C23. The use according to Feature C13, wherein the aqueous alcohol comprises alcohol at concentrations equal to or greater than about 30% volume/volume.
C24. The use according to Feature C23, wherein the aqueous alcohol comprises alcohol at concentrations equal to or greater than about 40% volume/volume.
C25. The use of a once-per-day opioid sustained release dosage form comprising an opioid and a sustained release dosing structure that provides once-per-day dosing in combination with aqueous alcohol, for reducing alcohol-induced dose dumping;
   wherein the once-per-day opioid sustained release dosage form releases less than or equal to about 80 weight percent of the dose of the opioid from the once-per-day opioid sustained release dosage form as measured (a) using an in vitro test method that comprises test media and (b) in a period of about 2 hours following initiation of the in vitro test method; and
   wherein the test media comprises aqueous alcohol that comprises alcohol at concentrations equal to or greater than about 20% volume/volume.
C26. The use according to Feature C25, wherein the sustained release dosing structure comprises an osmotic oral sustained release dosing structure.
C27. The use according to Feature C25, wherein the alcohol comprises ethanol.
C28. The use according to Feature C25, wherein the once-per-day opioid sustained release dosage form releases less than or equal to about 50 weight percent of the dose of the opioid from the once-per-day opioid sustained release dosage form.
C29. The use according to Feature C28, wherein the once-per-day opioid sustained release dosage form releases less than or equal to about 25 weight percent of the dose of the opioid from the once-per-day opioid sustained release dosage form.
C30. The use according to Feature C25, wherein the once-per-day opioid sustained release dosage form comprises the opioid in an amount ranging from about 1 mg to about 100 mg.
C31. The use according to Feature C30, wherein the once-per-day opioid sustained release dosage form comprises the opioid in an amount ranging from about 1 mg to about 50 mg.
C32. The use according to Feature C25, wherein the once-per-day opioid sustained release dosage form further comprises an immediate release component for immediate release of the opioid.
C33. The use according to Feature C25, wherein the once-per-day opioid sustained release dosage form further comprises an opioid antagonist.
C34. The use according to Feature C25, wherein releasing the opioid from the opioid sustained release dosage form comprises sustainably releasing the opioid from the sustained release dosing structure that provides once-per-day dosing.
C35. The use according to Feature C25, wherein the aqueous alcohol comprises alcohol at concentrations equal to or greater than about 30% volume/volume.
C36. The use according to Feature C25, wherein the aqueous alcohol comprises alcohol at concentrations equal to or greater than about 40% volume/volume.
C37. The use of an opioid sustained release dosage form comprising an opioid and a sustained release dosing structure in combination with aqueous alcohol, for reducing alcohol-induced dose dumping;
   wherein the aqueous alcohol comprises alcohol at concentrations equal to or greater than about 20% volume/volume; and
   wherein a ratio of (i) a mean single dose maximum plasma opioid concentration achieved when the dosage form is used in combination with the aqueous alcohol to (ii) a mean single dose maximum plasma opioid concentration achieved when the dosage form is used alone, is equal to or less than about 1.8:1.
C38. The use according to Feature C37, wherein the sustained release dosing structure comprises an osmotic oral sustained release dosing structure.
C39. The use according to Feature C37, wherein the alcohol comprises ethanol.
C40. The use according to Feature C37, wherein the ratio of (i) a mean single dose maximum plasma opioid concentration achieved when the opioid sustained release dosage form is used in combination with the aqueous alcohol to (ii) a mean single dose maximum plasma opioid concentration achieved when the opioid sustained release dosage form is used alone, is equal to or less than about 1.6:1.
C41. The use according to Feature C40, wherein the ratio of (i) a mean single dose maximum plasma opioid concentration achieved when the opioid sustained release dosage form is used in combination with aqueous alcohol to (ii) a mean single dose maximum plasma opioid concentration achieved when the opioid sustained release dosage form is used alone, is equal to or less than about 1.4:1.
C42. The use according to Feature C37, wherein the opioid sustained release dosage form comprises the opioid in an amount ranging from about 1 mg to about 100 mg.
C43. The use according to Feature C42, wherein the opioid sustained release dosage form comprises the opioid in an amount ranging from about 1 mg to about 50 mg.
C44. The use according to Feature C37, wherein the opioid sustained release dosage form comprises a once-per-day opioid sustained release dosage form.
C45. The use according to Feature C37, wherein the opioid sustained release dosage form comprises a twice-per-day opioid sustained release dosage form.
C46. The use according to Feature C37, wherein the opioid sustained release dosage form further comprises an immediate release component for immediate release of the opioid.
C47. The use according to Feature C37, wherein the opioid sustained release dosage form further comprises an opioid antagonist.
C48. The use according to Feature C37, wherein releasing the opioid from the opioid sustained release dosage form comprises sustainably releasing the opioid from the sustained release dosing structure.
C49. The use according to Feature C37, wherein the aqueous alcohol comprises alcohol at concentrations equal to or greater than about 30% volume/volume.
C50. The use according to Feature C49, wherein the aqueous alcohol comprises alcohol at concentrations equal to or greater than about 40% volume/volume.
C51. The use of an opioid sustained release dosage form comprising an opioid and a sustained release dosing structure in combination with aqueous alcohol, for reducing alcohol-induced dose dumping;
   wherein the aqueous alcohol comprises alcohol at concentrations equal to or greater than about 20% volume/volume; and
   wherein a ratio of (i) an individual patient single dose maximum plasma opioid concentration achieved when the dosage form is used in combination with the aqueous alcohol to (ii) an individual patient single dose maximum plasma opioid concentration achieved when the dosage form is used alone, is equal to or less than about 5:1.
C52. The use according to Feature C51, wherein the sustained release dosing structure comprises an osmotic oral sustained release dosing structure.
C53. The use according to Feature C51, wherein the alcohol comprises ethanol.
C54. The use according to Feature C51, wherein the ratio of (i) an individual patient single dose maximum plasma opioid concentration achieved when the opioid sustained release dosage form is used in combination with the aqueous alcohol to (ii) an individual patient single dose maximum plasma opioid concentration achieved when the opioid sustained release dosage form is used alone, is equal to or less than about 4:1.
C55. The use according to Feature C54, wherein the ratio of (i) an individual patient single dose maximum plasma opioid concentration achieved when the opioid sustained release dosage form is used in combination with the aqueous alcohol to (ii) an individual patient single dose maximum plasma opioid concentration achieved when the opioid sustained release dosage form is used alone, is equal to or less than about 3:1.
C56. The use according to Feature C51, wherein the opioid sustained release dosage form comprises the opioid in an amount ranging from about 1 mg to about 100 mg.
C57. The use according to Feature C56, wherein the opioid sustained release dosage form comprises the opioid in an amount ranging from about 1 mg to about 50 mg.
C58. The use according to Feature C51, wherein the opioid sustained release dosage form further comprises an immediate release component for immediate release of the opioid.
C59. The use according to Feature C51, wherein the opioid sustained release dosage form further comprises an opioid antagonist.
C60. The use according to Feature C51, wherein releasing the opioid from the opioid sustained release dosage form comprises sustainably releasing the opioid from the sustained release dosing structure.
C61. The use according to Feature C51, wherein the aqueous alcohol comprises alcohol at concentrations equal to or greater than about 30% volume/volume.
C62. The use according to Feature C61, wherein the aqueous alcohol comprises alcohol at concentrations equal to or greater than about 40% volume/volume.
C63. The use according to Feature C51, wherein the opioid sustained release dosage form comprises a once-per-day opioid sustained release dosage form.
C64. The use according to Feature C65, wherein the opioid sustained release dosage form comprises a twice-per-day opioid sustained release dosage form.
C65. The use of an opioid sustained release dosage form comprising an opioid and a sustained release dosing structure in combination with aqueous alcohol, for reducing alcohol-induced dose dumping;
   wherein the opioid sustained release dosage form releases less than about 80 weight percent of the dose of the opioid from the opioid sustained release dosage form as measured (a) using an in vitro test method that comprises test media and (b) in a period of about 2 hours following initiation of the in vitro test method; and
   wherein the test media comprises aqueous alcohol that comprises alcohol at concentrations equal to or greater than about 20% volume/volume.
C66. The use according to Feature C65, wherein the sustained release dosing structure comprises an osmotic oral sustained release dosing structure.
C67. The use according to Feature C65, wherein the alcohol comprises ethanol.
C68. The use according to Feature C67, wherein the opioid sustained release dosage form releases less than or equal to about 50 weight percent of the dose of the opioid from the opioid sustained release dosage form.
C69. The use according to Feature C68, wherein the opioid sustained release dosage form releases less than or equal to about 25 weight percent of the dose of the opioid from the opioid sustained release dosage form.
C70. The use according to Feature C65, wherein the opioid sustained release dosage form comprises the opioid in an amount ranging from about 1 mg to about 100 mg.
C71. The use according to Feature C70, wherein the opioid sustained release dosage form comprises the opioid in an amount ranging from about 1 mg to about 50 mg.
C72. The use according to Feature C65, wherein the opioid sustained release dosage form further comprises an immediate release component for immediate release of the opioid.
C73. The use according to Feature C65, wherein the opioid sustained release dosage form further comprises an opioid antagonist.
C74. The use according to Feature C65, wherein releasing the opioid from the opioid sustained release dosage form comprises sustainably releasing the opioid from the sustained release dosing structure.
C75. The use according to Feature C65, wherein the aqueous alcohol comprises alcohol at concentrations equal to or greater than about 30% volume/volume.
C76. The use according to Feature C75, wherein the aqueous alcohol comprises alcohol at concentrations equal to or greater than about 40% volume/volume.
C77. The use according to Feature C67, wherein the opioid sustained release dosage form comprises a once-per-day opioid sustained release dosage form.
C78. The use according to Feature C65, wherein the opioid sustained release dosage form comprises a twice-per-day opioid sustained release dosage form.
C79. The use of a once-per-day opioid sustained release dosage form comprising an opioid and a sustained release dosing structure that provides once-per-day dosing in combination with aqueous alcohol, for reducing alcohol-induced dose dumping;
   wherein the aqueous alcohol comprises alcohol at concentrations equal to or greater than about 20% volume/volume; and
   wherein a ratio of (i) the median single dose, time to maximum plasma concentration achieved when the dosage form is used in combination with the aqueous alcohol to (ii) the median single dose, time to maximum plasma concentration achieved when the once-per-day opioid sustained release dosage form is used alone, ranges from about 0.5 to about 1.0.
C80. The use of an opioid sustained release dosage form comprising an opioid and a sustained release dosing structure in combination with aqueous alcohol, for reducing alcohol-induced dose dumping;
   wherein the aqueous alcohol comprises alcohol at concentrations equal to or greater than about 20% volume/volume; and
   wherein a ratio of (i) the median single dose, time to maximum plasma concentration achieved when the dosage form is used in combination with the aqueous alcohol to (ii) the median single dose, time to maximum plasma concentration achieved when the opioid sustained release dosage form is used alone, ranges from about 0.5 to about 1.0.
C81. The use of a once-per-day opioid sustained release dosage form in combination with aqueous alcohol, for reducing alcohol-induced dose dumping;
   wherein the once-per-day opioid sustained release dosage form releases less than or equal to about 80 weight percent of the dose of the opioid from the once-per-day opioid sustained release dosage form as measured (a) using an in vitro test method that comprises test media and (b) in a period of about 2 hours following initiation of the in vitro test method; and
   wherein the test media comprises aqueous alcohol that comprises alcohol at concentrations equal to or greater than about 20% volume/volume.
C82. The use according to Feature C81, wherein the sustained release dosage form comprises an osmotic oral sustained release dosing structure.
C83. The use according to Feature C81, wherein the alcohol comprises ethanol.
C84. The use according to Feature C81, wherein the once-per-day opioid sustained release dosage form releases less than or equal to about 50 weight percent of the dose of the opioid from the once-per-day opioid sustained release dosage form.
C85. The use according to Feature C84, wherein the once-per-day opioid sustained release dosage form releases less than or equal to about 25 weight percent of the dose of the opioid from the once-per-day opioid sustained release dosage form.
C86. The use according to Feature C81, wherein the once-per-day opioid sustained release dosage form comprises the opioid in an amount ranging from about 1 mg to about 100 mg.
C87. The use according to Feature C86, wherein the once-per-day opioid sustained release dosage form comprises the opioid in an amount ranging from about 1 mg to about 50 mg.
C88. The use according to Feature C81, wherein the once-per-day opioid sustained release dosage form further comprises an immediate release component for immediate release of the opioid.
C89. The use according to Feature C81, wherein the once-per-day opioid sustained release dosage form further comprises an opioid antagonist.
C90. The use according to Feature C81, wherein releasing the opioid from the opioid sustained release dosage form comprises sustainably releasing the opioid from the sustained release dosing structure that provides once-per-day dosing.
C91. The use according to Feature C81, wherein the aqueous alcohol comprises alcohol at concentrations equal to or greater than about 30% volume/volume.
C92. The use according to Feature C81, wherein the aqueous alcohol comprises alcohol at concentrations equal to or greater than about 40% volume/volume.
C93. The use of an opioid sustained release dosage form in combination with aqueous alcohol, for reducing alcohol-induced dose dumping;
   wherein the opioid sustained release dosage form releases less than about 80 weight percent of the dose of the opioid from the opioid sustained release dosage form as measured (a) using an in vitro test method that comprises test media and (b) in a period of about 2 hours following initiation of the in vitro test method; and
   wherein the test media comprises aqueous alcohol that comprises alcohol at concentrations equal to or greater than about 20% volume/volume.
C94. The use according to Feature C93, wherein the sustained release dosage form comprises an osmotic oral sustained release dosing structure.
C95. The use according to Feature C93, wherein the alcohol comprises ethanol.
C96. The use according to Feature C93, wherein the opioid sustained release dosage form releases less than or equal to about 50 weight percent of the dose of the opioid from the opioid sustained release dosage form.
C97. The use according to Feature C96, wherein the opioid sustained release dosage form releases less than or equal to about 25 weight percent of the dose of the opioid from the opioid sustained release dosage form.
C98. The use according to Feature C93, wherein the opioid sustained release dosage form comprises the opioid in an amount ranging from about 1 mg to about 100 mg.
C99. The use according to Feature C98, wherein the opioid sustained release dosage form comprises the opioid in an amount ranging from about 1 mg to about 50 mg.
C100. The use according to Feature C93, wherein the opioid sustained release dosage form further comprises an immediate release component for immediate release of the opioid.
C101. The use according to Feature C93, wherein the opioid sustained release dosage form further comprises an opioid antagonist.
C102. The use according to Feature C93, wherein releasing the opioid from the opioid sustained release dosage form comprises sustainably releasing the opioid from the sustained release dosing structure.
C103. The use according to Feature C93, wherein the aqueous alcohol comprises alcohol at concentrations equal to or greater than about 30% volume/volume.
C104. The use according to Feature C 103, wherein the aqueous alcohol comprises alcohol at concentrations equal to or greater than about 40% volume/volume.
C105. The use according to Feature C93, wherein the opioid sustained release dosage form comprises a once-per-day opioid sustained release dosage form.
C106. The use according to Feature C93, wherein the opioid sustained release dosage form comprises a twice-per-day opioid sustained release dosage form.

Additionally, the features of the invention include, without limitation, the following:
D1. A sustained release oral dosage form comprising an opioid and a sustained release dosing structure wherein said sustained release dosing structure releases said opioid from said sustained release dosage form in the presence of aqueous alcohol comprising alcohol at concentrations equal to or greater than about 20% volume/volume with a ratio of a mean single dose maximum plasma opioid concentration achieved when said opioid sustained release dosage form is co-administered in a patient with said aqueous alcohol to a mean single dose maximum plasma opioid concentration achieved when said opioid sustained release dosage form is administered to a patient without co-administration of said aqueous alcohol is equal to or less than about 1.8:1.
D2. The sustained release oral dosage form according to Feature D1, wherein said ratio is equal to or less than about 1.6:1, preferably equal to or less than about 1.4:1.
D3. A sustained release oral dosage form comprising an opioid and a sustained release dosing structure wherein said sustained release dosing structure releases said opioid from said sustained release dosage form in the presence of aqueous alcohol comprising alcohol at concentrations equal to or greater than about 20% volume/volume with a ratio of an individual patient single dose maximum plasma opioid concentration achieved when said opioid sustained release dosage form is co-administered in the patient with the aqueous alcohol to an individual patient single dose maximum plasma opioid concentration achieved when said opioid sustained release dosage form is administered to a patient without coadministration of the aqueous alcohol is equal to or less than about 5:1.
D4. The sustained release oral dosage form according to Feature D3, wherein said ratio is equal to or less than about 4:1, preferably equal to or less than about 3:1.
D5. A sustained release oral dosage form comprising an opioid and a sustained release dosing structure wherein said sustained release dosing structure releases said opioid from said sustained release dosage form in the presence of aqueous alcohol comprising alcohol at concentrations equal to or greater than about 20% volume/volume with said opioid sustained release dosage form releasing less than or equal to about 80 weight percent of the dose of opioid from said opioid sustained release dosage form as measured (a) using an in vitro test method that comprises test media and (b) in a period of about 2 hours following initiation of the in vitro test method; and wherein said test media comprises aqueous alcohol at concentrations equal to or greater than about 20% volume/volume.
D6. The sustained release oral dosage form according to Feature D5, wherein said release is less than or equal to about 50 weight percent of the dose of opioid, preferably less than or equal to about 25 weight percent of the dose of opioid.
D7. A sustained release oral dosage form comprising an opioid and a sustained release dosing structure wherein said sustained release dosing structure releases said opioid from said sustained release dosage form in the presence of aqueous alcohol comprising alcohol at concentrations equal to or greater than about 20% volume/volume with a ratio of the median single dose time to maximum plasma concentration achieved when said sustained release dosage form is co-administered in the patient with the aqueous alcohol to the median single dose time to maximum plasma concentration achieved when said sustained release dosage form is administered in a patient without co-administration of the aqueous alcohol ranges from about 0.5 to about 1.0.
D8. The sustained release oral dosage form according to any of Features D1 to D7, which provides once-per-day or twice-per-day dosing.
D9. The sustained release oral dosage form according to any of Features D1 to D8, wherein said opioid is selected from morphine, codeine, thebaine, diamorphine, oxycodone, hydrocodone, dihydrocodeine, hydromorphone, oxymorphone, nicomorphine, methadone, levomethadyl acetate hydrochloride, pethidine, ketobemidone, propoxyphene, dextropropoxyphene, dextromoramide, bezitramide, piritramide, pentazocine, phenazocine and pharmaceutically acceptable salts thereof.
D10. The sustained release oral dosage form according to any of Features D1 to D9, wherein said aqueous alcohol is equal to or greater than about 25% volume/volume, more preferably equal to or greater than about 30% volume/volume, still more preferably equal to or greater than about 35% volume/volume, and most preferably equal to or greater than about 40% volume/volume.
D11. The sustained release oral dosage form according to any of Features D1 to D10, further comprising an opioid antagonist, such as naltrexone levallorphan, naloxone, naltrexone, buprenorphine, nalbuphine, nalorphine, nalmefene diprenorphine, cyclazocine, etazocine, metazocine or naloxone.
D12. The sustained release oral dosage form according to any of Features D1 to D11, wherein said oral dosage form comprises said opioid in an amount in the range from about 0.001 mg to about 5000 mg, preferably from about 0.01 to about 1000 mg, more preferably from about 0.1 to about 750 mg, still preferably from about 0.5 to about 500 mg, even more preferably from about 0.5 to about 250 mg, even more preferably from about 1 to about 100 mg, and most preferably from about 1 to about 50 mg.
D13. The sustained release oral dosage form according to any of Features D1 to D12, wherein said oral dosage form is selected from a diffusion system, a dissolution system, a combination diffusion/dissolution system, an ion-exchange resin system, an osmotic system, a gastric retention dosage form, sustained microsphere formulations, and an osmotic sustained release dosage form.
D14. The sustained release oral dosage form according to Feature D13, wherein said diffusion system is selected from a reservoir device or a matrix device.
D 15. The sustained release oral dosage form according to Feature D13, wherein said dissolution system is selected from an encapsulated dissolution system, such as tiny time pills, beads, and a matrix dissolution system.
D16. The sustained release oral dosage form according to Feature D13, wherein said osmotic dosage form comprises a compartment formed, at least in part, by a semipermeable membrane.
D17. The sustained release oral dosage form according to Feature D16, wherein said semi-permeable membrane is coated with a film of polyvinyl alcohol.
D18. The sustained release oral dosage form according to Feature D16 or D 17, comprising a drug composition in form of a slurry, suspension or solution, a small exit orifice and an expandable layer.
D19. The sustained release oral dosage form according to Feature D18, wherein said drug layer is provided with a subcoat or an annealed coating in conjunction with the semipermeable membrane.
D20. The sustained release oral dosage form according to any of Features D16 to D19, wherein said osmotic sustained release dosage form comprises an enteric coating or a non-enteric coating.
D21. The sustained release oral dosage form according to Feature D20, wherein said enteric coating comprises a material selected from CAP, HMPCP, and PVAP.
D22. The sustained release oral dosage form according to Feature D13 in the form of an elementary osmotic pump dosage form, comprising a semi-permeable membrane surrounding and enclosing and internal compartment containing a drug layer, comprising said drug in admixture with one or more excipient adapted to provide an osmotic activity gradient and for forming a deliverable complex formulation upon imbibition of fluid.
D23. The sustained release oral dosage form according to Feature D22, wherein said excipient includes a suitable drug carrier, a binding agent, a lubricant, and an osmagent.
D24. The sustained release oral dosage form according to Feature D22 or D23, wherein said semi-permeable membrane comprises a polymer selected from homopolymers and copolymers, such as, cellulose esters, cellulose ethers, and cellulose ester-ethers.
D25. The sustained release oral dosage form according to any of Features D22 to D24, further comprising a flux-regulating agent, in particular selected from polyhydric alcohols, polyalkylene glycols, polyalkylenediols, polyesters of alkylene glycols, and the like.
D26. The sustained release oral dosage form according to Feature D13 in the form of an osmotic sustained release dosage form comprising a first drug layer comprising osmotically active components, and a second drug layer comprising more drug than the first drug layer, and, optionally, an expandable layer.
D27. The sustained release oral dosage form according to Feature D26, wherein the osmotically active components are selected from an osmagent such as a salt, and one or more osmopolymer(s) having relatively small molecular weights which exhibit swelling as fluid is imbibed.
D28. The sustained release oral dosage form according to Feature D26 or D27, wherein the first drug layer further includes excipients, such as binders, lubricants, antioxidants and colorants.
D29. The sustained release oral dosage form according to any of Features D26 to D28, wherein said second drug layer comprises the opioid in admixture with selected excipients adapted to provide an osmotic activity gradient, such as a suitable drug carrier.
D30. The sustained release oral dosage form according to Feature D29, wherein the second layer is free from osmotically active agents.
D31. The sustained release oral dosage form according to any of Features D26 to D30, further comprising an exit orifice.
D32. The sustained release oral dosage form according to any of Features D26 to D31, wherein the first and second drug layer further comprises a hydrophilic polymer carrier, in particular a carrier that erodes in the gastric environment.
D33. The sustained release oral dosage form according to any of Features D26 to D32, further comprising a semipermeable membrane, in particular comprising cellulose esters, cellulose ethers, and cellulose ester-ethers.
D34. The sustained release oral dosage form according to any of Features D26 to D33, further comprising a flux-regulating agent, in particular a flux enhancer or a flux decreasing agent selected from polyhydric alcohols, polyalkylene glycols, polyalkylenediols, polyesters of alkylene glycols, and the like.
D35. The sustained release oral dosage form according to any of Features D26 to D34, wherein the expandable layer comprises a hydroactive layer comprising osmopolymers or osmagents.
D36. The sustained release oral dosage form according to Feature D13, in the form of a soft capsule or a hard capsule.
D37. The sustained release oral dosage form according to Feature D36, in the form of a one-piece soft capsule of a sealed construction encapsulating the drug formulation therein.
D38. The sustained release oral dosage form according to Feature D36 or D37, wherein said soft capsule is surrounded by an unsymmetrical hydro-activated layer as an expandable layer and an exit orifice.
D39. The sustained release oral dosage form according to any of Features D36 to D38, wherein said soft capsule further comprises a barrier layer.
D40. The sustained release oral dosage form according to any of Features D36 to D39, wherein the expandable layer is formed in discrete sections that do not entirely encompass the barrier layer-coated capsule.
D41. The sustained release oral dosage form according to Feature D36, in the form of a two-piece hard capsule composed of two parts, a cap and a body.
D42. The sustained release oral dosage form according to Feature D41, wherein said capsule is encapsulated with a semipermeable lamina.
D43. The sustained release oral dosage form according to Feature D41 or D42, wherein said hard capsule is made with each part having matched locking rings near their opened end that permit joining and locking together the overlapping cap and body after filling with formulation.
D44. The sustained release oral dosage form according to any of Features D36 to D43, wherein said capsule further comprises a semipermeable membrane.
D45. The sustained release oral dosage form according to Feature D44, wherein said semipermeable membrane comprises a flux regulating agent.
D46. The sustained release oral dosage form according to Feature D43 to D45, wherein said semipermeable membrane surrounds and forms a compartment containing a one or a plurality of layers, one of which is an expandable layer, preferably containing an osmotic agent.
D47. The sustained release oral dosage form according to any of Features D36 to D46, further comprising a barrier layer, preferably formulated with plasticizers.
D48. The sustained release oral dosage form according to Feature D13 in the form of a cylindrically shaped matrix comprising the opioid, with ends of the matrix that are rounded and convex in shape, and bands that concentrically surround the cylindrical matrix and are formed of a material that is relatively insoluble in an aqueous environment.
D49. The sustained release oral dosage form according to Feature D13 in the form of a gastric retention dosage form comprising a tablet or capsule comprising a plurality of particles of a dispersion of a limited solubility drug in a hydrophilic, water-swellable, crosslinked polymer that maintains its physical integrity over the dosing lifetime but thereafter rapidly dissolves.
D50. The sustained release oral dosage form according to Feature D13 in the form of a matrix dosage form containing a gelling component, a hydrophobic excipient, a drug, and a diluent.
D51. The sustained release oral dosage form according to Feature D13 in the form of osmotic beads comprising nonpareil seeds or other substrates having sufficient osmotic activity, coated with a semi-permeable film having a wide distribution of coating thicknesses or a film not semi-permeable to water.
D52. The sustained release oral dosage form according to Feature D51 1 wherein said osmotic beads are present in a capsule.
D53. The sustained release oral dosage form according to any of Features D1 to D52 for use in medicine.
D54. Use of a sustained release oral dosage form comprising an opioid and a sustained release dosing structure that provides sustained release dosing; for the production of a medicament for the treatment of pain when
   said sustained release opioid oral dosage form is co-administered with aqueous alcohol in a patient; wherein the aqueous alcohol comprises alcohol at concentrations equal to or greater than about 20% volume/volume; and
   releasing opioid from said sustained release opioid oral dosage form;
   wherein a ratio of a mean single dose maximum plasma opioid concentration achieved when said sustained release opioid oral dosage form is co-administered to the patient with the aqueous alcohol to a mean single dose maximum plasma opioid concentration achieved when said sustained release opioid oral dosage form is administered to a patient without co-administration of the aqueous alcohol is equal to or less than about 1.8:1.
D55. Use of a sustained release oral dosage form according to Feature D54, wherein said ratio is equal to or less than about 1.6:1, preferably equal to or less than about 1.4:1.
D56. Use of a sustained release oral dosage form comprising an opioid and a sustained release dosing structure that provides sustained release dosing; for the production of a medicament for the treatment of pain when
   said sustained release opioid oral dosage form is co-administered with aqueous alcohol in a patient; wherein the aqueous alcohol comprises alcohol at concentrations equal to or greater than about 20% volume/volume; and
   releasing opioid from said sustained release opioid oral dosage form;
   wherein a ratio of an individual patient single dose maximum plasma opioid concentration achieved when said opioid sustained release dosage form is coadministered to the patient with the aqueous alcohol to an individual patient single dose maximum plasma opioid concentration achieved when said opioid sustained release dosage form is administered to a patient without coadministration of the aqueous alcohol is equal to or less than about 5:1.
D57. Use of a sustained release oral dosage form according to Feature D56, wherein said ratio is equal to or less than about 4:1, preferably equal to or less than about 3:1.
D58. Use of a sustained release oral dosage form comprising an opioid and a sustained release dosing structure that provides sustained release dosing; for the production of a medicament for the treatment of pain when
   said sustained release opioid oral dosage form is co-administered with aqueous alcohol in a patient; wherein the aqueous alcohol comprises alcohol at concentrations equal to or greater than about 20% volume/volume; and
   releasing opioid from said sustained release opioid oral dosage form;
   with said opioid sustained release dosage form releasing less than or equal to about 80 weight percent of the dose of opioid from said opioid sustained release dosage form as measured (a) using an in vitro test method that comprises test media and (b) in a period of about 2 hours following initiation of the in vitro test method; and wherein said test media comprises aqueous alcohol at concentrations equal to or greater than about 20% volume/volume.
D59. The sustained release oral dosage form according to Feature D58, wherein said release is less than or equal to about 50 weight percent of the dose of opioid, preferably less than or equal to about 25 weight percent of the dose of opioid.
D60. Use of a sustained release oral dosage form comprising an opioid and a sustained release dosing structure that provides sustained release dosing; for the production of a medicament for the treatment of pain when
   said sustained release opioid oral dosage form is co-administered with aqueous alcohol in a patient; wherein the aqueous alcohol comprises alcohol at concentrations equal to or greater than about 20% volume/volume; and
   releasing opioid from said sustained release opioid oral dosage form;
   with a ratio of the median single dose time to maximum plasma concentration achieved when said sustained release dosage form is co-administered in the patient with the aqueous alcohol to the median single dose time to maximum plasma concentration achieved when said sustained release dosage form is administered in a patient without co-administration of the aqueous alcohol ranges from about 0.5 to about 1.0.
D61. Use of a sustained release oral dosage form according to any of Features D54 to D60, which provides once-per-day or twice-per-day dosing.
D62. Use of a sustained release oral dosage form according to any of Features D54 to D61, wherein said sustained release dosing structure comprises an osmotic oral sustained release dosing structure.
D63. Use according to any of Features D54 to D62, wherein said alcohol comprises ethanol.
D64. Use according to any of Features D54 to D63, wherein said aqueous alcohol is equal to or greater than about 25% volume/volume, more preferably equal to or greater than about 30% volume/volume, still more preferably equal to or greater than about 35% volume/volume, and most preferably equal to or greater than about 40% volume/volume.
D65. Use according to any of Features D54 to D64, wherein said oral dosage form comprises said opioid in an amount in the range from about 0.001 mg to about 5000 mg, preferably from about 0.01 to about 1000 mg, more preferably from about 0.1 to about 750 mg, still preferably from about 0.5 to about 500 mg, even more preferably from about 0.5 to about 250 mg, even more preferably from about 1 to about 100 mg, and most preferably from about 1 to about 50 mg.
D66. Use according to any of Features D54 to D65, wherein said sustained release opioid oral dosage form further comprises an immediate release component for immediate release of the opioid.
D67. Use according to any of Features D54 to D66, wherein said opioid is selected from morphine, codeine, thebaine, diamorphine, oxycodone, hydrocodone, dihydrocodeine, hydromorphone, oxymorphone, nicomorphine, methadone, levomethadyl acetate hydrochloride, pethidine, ketobemidone, propoxyphene, dextropropoxyphene, dextromoramide, bezitramide, piritramide, pentazocine, phenazocine and pharmaceutically acceptable salts thereof.
D68. Use according to any of Features D54 to D67, wherein said sustained release opioid oral dosage form further comprises an opioid antagonist, such as naltrexone levallorphan, naloxone, naltrexone, buprenorphine, nalbuphine, nalorphine, nalmefene diprenorphine, cyclazocine, etazocine, metazocine or naloxone.
D69. Use according to any of Features D54 to D68, wherein said coadministering is performed simultaneously or separately from said aqueous alcohol.
D70. Use according to any of Features D54 to D69, wherein said oral dosage form is selected from a diffusion system, a dissolution system, a combination diffusion/dissolution system, an ion-exchange resin system, an osmotic system, a gastric retention dosage form, sustained microsphere formulations, and an osmotic sustained release dosage form.
D71. The sustained release oral dosage form according to any one of Features D1 to D53 or the use according to any one of Features D54 to D70 wherein said opioid is selected from the group consisting of hydromorphone hydrochloride, oxycodone hydrochloride, morphine sulphate, oxymorphone hydrochloride and hydrocodone bitartrate.

**Table 1**

| Summary of in vitro Release Attributes in Ethanol Solutions for Hydromorphone 16 mg Tablets (of Example 1) | | | | |
|---|---|---|---|---|
| Hydromorphone | Ethanol/Water Solution Composition (% v/v) | | | |
| | 0% (control) | 4% | 20% | 40% |
| T90 (hr) | 18 | 18 | 15 | 12 |
| Cumulative % Released at 2 hours (% label claim) | < 1 | < 1 | < 1 | 4 |
| Average Release Rate (% label claim/hr) | 6 | 6 | 7 | 10 |
| Average Release Rate Relative to 0% Ethanol (%) | Reference | 100 | 116 | 160 |

**Table 2**

| Summary of in vitro Release Attributes in Ethanol-based Solutions for Palladone XL 32 mg Capsules | | |
|---|---|---|
| Palladone XL | Ethanol/Water Solution Compositions (v/v) | |
| | 0% (control) | 27% |
| T90 (hr) | >24 | 1 |
| Cumulative % Released at 2 hours (% label claim) | 21 | 100 |

**Table 3**

| Mean (SD) Hydromorphone Pharmacokinetic Parameters - Group 1 Fasting | | | | |
|---|---|---|---|---|
| | 0% Alcohol | 4% Alcohol | 20% Alcohol | 40% Alcohol |
| Cmax (ng/mL) | 1.37(0.32) | 1.56 (0.39) | 1.90 (0.66) | 1.89(0.85) |
| Tmax (h) [Median (Range)] | 16 (6-27) | 12 (6-27) | 12 (4-16) | 12 (6-24) |
| T1/2 (h) | 12.4(5.1) | 12.6 (6.5) | 12.4 (7.2) | 11.1 (3.0) |
| AUCinf | 40.6(11.0) | 39.9 (14.1) | 43.7 (12.1) | 42.2 (13.2) |

| Ratio- Arithmetic Mean (SD:Range) | | | | |
|---|---|---|---|---|
| Cmax | Ref | 1.19 (0.23:0.8-1.7) | 1.35 (0.42:0.7-2.4) | 1.37 (0.53:0.7-2.5) |

| Ratio -% Geometric Mean (90% Cl) | | | | |
|---|---|---|---|---|
| Cmax | Ref | 116.70 | 131.16 | 128.31 |
| | | (104.48- 130.36) | (117.01-147.02) | (114.18-144.17) |
| AUCinf | Ref | 96.83 | 103.21 | 101.65 |
| | | (87.48-107.19) | (92.93-114.62) | (91.32-113.13) |

**Table 4**

| Mean (SD) Hydromorphone Pharmacokinetic Parameters - Group 2 Fed | | | | |
|---|---|---|---|---|
| | 0% Alcohol | 4% Alcohol | 20% Alcohol | 40% Alcohol |
| Cmax (ng/mL) | 1.42 (0.50) | 1.64 (0.60) | 1.52 (0.32) | 1.56 (0.56) |
| Tmax (h) [Median (Range)] | 16 (6-27) | 12 (8-24) | 12 (6-24) | 16 (6-27) |
| T1/2 (h) | 11.6 (5.1) | 11.6 (4.9) | 10.4 (3.9) | 10.8 (4.8) |
| AUCinf | 37.1 (8.6) | 36.7 (10.5) | 36.6 (9.7) | 34.8(11.9) |

| Ratio-Arithmetic Mean (SD:Range) | | | | |
|---|---|---|---|---|
| Cmax | Ref | 1.20 (0.34:0.7-1.8) | 1.20 (0.33:0.8-1.9) | 1.14 (0.36:0.6-2.0) |

| Ratio - % Geometric Mean (90% Cl) | | | | |
|---|---|---|---|---|
| Cmax | Ref | 113.72 (99.97-129.36) | 114.36 (100.14-130.61) | 110.34 (97.08-125.41) |
| AUCinf | Ref | 94.72 (86.44-103.79) | 106.21 (96.63-116.73) | 94.09 (85.91-103.04) |

**Table 5**

| Formulation of Oxycodone HCl 30 mg Tablets with Stearyl Alcohol Dose = 30 mg/tablet; Tablet = 375 mg ea. | |
|---|---|
| Substances | Wt % |
| Oxycodone HCl | 8.02 |
| Lactose | 56.72 |
| Eudragit RS PO | 11.97 |
| Stearyl Alcohol | 20.29 |
| Talc | 1.99 |
| Mg Stearate | 1.00 |

**Table 6**

| Formulation of Oxycodone HCl 30 mg Tablets, No Stearyl Alcohol Dose = 30 mg/tablet; Tablet = 300 mg ea. | |
|---|---|
| Substances | Wt % |
| Oxycodone HCl | 10.09 |
| Lactose | 71.13 |
| Eudragit RS PO | 14.99 |
| Talc | 2.49 |
| Mg Stearate | 1.30 |

**Table 7**

| Formulation of Hydromorphone HCl 30 mg Tablets with Stearyl Alcohol Dose = 30 mg/tablet; Tablet = 375 mg ea. | |
|---|---|
| Substances | Wt % |
| Hydromorphone HCl | 8.02 |
| Lactose | 56.72 |
| Eudragit RS PO | 11.97 |
| Stearyl Alcohol | 20.29 |
| Talc | 1.99 |
| Mg Stearate | 1.00 |

**Table 8**

| Formulation of Hydromorphone HCl 30 mg Tablets, No Stearyl Alcohol Dose = 30 mg/tablet; Tablet = 300 mg ea. | |
|---|---|
| Substances | Wt % |
| Hydromorphone HCl | 10.09 |
| Lactose | 71.13 |
| Eudragit RS PO | 14.99 |
| Talc | 2.49 |
| Mg Stearate | 1.30 |

**Table 9**

| Formulation of Oxycodone HCl 25 mg Tablets, No Eudragit RS Dose = 25 mg/tablet; Tablet = 310 mg ea. | |
|---|---|
| Substances | Wt% |
| Oxycodone HCl | 8.05 |
| Lactose | 69.04 |
| Stearyl Alcohol | 19.92 |
| Talc | 1.99 |
| Mg Stearate | 1.00 |

**Table 10**

| Formulation of Hydromorphone HCl 25 mg Tablets, No Eudragit RS Dose = 25 mg/tablet; Tablet = 310 mg ea. | |
|---|---|
| Substances | Wt % |
| Hydromorphone HCl | 7.99 |
| Lactose | 69.03 |
| Stearyl Alcohol | 19.98 |
| Talc | 2.00 |
| Mg Stearate | 1.00 |

**Table 11**

| Formulation of Oxycodone HCl 30 mg Tablets Weight % | | | |
|---|---|---|---|
| Substances | Stearyl Alcohol | Hydrogenated Poly Castor Oil | Carnauba Wax |
| Oxycodone HCl | 8.02 | 8.06 | 8.08 |
| Lactose | 56.72 | 57.00 | 57.13 |
| Eudragit RS PO | 11.97 | 12.03 | 12.06 |
| Stearyl Alcohol | 20.29 | 19.92 | 19.76 |
| Talc | 1.99 | 1.98 | 1.98 |
| Mg Stearate | 1.00 | 0.99 | 0.99 |

## Claims

1. An opioid sustained release dosage form for use for reducing adverse effects associated with alcohol-induced dose dumping in patients who are orally receiving the dosage form, wherein:
the dosage form, when tested using an in vitro test method that employs a test medium that comprises aqueous alcohol at a concentration of 20% volume/volume, releases less than or equal to 50 weight percent of the dose of the opioid in a period of 2 hours following initiation of the in vitro test method;
and wherein:
(a) the dosage form comprises a semi-permeable membrane; and/or
(b) the dosage form comprises at least one hydrophobic component that is relatively insoluble in water and minimally swells in aqueous alcohol, wherein: (i) when the hydrophobic component comprises a hydrophobic polymer, the polymer exhibits equal or lesser swelling and/or solubility in aqueous alcohol than in water, and (ii) when the hydrophobic component is non-polymeric, the component exhibits less solubility/swelling in aqueous alcohol than in water.

2. A dosage form of claim 1 wherein the opioid is hydromorphone.

3. A dosage form of any previous claim which satisfies the in vitro test when the alcohol concentration in the test medium is 40% volume/volume.

4. A dosage form of any previous claim comprising an immediate release component for immediate release of the opioid.

5. A dosage form of any previous claim comprising an opioid antagonist.

6. A dosage form of any previous claim which is a once-per-day dosage form.

7. A dosage form of any previous claim which is a twice-per-day dosage form.

8. A dosage form of any previous claim which is an osmotic sustained release dosage form.

9. A dosage form of any previous claim for use for providing to a patient population which includes individuals that can be expected to at least occasionally co-ingest the dosage form with an alcoholic beverage.

10. A dosage form of any previous claim which is provided to patients without special label warnings to physicians and/or patients regarding potential lethal effects from taking the dosage form with alcohol.

## Patentansprüche

1. Darreichungsform für ein Opioid mit verzögerter Freisetzung zur Verwendung bei der Abschwächung unerwünschter Nebenwirkungen im Zusammenhang mit einer Alkoholinduzierten Sturzentleerung bei Patienten, denen die Darreichungsform oral verabreicht wird, wobei:
die Darreichungsform, wenn sie in einem in-vitro-Testverfahren, das wässrigen Alkohol bei einer Konzentration von 20% Volumen/Volumen beinhaltet, getestet wird, in einem Zeitraum von 2 Stunden im Anschluss an die initiierung des in-vitro-Testvertahrens höchstens 50 Gewichtsprozent der Opioiddosis freisetzt;
und wobei:
(a) die Darreichungsform eine halbdurchlässige Membran beinhaltet,
und/oder
(b) die Darreichungsform ferner mindestens eine hydrophobe Komponente beinhaltet, die in Wasser relativ schlecht löslich ist und die in wässrigem Alkohol minimal aufquillt, wobei: (i) wenn die hydrophobe Komponente ein hydrophobes Polymer beinhaltet, das Polymer in wässrigem Alkohol höchstens im gleichen Maße aufquillt und/oder löslich ist wie in Wasser, und (ii) wenn die hydrophobe Komponente nicht-polymer ist, die Komponente in wässrigem Alkohol in geringerem Maße löslich ist / aufquillt als in Wasser.

2. Darreichungsform nach Anspruch 1, wobei das Opioid Hydromorphon ist.

3. Darreichungsform nach einem der vorangehenden Ansprüche, die den in-vitro-Test besteht, wenn die Alkoholkonzentration im Testmedium 40% Volumen/Volumen beträgt.

4. Darreichungsform nach einem der vorangehenden Ansprüche, eine Komponente mit sofortiger Freisetzung aufweisend, die das Opioid sofort freisetzt.

5. Darreichungsform nach einem der vorangehenden Ansprüche, einen Opioid-Antagonisten aufweisend.

6. Darreichungsform nach einem der vorangehenden Ansprüche, bei der es sich um eine einmal täglich zu verabreichende Darreichungsform handelt.

7. Darreichungsform nach einem der vorangehenden Ansprüche, bei der es sich um eine zweimal täglich zu verabreichende Darreichungsform handelt.

8. Darreichungsform nach einem der vorangehenden Ansprüche, bei der es sich um eine osmotische Darreichungsform mit verzögerter Freisetzung handelt.

9. Darreichungsform nach einem der vorangehenden Ansprüche zur Verwendung für die Ausgabe an eine Patientenpopulation, die Individuen beinhaltet, von denen angenommen werden kann, dass sie die Darreichungsform zumindest gelegentlich zusammen mit einem alkoholischen Getränk einnehmen.

10. Darreichungsform nach einem der vorangehenden Ansprüche, die an Patienten ausgegeben wird, ohne dass spezielle Hinweise auf dem Etikett Ärzte und/oder Patienten vor möglichen tödlichen Wirkungen aufgrund einer Einnahme der Darreichungsform mit Alkohol warnen.

## Revendications

1. Une forme posologique à libération soutenue d'opioïde destinée à une utilisation dans la réduction d'effets indésirables associés à une libération massive induite par l'alcool chez des patients qui reçoivent la forme posologique par voie orale, où :
la forme posologique, lorsqu'elle est testée au moyen d'un procédé d'essai in vitro qui emploie un milieu d'essai qui contient un alcool aqueux à une concentration de 20% volume/volume, libère une quantité inférieure ou égale à 50% en poids de la dose de l'opioïde pendant une période de deux heures suivant le démarrage du procédé d'essai in vitro, et où :
(a) la forme posologique comprend une membrane semi-perméable, et/ou
(b) la forme posologique comprend au moins un composant hydrophobe qui est relativement insoluble dans l'eau et gonfle de manière minimale dans un alcool aqueux, où : (i) lorsque le composant hydrophobe comprend un polymère hydrophobe, le polymère présente un gonflage et/ou une solubilité égaux ou inférieurs dans un alcool aqueux que dans de l'eau, et (ii) lorsque le composant hydrophobe est non polymère, le composant présente un gonflage/solubilité inférieurs dans un alcool aqueux que dans de l'eau.

2. Une forme posologique selon la Revendication 1 où l'opioïde est hydromorphone.

3. Une forme posologique selon l'une quelconque des Revendications précédentes qui satisfait à l'essai in vitro lorsque la concentration d'alcool dans le milieu d'essai est de 40% volume/volume.

4. Une forme posologique selon l'une quelconque des Revendications précédentes contenant un composant à libération immédiate pour une libération immédiate de l'opioïde.

5. Une forme posologique selon l'une quelconque des Revendications précédentes contenant un antagoniste d'opioïde.

6. Une forme posologique selon l'une quelconque des Revendications précédentes qui est une forme posologique à une prise par jour.

7. Une forme posologique selon l'une quelconque des Revendications précédentes qui est une forme posologique à deux prises par jour.

8. Une forme posologique selon l'une quelconque des Revendications précédentes qui est une forme posologique à libération soutenue osmotique.

9. Une forme posologique selon l'une quelconque des Revendications précédentes pour une utilisation destinée à une population de patients qui comprend des individus dont on peut s'attendre qu'ils co-ingèrent au moins occasionnellement la forme posologique avec une boisson alcoolisée.

10. Une forme posologique selon l'une quelconque des Revendications précédentes qui est fournie à des patients sans avertissements d'étiquetage spéciaux destinés à des médecins et/ou des patients concernant des effets létaux potentiels liés à une prise de la forme posologique avec de l'alcool.
